# EUROPEAN PATENT APPLICATION

(11) **EP 4 556 465 A1**
(43) Date of publication of application: **21.05.2025**
(21) Application number: 23893553.0
(22) Date of filing: 30.10.2023
(51) Int. Cl.: C07D 209/46, C07D 209/44, C07D 471/04, A61K 31/4035, A61K 31/4375, A61P 35/00

(54) **NOVEL BICYCLIC PD-L1 INHIBITORS, PREPARATION METHODS THEREFOR AND MEDICINAL USES THEREOF**

(30) Priority: 22.11.2022 CN 202211464835
(71) Applicant: Xi'an Xintong Pharmaceutical Research Co., Ltd., Xi'an, Shaanxi 710000 (CN)
(72) Inventor: XU, Yungen, Xi'an, Shaanxi 710000 (CN); DU, Huijie, Xi'an, Shaanxi 710000 (CN); ZHU, Qihua, Xi'an, Shaanxi 710000 (CN); ZOU, Yi, Xi'an, Shaanxi 710000 (CN); GUO, Weibo, Xi'an, Shaanxi 710000 (CN); ZHANG, Qiang, Xi'an, Shaanxi 710000 (CN); ZHANG, Chi, Xi'an, Shaanxi 710000 (CN); XU, Yongling, Xi'an, Shaanxi 710000 (CN); XIA, Yu, Xi'an, Shaanxi 710000 (CN); LIU, Beibei, Xi'an, Shaanxi 710000 (CN); WANG, Shuping, Xi'an, Shaanxi 710000 (CN); QIN, Long, Xi'an, Shaanxi 710000 (CN); HAO, Zhongyan, Xi'an, Shaanxi 710000 (CN)
(74) Representative: Murgitroyd & Company
(86) International application number: PCT/CN2023/127693
(87) International publication number: WO 2024/109462

(57) **Abstract**

The present invention relates to the field of medicinal chemistry, and discloses pyridoheterocyclic derivatives having PD-1/PD-L1 inhibitory activity, a preparation method therefor and a use thereof. The present invention also discloses a composition containing the pyridoheterocyclic derivative having PD-1/PD-L1 inhibitory activity, or a pharmaceutically acceptable salt thereof, and a pharmaceutically acceptable carrier, and a use of the composition in the preparation of a PD-1/PD-L1 inhibitor. The compound of the present invention may be used to treat tumors.

## Description

### TECHNICAL FIELD

The present invention belongs to the field of medicinal chemistry, and in particular relates to biphenyl derivatives having PD-1/PD-L1 inhibitory activity, a preparation method therefor, a pharmaceutical composition containing these compounds and a use of the pharmaceutical composition in the treatment of tumors.

### BACKGROUND

In recent years, tumor immunotherapy has become a focus in the field of tumor treatment, and unlike traditional treatment methods that directly target tumor cells, the tumor immunotherapy utilizes the human body's own immune system to kill tumor cells. Activation of immune checkpoint pathways will inhibit the activation of T cells, preventing excessive activation of the human immune system, maintaining the immune tolerance of the normal body, and avoiding the occurrence of autoimmune diseases. Tumors cause immune escape by overactivating immune checkpoint pathways by themselves and some lymphocytes. Among these immune checkpoints, excessive activation of PD-1/PD-L1 plays a vital role in tumor development. Blocking PD-1/PD-L1 interaction can reactivate the immune system and kill tumor cells, showing good efficacy in clinical treatment of tumors such as melanoma, colon cancer, and non-small cell lung cancer (Clinical and Translational Oncology, 2019, 21: 702-712; Lung Cancer: Targets and Therapy, 2017: 8; Hum VaccinImmunother, 2014, 10 (11): 3111-3116; Journal of Medicinal Chemistry, 2020, 63 (22): 13825-13850).

To date, a plurality of PD-1/PD-L1-targeted mAbs have been approved by FDA for tumor immunotherapy, and hundreds of mAbs are currently actively undergoing clinical trials. Although these monoclonal antibodies improve the prognosis of many cancer patients, only a small number of patients can have a lasting response due to the development of intrinsic resistance and acquired resistance. The use of mAbs will also lead to serious immune-related adverse events (irAE), common toxic side effects including skin inflammation, colitis, hepatitis, hypothyroidism, hypophysitis, and the like. In addition, macromolecular drugs also have disadvantages such as poor tissue permeability, complicated preparation, high cost, and poor patient compliance. Therefore, the development of small molecule inhibitors is expected to solve the problems of the monoclonal antibodies described above.

Compared with the monoclonal antibodies, small molecule drugs have the following obvious advantages: 1) the small molecule drugs have a simple mode of administration, are more suitable for oral administration, have adjustable drug half-life, and avoid serious immunotherapy-related adverse events; 2) the small molecule drugs have good membrane permeability, and can be directly exposed to the tumor microenvironment or cross physiological barriers; 3) the small molecule drugs can directly act on intracellular targets that cannot be reached by macromolecules; 4) the small molecule drugs are readily available and are more flexible in the selection of dosage forms and doses; and 5) the small molecule drugs have a low production cost, do not require refrigeration, and are convenient to store and transport.

At present, there are no PD-1/PD-L1 small molecule inhibitor on the market, so the development of an inhibitor that blocks the PD-1/PD-L1 protein-protein interaction has significant practical significance and potential application prospects.

### SUMMARY

An object of the present invention: in view of the above technical problems existing in the prior art, the present invention provides a pyridoheterocyclic derivative having PD-1/PD-L1 inhibitory activity, a preparation method therefor and a pharmaceutical use thereof as a PD-1/PD-L1 protein-protein interaction inhibitor.

Technical solution: the present invention discloses a pyridoheterocyclic derivative represented by general formula (I) or a pharmaceutically acceptable salt thereof: 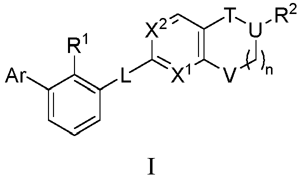 wherein:
Ar represents 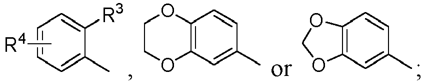
L represents -(CH₂)ₘ-, -O-, -NH-, -CH₂O-, -CF₂O-, -CH₂NH-, -CONH-, -HNCO-, - NHCH₂-, -OCF₂-, -OCH₂- or -CH=CH-, wherein m represents 0, 1 or 2;
X¹ and X² each independently represent N or CH;
T and V each independently represent 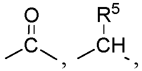 -O-, 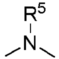 or -S-; wherein R⁵ represents H, C₁-C₆ alkyl or C₃-C₇ cycloalkyl;
U represents CH or N;
n represents 0, 1, 2 or 3;
R¹ and R³ each independently represent H, D, halogen, CN, C₁-C₃ haloalkyl, C₁-C₃ alkyl or cyclopropyl;
R² represents H, substituted C₁-C₆ alkyl, substituted C₃-C₇ cycloalkyl or substituted C₃-C₇ heterocycloalkyl, substituents being H, OH, NH₂, COOH, an amide group, an ester group, alkoxy or an aldehyde group, and being monosubstituted or polysubstituted, the heterocycloalkyl containing 1 to 3 heteroatoms selected from N, O and S;
R⁴ represents H, halogen, CN, CF₃, OH, NH₂, -O(CH₂)ₚR⁶, substituted C₁-C₆ alkyl, substituted C₃-C₇ cycloalkyl or substituted C₃-C₇ heterocycloalkyl, substituents being H, OH, NH₂, COOH, an amide group, an ester group or alkoxy, and being monosubstituted or polysubstituted, wherein p represents 1, 2, 3 or 4, the heterocycloalkyl containing 1 to 3 heteroatoms selected from N, O and S; and
R⁶ represents NR⁷R⁸, OR⁷ or substituted C₄-C₆ azacycloalkyl, wherein R⁷ represents H or C₁-C₃ alkyl, R⁸ represents substituted C₁-C₆ alkyl, the C₄-C₆ azacycloalkyl being tetrahydropyrrol-1-yl, piperidin-1-ylmorpholin-1-ylpiperazin-1-yl or azetidin-1-yl, substituents being OH, NH₂, COOH, an amide group, an ester group or alkoxy, and being monosubstituted or polysubstituted.

Wherein:
Ar preferably represents 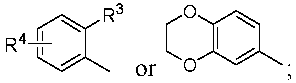
L represents -(CH₂)ₘ-, -CH₂O-, -CF₂O-, -CONH-, -NHCO- or -OCH₂-, wherein m represents 0;
X¹ and X² each independently represent N or CH;
T and V each represent 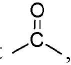 -CH₂-, -O-, -NH- or 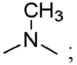
U represents N;
n represents 0 or 1;
R¹ and R³ each independently represent H, D, F, Cl, Br, CN, CH₃ or CF₃;
R² represents H, 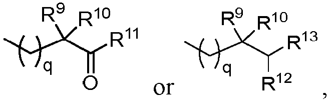 wherein q represents 0 or 1, and R⁹
and R¹⁰ each independently represent H, OH, COOH, CH₂COOH, CH₂NH₂, CH₂OH, CH₂CH₂OH, F, Cl, Br, CH₃ or CH₂CH₃;
R¹¹ represents OH, NH₂, NHCH₃, CH₃, OCH₃, OCH₂CH₃ or OCH(CH₃)₂;
R¹² represents CONH₂, NHCOCH₃, OH, CH₂OH, CH₂CH₂OH, COOH, COOCH₃, COOCH₂CH₃ or COOCH(CH₃)₂;
R¹³ represents H, CH₃, CH₂CH₃, CH₂OH or CH₂CH₂OH;
R⁴ represents H, F, Cl, Br, CN, CF₃, OH, NH₂ or -O(CH₂)ₚR⁶, wherein p represents 2, 3 or 4, and R⁶ represents OH, 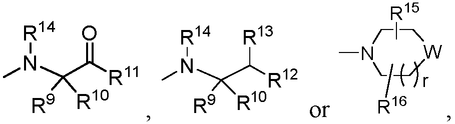 wherein R⁹, R¹⁰, R¹¹, R¹² and R¹³ are as previously defined, R¹⁴ represents CH₃, CH₂CH₃, CH₂CH₂OH, formyl or acetyl, R¹⁵ and R¹⁶ each independently represent H, OH, COOH, NH₂, CH₃, CH₂CH₃, CH₂OH, CH₂CH₂OH, CONH₂, cyclopropyl, COOCH₃, COOCH₂CH₃ or COOCH(CH₃)₂, W represents -CH₂-, -O-, -NH-, 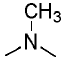 or 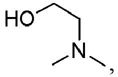 and r represents 0 or 1.

Wherein more preferably:
Ar represents 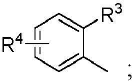
L represents -CH₂O- or -NHCO-;
X¹ represents CH or N, and X² represents CH;
T represents 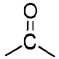 or -CH₂-;
U represents N;
V represents -CH₂-; and
n=0 or 1.
R¹ and R³ each independently represent F, Cl, Br, CN, CH₃ or CF₃.
R² represents H, 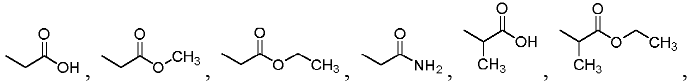 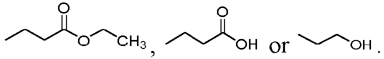
R⁴ represents H, F, Cl, CN, CF₃, OH, NH₂ or -O(CH₂)₃R⁶, wherein R⁶ represents OH, CONH₂, CH₂OH, COOH, COOCH₃, COOCH₂CH₃ or

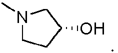

More preferably, the compound is any one of the following compounds:

| Compound name | Compound structure |
|---|---|
| Ethyl 2-(5-(2'-fluoro-2-methyl-[1,1'-biphenyl]-3-yl)-1-oxoisoindolin-2-yl)acetate | 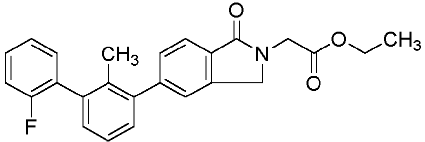 I-A-1 |
| Ethyl 3-(5-(2'-fluoro-2-methyl-[1,1'-biphenyl]-3-yl)-1-oxoisoindolin-2-yl)propanoate | 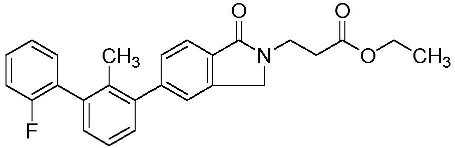 I-A-2 |
| Ethyl 2-(5-(2'-fluoro-2-methyl-[1,1'-biphenyl]-3-yl)-1-oxoisoindolin-2-yl)propanoate | 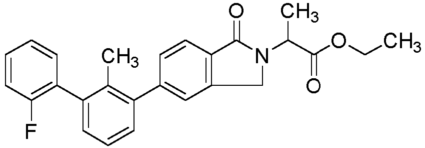 I-A-3 |
| 2-(5-(2'-Fluoro-2-methyl-[1,1'-biphenyl]-3-yl)-1-oxoisoindolin-2-yl)acetic acid | 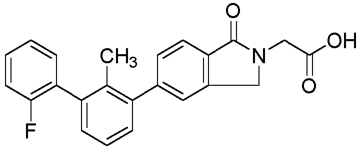 I-A-4 |
| 3-(5-(2'-Fluoro-2-methyl-[1,1'-biphenyl]-3-yl)-1-oxoisoindolin-2-yl)propanoic acid | 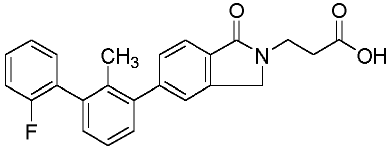 I-A-5 |
| 2-(5-(2'-Fluoro-2-methyl-[1,1'-biphenyl]-3-yl)-1-oxoisoindolin-2-yl)propanoic acid | 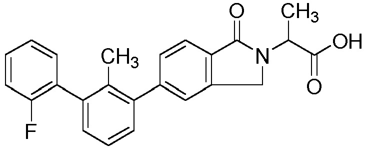 I-A-6 |
| 5-(2'-Fluoro-2-methyl-[1,1'-biphenyl]-3-yl)-2-(2-hydroxyethyl)isoindo 1-1-one | 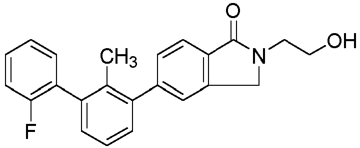 I-A-7 |
| 2-(5-(2'-Fluoro-2-methyl-[1,1'-biphenyl]-3-yl)-1-oxoisoindolin-2-yl)acetamide | 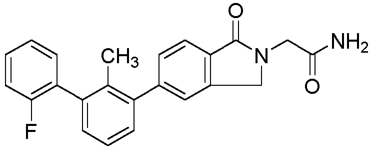 I-A-8 |
| 2-(5-(2'-Fluoro-2-methyl-[1,1'-biphenyl]-3-yl)isoindolin-2-yl)ethan-1-ol | 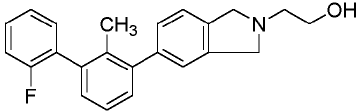 I-A-9 |
| 5-(2'-Fluoro-2-methyl-[1,1'-biphenyl]-3-yl)isoindolin-1-one | 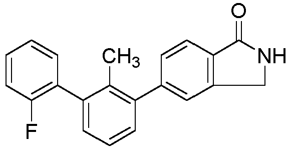 I-A-10 |
| 5-(2'-Fluoro-2-methyl-[1,1'-biphenyl]-3-yl)isoindoline | 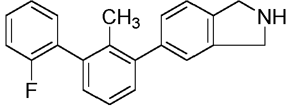 I-A-11 |
| Ethyl 2-(2-(2'-fluoro-2-methyl-[1,1'-biphenyl]-3-yl)-7,8-dihydro-1,6-naphthyridin-6(5*H*)-yl)acetate | 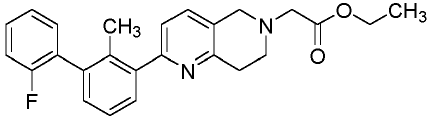 I-B-1 |
| 2-(2-(2'-Fluoro-2-methyl-[1,1'-biphenyl]-3-yl)-7,8-dihydro-1,6-naphthyridin-6(5*H*)-yl)acetic acid | 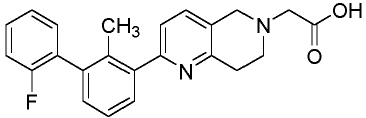 I-B-2 |
| 2-(2-(2'-Fluoro-2-methyl-[1,1'-biphenyl]-3-yl)-7,8-dihydro-1,6-naphthyridin-6(5*H*)-yl)ethan-1-ol | 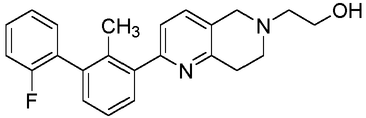 I-B-3 |
| 2-(2'-Fluoro-2-methyl-[1,1'-biphenyl]-3-yl)-5,6,7,8-tetrahydro-1,6-naphthyridine | 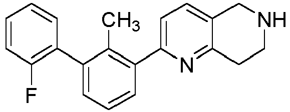 I-B-4 |
| Ethyl 2-(2-((3-(2,3-dihydrobenzo[*b*][1,4] dioxin-6-yl)-2-methylbenzyl)oxy)-7,8-dihydro-1,6-naphthyridin-6(5*H*)-yl)acetate | 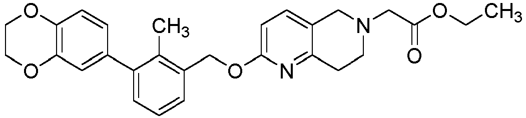 I-C-1 |
| 2-((3-(2,3-Dihydrobenzo[b][1,4] dioxin-6-yl)-2-methylbenzyl)oxy)-5,6,7,8-tetrahydro-1,6-naphthyridine | 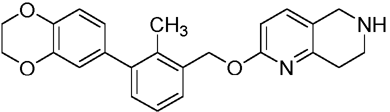 I-C-2 |
| Ethyl 2-(2-((2'-fluoro-2-methyl-[1,1'-biphenyl]-3-yl)methoxy)-7,8-dihydro-1,6-naphthyridin-6(5*H*)-yl)acetate | 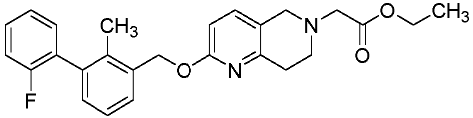 I-D-1 |
| Ethyl 2-(2-((2,2'-dimethyl-[1,1'-biphenyl]-3-yl)methoxy)-7,8-dihydro-1,6-naphthyridin-6(5*H*)-yl)acetate | 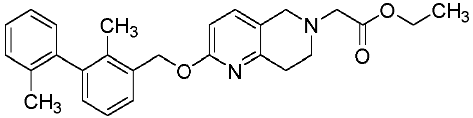 I-D-2 |
| Ethyl 2-(2-((2'-chloro-2-methyl-[1,1'-biphenyl]-3-yl)methoxy)-7,8-dihydro-1,6-naphthyridin-6(5*H*)-yl)acetate | 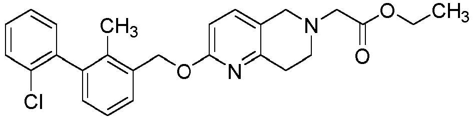 I-D-3 |
| Ethyl 2-(2-((2-methyl-[1,1'-biphenyl]-3-yl)methoxy)-7,8-dihydro-1,6-naphthyridin-6(5*H*)-yl)acetate | 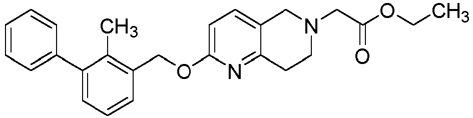 I-D-4 |
| Ethyl 2-(2-((2-chloro-2'-fluoro-[1,1'-biphenyl]-3-yl)methoxy)-7,8-dihydro-1,6-naphthyridin-6(5*H*)-yl)acetate | 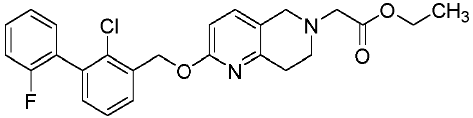 I-D-5 |
| Ethyl 2-(2-((2-chloro-2'-methyl-[1,1'-biphenyl]-3-yl)methoxy)-7,8-dihydro-1,6-naphthyridin-6(5*H*)-yl)acetate | 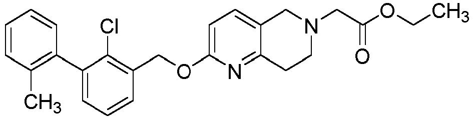 I-D-6 |
| Ethyl 2-(2-((2,2'-dichloro-[1,1'-biphenyl]-3-yl)methoxy)-7,8-dihydro-1,6-naphthyridin-6(5*H*)-yl)acetate | 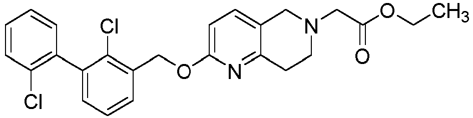 I-D-7 |
| Ethyl 2-(2-((2-chloro-[1,1'-biphenyl]-3-yl)methoxy)-7,8-dihydro-1,6-naphthyridin-6(5*H*)-yl)acetate | 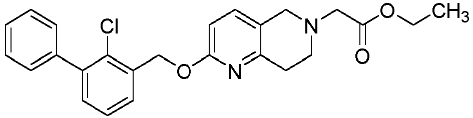 I-D-8 |
| Ethyl 2-(2-((2,2'-difluoro-[1,1'-biphenyl]-3-yl)methoxy)-7,8-dihydro-1,6-naphthyridin-6(5*H*)-yl)acetate | 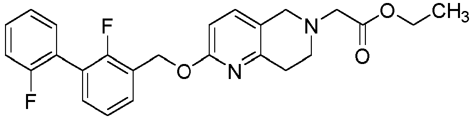 I-D-9 |
| Ethyl 2-(2-((2-fluoro-2'-methyl-[1,1'-biphenyl]-3-yl)methoxy)-7,8-dihydro-1,6-naphthyridin-6(5*H*)-yl)acetate | 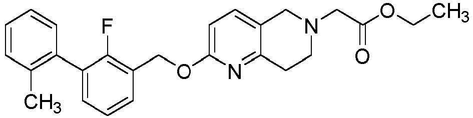 I-D-10 |
| Ethyl 2-(2-((2'-chloro-2-fluoro-[1,1'-biphenyl]-3-yl)methoxy)-7,8-dihydro-1,6-naphthyridin-6(5*H*)-yl)acetate | 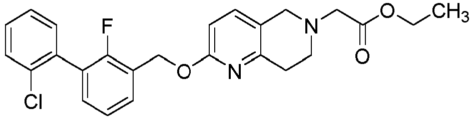 I-D-11 |
| Ethyl 2-(2-((2-fluoro-[1,1'-biphenyl]-3-yl)methoxy)-7,8-dihydro-1,6-naphthyridin-6(5*H*)-yl)acetate | 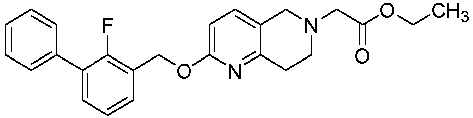 I-D-12 |
| 2-(2-((2'-fluoro-2-methyl-[1,1'-biphenyl]-3-yl)methoxy)-7,8-dihydro-1,6-naphthyridin-6(5*H*)-yl)acetic acid | 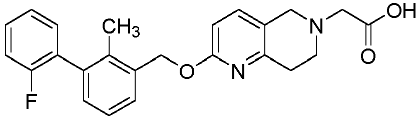 I-D-13 |
| 2-(2-((2'-fluoro-2-methyl-[1,1'-biphenyl]-3-yl)methoxy)-7,8-dihydro-1,6-naphthyridin-6(5*H*)-yl)ethan-1-ol | 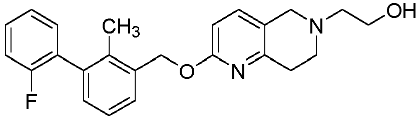 I-D-14 |
| 2-(2-((2,2'-dimethyl-[1,1'-biphenyl]-3-yl)methoxy)-7,8-dihydro-1,6-naphthyridin-6(5*H*)-yl)ethan-1-ol | 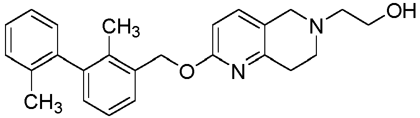 I-D-15 |
| 2-(2-((2'-chloro-2-methyl-[1,1'-biphenyl]-3-yl)methoxy)-7,8-dihydro-1,6-naphthyridin-6(5*H*)-yl)ethan-1-ol | 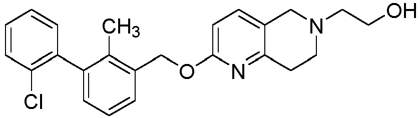 I-D-16 |
| 2-(2-((2-methyl-[1,1'-biphenyl]-3-yl)methoxy)-7,8-dihydro-1,6-naphthyridin-6(5*H*)-yl)ethan-1-ol | 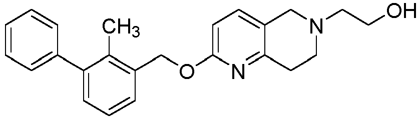 I-D-17 |
| 2-(2-((2-chloro-2'-fluoro-[1,1'-biphenyl]-3-yl)methoxy)-7,8-dihydro-1,6-naphthyridin-6(5*H*)-yl)ethan-1-ol | 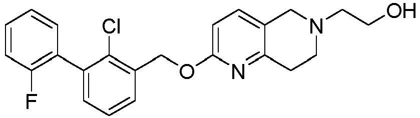 I-D-18 |
| 2-(2-((2-chloro-2'-methyl-[1,1'-biphenyl]-3-yl)methoxy)-7,8-dihydro-1,6-naphthyridin-6(5*H*)-yl)ethan-1-ol | 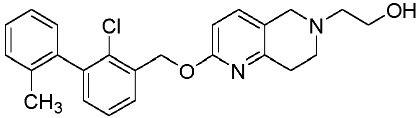 I-D-19 |
| 2-(2-((2,2'-dichloro-[1,1'-biphenyl]-3-yl)methoxy)-7,8-dihydro-1,6-naphthyridin-6(5*H*)-yl)ethan-1-ol | 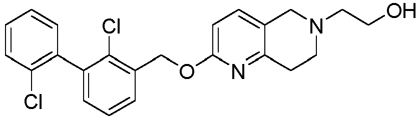 I-D-20 |
| 2-(2-((2-chloro-[1,1'-biphenyl]-3-yl)methoxy)-7,8-dihydro-1,6-naphthyridin-6(5*H*)-yl)ethan-1-ol | 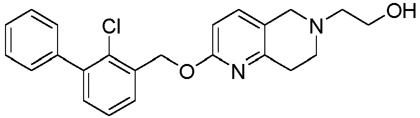 I-D-21 |
| 2-(2-((2,2'-difluoro-[1,1'-biphenyl]-3-yl)methoxy)-7,8-dihydro-1,6-naphthyridin-6(5*H*)-yl)ethan-1-ol | 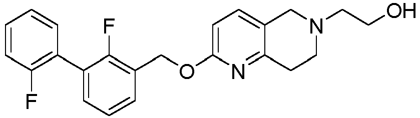 I-D-22 |
| 2-(2-((2-fluoro-2'-methyl-[1,1'-biphenyl]-3-yl)methoxy)-7,8-dihydro-1,6-naphthyridin-6(5*H*)-yl)ethan-1-ol | 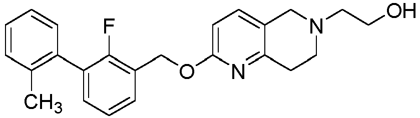 I-D-23 |
| 2-(2-((2'-chloro-2-fluoro-[1,1'-biphenyl]-3-yl)methoxy)-7,8-dihydro-1,6-naphthyridin-6(5*H*)-yl)ethan-1-ol | 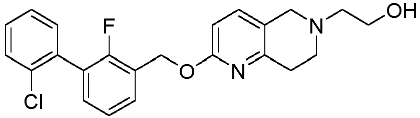 I-D-24 |
| 2-(2-((2-fluoro-[1,1'-biphenyl]-3-yl)methoxy)-7,8-dihydro-1,6-naphthyridin-6(5*H*)-yl)ethan-1-ol | 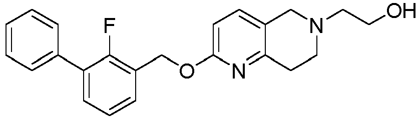 I-D-25 |
| 2-(2-((2-chloro-2'-fluoro-[1,1'-biphenyl]-3-yl)methoxy)-7,8-dihydro-1,6-naphthyridin-6(5*H*)-yl)acetic acid | 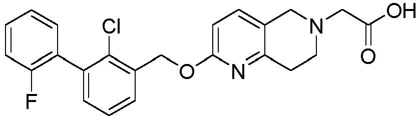 I-D-26 |
| 2-((2-chloro-2'-fluoro-[1,1'-biphenyl]-3-yl)methoxy)-5,6,7,8-tetrahydro-1,6-naphthyridine | 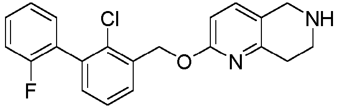 I-D-27 |
| 2-(2-((2-chloro-2'-fluoro-[1,1'-biphenyl]-3-yl)methoxy)-7,8-dihydro-1,6-naphthyridin-6(5*H*)-yl)acetaldehyde | 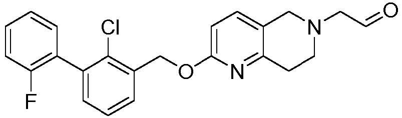 I-D-28 |
| 2-((2-chloro-2'-fluoro-[1,1'-biphenyl]-3-yl)methoxy)-6-(2-hydroxyethyl)-5,6,7,8-tetrahydro-1,6-naphthyridine 6-oxide | 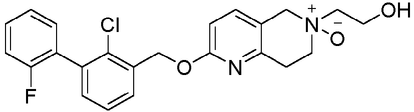 I-D-29 |
| 2-(2-((2'-fluoro-[1,1'-biphenyl]-3-yl)methoxy)-7,8-dihydro-1,6-naphthyridin-6(5*H*)-yl)acetic acid | 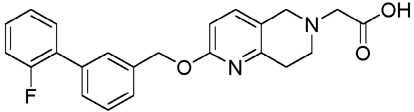 I-D-30 |
| 2-((2-chloro-2'-fluoro-[1,1'-biphenyl]-3-yl)methoxy)-5,6,7,8-tetrahydro-1,6-naphthyridine 6-oxide | 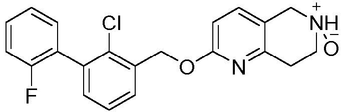 I-D-31 |
| Methyl 2-(7-((2,2'-dimethyl-[1,1'-biphenyl]-3-yl)methoxy)-1-methyl-1,4-dihydropyrido[2,3-*d*]pyrimidin-3(2*H*)-yl)acetate | 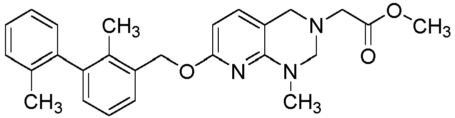 I-E-1 |
| Methyl (R)-2-(7-((3'-(3-(3-hydroxypyrrolidin-1-yl)propoxy)-2,2'-dimethyl-[1,1'-biphenyl]-3-yl)methoxy)-1-methyl-1,4-dihydropyrido[2,3-*d*]pyrimidin-3(2*H*)-yl)acetate | 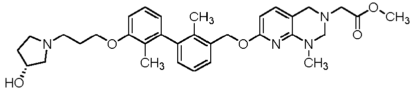 I-E-2 |
| 2-(7-((2,2'-dimethyl-[1,1'-biphenyl]-3-yl)methoxy)-1-methyl-1,4-dihydropyrido[2,3-*d*]pyrimidin-3(2*H*)-yl)acetic acid | 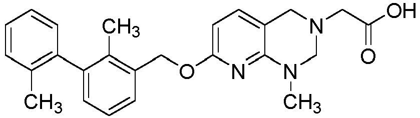 I-E-3 |
| (R)-2-(7-((3'-(3-(3-hydroxypyrrolidin-1-yl)propoxy)-2,2'-dimethyl-[1,1'-biphenyl]-3-yl)methoxy)-1-methyl-1,4-dihydropyrido[2,3-*d*]pyrimidin-3(2*H*)-yl)acetic acid | 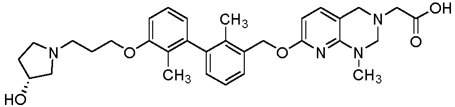 I-E-4 |
| 7-((2,2'-Dimethyl-[1,1'-biphenyl]-3-yl)methoxy)-1-methyl-1,2,3,4-tetrahydropyrido[2,3-*d*]pyrimidine | 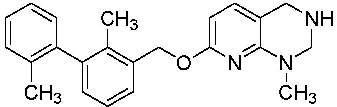 I-E-5 |
| (R)-1-(3-((2,2'-dimethyl-3'-(((1-methyl-1,2,3,4-tetrahydropyrido[2,3-*d*]pyrimidin-7-yl)oxy)methyl)-[1,1'-biphenyl]-3-yl)oxy)propyl)pyrrolidin-3-ol | 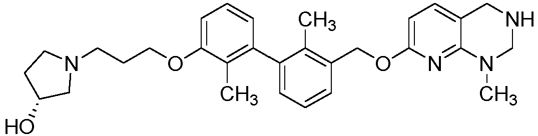 I-E-6 |
| Ethyl 2-(2-((2-chloro-2'-fluoro-[1,1'-biphenyl]-3-yl)carbamoyl)-7,8-dihydro-1,6-naphthyridin-6(5*H*)-yl)acetate | 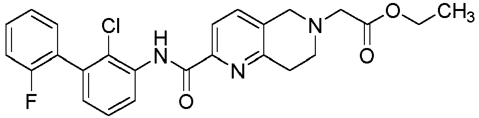 I-F-1 |
| N-(2-chloro-2'-fluoro-[1,1'-biphenyl]-3-yl)-6-(2-hydroxyethyl)-5,6,7,8-tetrahydro-1,6-naphthyridine-2-carboxamide | 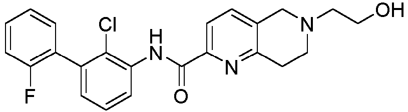 I-F-2 |
| 2-(2-((2-Chloro-2'-fluoro-[1,1'-biphenyl]-3-yl)carbamoyl)-7,8-dihydro-1,6-naphthyridin-6(5*H*)-yl)acetic acid | 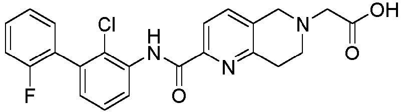 I-F-3 |
| N-(2-chloro-2'-fluoro-[1,1'-biphenyl]-3-yl)-5,6,7,8-tetrahydro-1,6-naphthyridine-2-carboxamide | 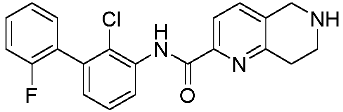 I-F-4 |
| Ethyl 2-(5-(2-methyl-[1,1'-biphenyl]-3-yl)isoindolin-2-yl)acetate | 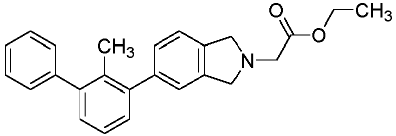 I-G-1 |
| Ethyl 2-(5-(2,2'-dimethyl-[1,1'-biphenyl]-3-yl)isoindolin-2-yl)acetate | 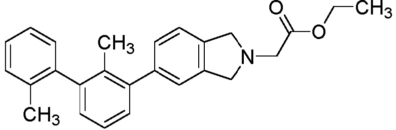 I-G-2 |
| Ethyl 2-(5-(2'-chloro-2-methyl-[1,1'-biphenyl]-3-yl)isoindolin-2-yl)acetate | 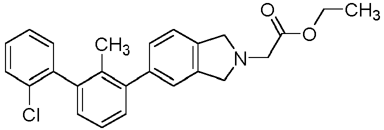 I-G-3 |
| Ethyl 2-(5-(2'-fluoro-2-methyl-[1,1'-biphenyl]-3-yl)isoindolin-2-yl)acetate | 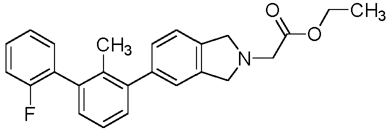 I-G-4 |
| 2-(5-(2-methyl-[1,1'-biphenyl]-3-yl)isoindolin-2-yl)acetic acid | 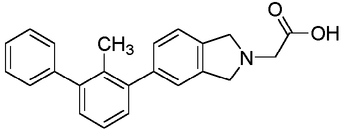 I-G-5 |
| 2-(5-(2,2'-dimethyl-[1,1'-biphenyl]-3-yl)isoindolin-2-yl)acetic acid | 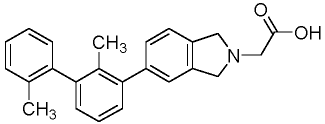 I-G-6 |
| 2-(5-(2'-chloro-2-methyl-[1,1'-biphenyl]-3-yl)isoindolin-2-yl)acetic acid | 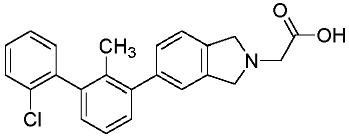 I-G-7 |
| 2-(5-(2'-fluoro-2-methyl-[1,1'-biphenyl]-3-yl)isoindolin-2-yl)acetic acid | 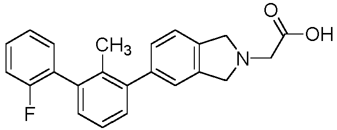 I-G-8 |
| 2-(5-(2-methyl-[1,1'-biphenyl]-3-yl)isoindolin-2-yl)-N-methylacetamide | 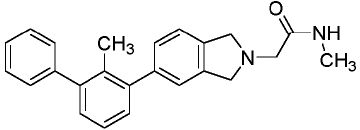 I-G-9 |
| 2-(5-(2,2'-dimethyl-[1,1'-biphenyl]-3-yl)isoindolin-2-yl)-N-methylacetamide | 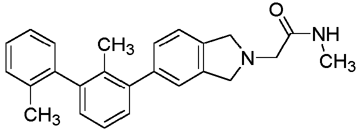 I-G-10 |
| 2-(5-(2'-chloro-2-methyl-[1,1'-biphenyl]-3-yl)isoindolin-2-yl)-N-methylacetamide | 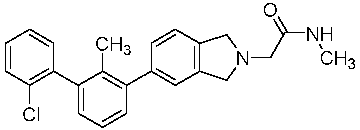 I-G-11 |
| 2-(5-(2'-fluoro-2-methyl-[1,1'-biphenyl]-3-yl)isoindolin-2-yl)-N-methylacetamide | 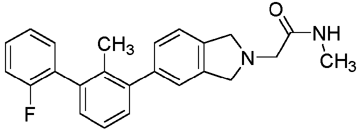 I-G-12 |
| 2-(5-(2-methyl-[1,1 '-biphenyl]-3-yl)isoindolin-2-yl)ethan-1-ol | 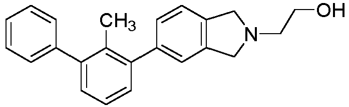 I-G-13 |
| 2-(5-(2,2'-dimethyl-[1,1'-biphenyl]-3-yl)isoindolin-2-yl)ethan-1-ol | 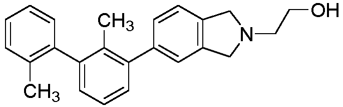 I-G-14 |
| 2-(5-(2'-chloro-2-methyl-[1,1'-biphenyl]-3-yl)isoindolin-2-yl)ethan-1-ol | 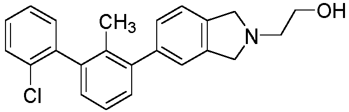 I-G-15 |
| 2-(5-(2'-Fluoro-2-methyl-[1,1'-biphenyl]-3-yl)isoindolin-2-yl)ethan-1-ol | 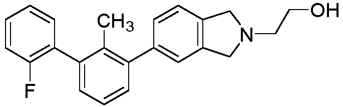 I-G-16 |
| 5-(2-Methyl-[1,1'-biphenyl]-3-yl)isoindoline | 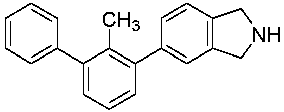 I-G-17 |
| 5-(2,2'-Dimethyl-[1,1'-biphenyl]-3-yl)isoindoline | 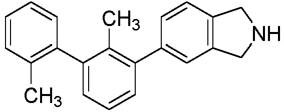 I-G-18 |
| 5-(2'-Chloro-2-methyl-[1,1'-biphenyl]-3-yl)isoindoline | 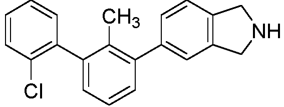 I-G-19 |
| 5-(2'-Fluoro-2-methyl-[1,1'-biphenyl]-3-yl)isoindoline | 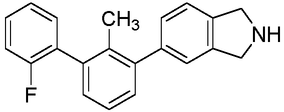 I-G-20 |
| Ethyl 2-(7-(2-methyl-[1,1'-biphenyl]-3-yl)-1,3,4,5-tetrahydro-2*H*-benzo[c]azepin-2-yl)acetate | 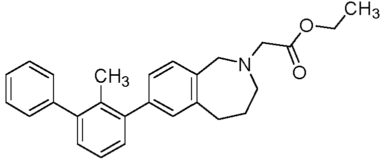 I-H-1 |
| Ethyl 2-(7-(2,2'-dimethyl-[1,1'-biphenyl]-3-yl)-1,3,4,5-tetrahydro-2*H*-benzo[c]azepin-2-yl)acetate | 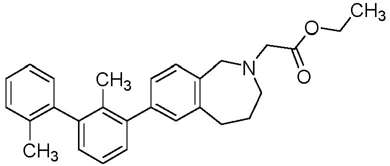 I-H-2 |
| Ethyl 2-(7-(2'-chloro-2-methyl-[1,1'-biphenyl]-3-yl)-1,3,4,5-tetrahydro-2*H*-benzo[c]azepin-2-yl)acetate | 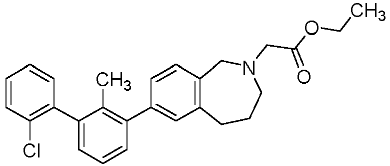 I-H-3 |
| Ethyl 2-(7-(2'-fluoro-2-methyl-[1,1'-biphenyl]-3-yl)-1,3,4,5-tetrahydro-2*H*-benzo[c]azepin-2-yl)acetate | 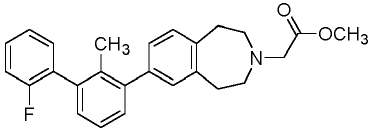 I-H-4 |
| 2-(7-(2-methyl-[1,1'-biphenyl]-3-yl)-1,3,4,5-tetrahydro-2*H*-benzo[c]azepin-2-yl)acetic acid | 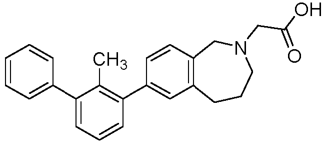 I-H-5 |
| 2-(7-(2,2'-Dimethyl-[1,1'-biphenyl]-3-yl)-1,3,4,5-tetrahydro-2*H*-benzo[c]azepin-2-yl)acetic acid | 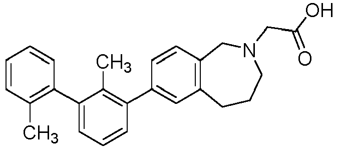 I-H-6 |
| 2-(7-(2'-Chloro-2-methyl-[1,1'-biphenyl]-3-yl)-1,3,4,5-tetrahydro-2*H*-benzo[c]azepin-2-yl)acetic acid | 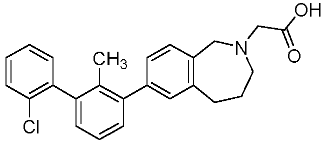 I-H-7 |
| 2-(7-(2'-Fluoro-2-methyl-[1,1'-biphenyl]-3-yl)-1,3,4,5-tetrahydro-2*H*-benzo[c]azepin-2-yl)acetic acid | 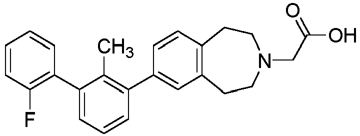 I-H-8 |
| 2-(7-(2-Methyl-[1,1'-biphenyl]-3-yl)-1,3,4,5-tetrahydro-2*H*-benzo[c]azepin-2-yl)ethan-1-ol | 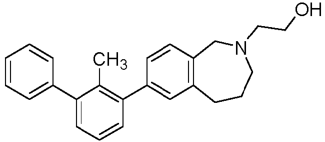 I-H-9 |
| 2-(7-(2,2'-Dimethyl-[1,1'-biphenyl]-3-yl)-1,3,4,5-tetrahydro-2*H*-benzo[c]azepin-2-yl)ethan-1-ol | 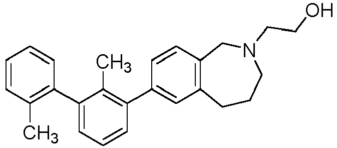 I-H-10 |
| 2-(7-(2'-Chloro-2-methyl-[1,1'-biphenyl]-3-yl)-1,3,4,5-tetrahydro-2*H*-benzo[c]azepin-2-yl)ethan-1-ol | 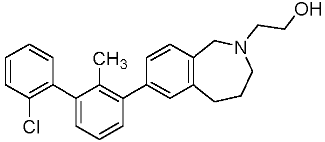 I-H-11 |
| 2-(7-(2'-Fluoro-2-methyl-[1,1'-biphenyl]-3-yl)-1,3,4,5-tetrahydro-2*H*-benzo[c]azepin-2-yl)ethan-1-ol | 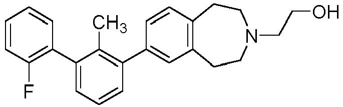 I-H-12 |
| 7-(2-Methyl-[1,1'-biphenyl]-3-yl)-2,3,4,5-tetrahydro-1*H*-benzo[c]azepine | 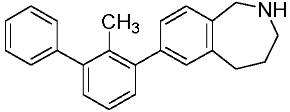 I-H-13 |
| 7-(2,2'-Dimethyl-[1,1'-biphenyl]-3-yl)-2,3,4,5-tetrahydro-1*H*-benzo[c]azepine | 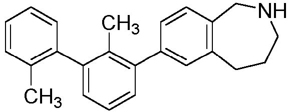 I-H-14 |
| 7-(2'-Chloro-2-methyl-[1,1'-biphenyl]-3-yl)-2,3,4,5-tetrahydro-1*H*-benzo[c]azepine | 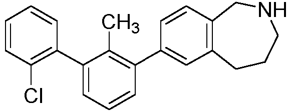 I-H-15 |
| 7-(2'-Fluoro-2-methyl-[1,1'-biphenyl]-3-yl)-2,3,4,5-tetrahydro-1*H*-benzo[d]azepine | 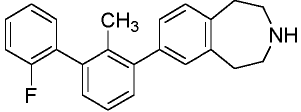 I-H-16 |

The pharmaceutically acceptable salt is an acid addition salt formed by the compound of general formula (I) and hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid or ferulic acid.

Provided is a use of a pyridoheterocycle-containing compound or a pharmaceutically acceptable salt thereof in the preparation of a PD-L1 inhibitor medicament for the treatment of tumors.

The compounds (I) of the present invention are divided into types I-A, I-B, I-C, I-D, I-E and I-F, and their synthesis methods are respectively described as follows:
when Ar represents 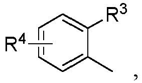 X¹ and X² each independently represent CH, L represents -(CH₂)ₘ-, m=0, T represents C=O, U represents N, V represents CH₂, and n=0, a synthetic route for formula I-A is as follows: 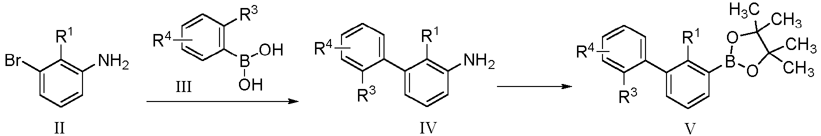 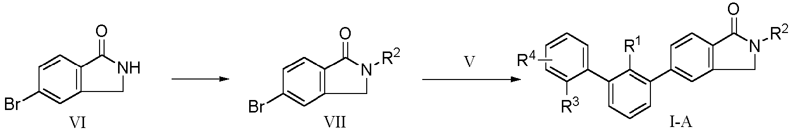 wherein R¹, R², R³ and R⁴ are as previously defined.

A compound IV is prepared from a compound II and a compound III via a Suzuki reaction, wherein a solvent used is selected from toluene, *N,N-*dimethylformamide (DMF), N,N-dimethylacetamide (DMAc), ethylene glycol dimethyl ether, 1,4-dioxane, tetrahydrofuran, methanol, ethanol, acetonitrile, acetone, water or a mixed solvent consisting of any two solvents, preferably a mixed solvent of 1,4-dioxane and water; a base used is selected from sodium ethoxide, sodium acetate, potassium acetate, potassium phosphate, potassium bicarbonate, sodium carbonate, potassium carbonate or triethylamine, preferably potassium carbonate; and a catalyst used is selected from [1,1'-bis(diphenylphosphino)ferrocene]dichloropalladium (Pd(dppf)Cl₂), tetrakis(triphenylphosphine)palladium (Pd(PPh₃)₄), bis(triphenylphosphine)dichloropalladium (Pd(PPh₃)₂CI₂), tris(dibenzylideneindeneacetone)dipalladium (Pd₂(dba)₃) or palladium acetate (Pd(OAc)₂), preferably Pd(PPh₃)₄. The reaction temperature is selected from 50-120°C, preferably 60-100°C.

A compound V is prepared from the compound IV via a Sandmeyer reaction, wherein a diazotizing reagent used is selected from tert-butyl nitrite (t-BuONO), and sodium nitrite, preferably sodium nitrite; a catalyst used is selected from dibenzoyl peroxide (BPO), azobisisobutyronitrile (AIBN), concentrated hydrochloric acid or concentrated sulfuric acid, preferably concentrated hydrochloric acid; a solvent used is selected from tetrahydrofuran, methanol, ethanol, acetonitrile, acetone, water or a mixed solvent consisting of any two solvents, preferably a mixed solvent of methanol and water; and the reaction temperature is selected from -25°C to 80°C, preferably 0-25°C.

A compound VII is prepared from a compound VI, wherein an acid binding agent used is selected from triethylamine, N,N-diisopropylethylamine, sodium carbonate, potassium carbonate, cesium carbonate, sodium hydride, sodium methoxide, sodium ethoxide or potassium tert-butoxide, preferably sodium hydride; a solvent used is selected from dichloromethane, tetrahydrofuran, 1,4-dioxane, acetone, ethyl acetate, DMF or a mixed solvent consisting of any two solvents, preferably tetrahydrofuran or DMF.

A compound I-A is prepared from the compound VII and the compound V via a Suzuki reaction, wherein a solvent used is selected from toluene, DMF, DMAc, ethylene glycol dimethyl ether, 1,4-dioxane, tetrahydrofuran, methanol, ethanol, acetonitrile, acetone, water or a mixed solvent consisting of any two solvents, preferably a mixed solvent of 1,4-dioxane and water; a base used is selected from sodium ethoxide, sodium acetate, potassium acetate, potassium phosphate, potassium bicarbonate, sodium carbonate, potassium carbonate or triethylamine, preferably potassium carbonate; and a catalyst used is selected from Pd(PPh₃)₄, Pd(dppf)Cl₂, Pd(PPh₃)₂Cl₂, Pd(OAc)₂ or Pd₂(dba)₃, preferably Pd(PPh₃)₄. The reaction temperature is selected from 50-120°C, preferably 60-100°C.

When Ar represents 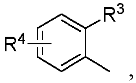 X¹ represents N, X² represents CH, L represents - (CH₂)ₘ-, m=0, T represents CH₂, U represents N, V represents CH₂, and n=1, a synthetic route for formula I-B is as follows: 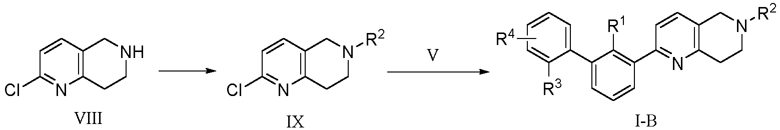 wherein R¹, R², R³ and R⁴ are as previously defined.

A compound IX is prepared from a compound VIII, wherein an acid binding agent used is selected from triethylamine, N,N-diisopropylethylamine, sodium carbonate, potassium carbonate, cesium carbonate, sodium methoxide, sodium ethoxide, potassium tert-butoxide or sodium hydride, preferably sodium hydride; and a solvent used is selected from dichloromethane, tetrahydrofuran, 1,4-dioxane, acetone, ethyl acetate, DMF or a mixed solvent consisting of any two solvents, preferably tetrahydrofuran or DMF.

A compound I-B is prepared from the compound IX and the compound V via a Suzuki reaction, wherein a solvent used is selected from toluene, DMF, DMAc, ethylene glycol dimethyl ether, 1,4-dioxane, tetrahydrofuran, methanol, ethanol, acetonitrile, acetone, water or a mixed solvent consisting of any two solvents, preferably a mixed solvent of 1,4-dioxane and water; a base used is selected from sodium ethoxide, sodium acetate, potassium acetate, potassium phosphate, potassium bicarbonate, sodium carbonate, potassium carbonate or triethylamine, preferably potassium carbonate; and a catalyst used is selected from Pd(PPh₃)₄, Pd(dppf)Cl₂, Pd(PPh₃)₂Cl₂, Pd(OAc)₂ or Pd₂(dba)₃, preferably Pd(PPh₃)₄.

When Ar represents 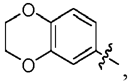 X¹ represents N, X² represents CH, L represents - (CH₂)ₘ-, m=0, T is CH₂, U represents N, V represents CH₂, and n=1, a synthetic route for formula I-C is as follows: 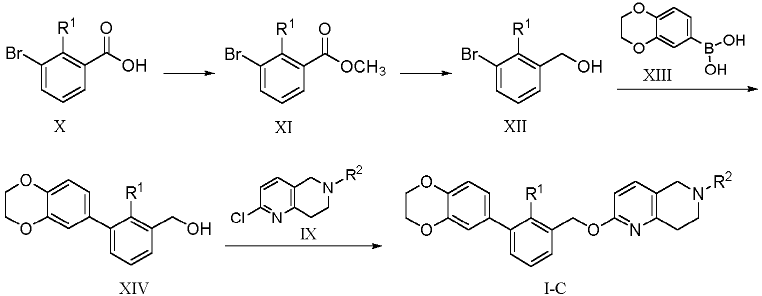 wherein R¹, R², R³ and R⁴ are as previously defined.

A compound X is subjected to a reaction in methanol in the presence of SOCl₂ to prepare XI, wherein the reaction temperature is selected from 50-120°C, preferably 60-100°C.

A compound XII is prepared from the compound XI by reduction, wherein a solvent used is selected from tetrahydrofuran, ethanol, DMF or 1,4-dioxane, preferably methanol; and a reduction agent selected is selected from lithium aluminum hydride, sodium borohydride or potassium borohydride, preferably lithium aluminum hydride.

A compound XIV is prepared from the compound XII and a compound XIII via a Suzuki reaction, wherein a solvent used is selected from toluene, DMF, DMAc, ethylene glycol dimethyl ether, 1,4-dioxane, tetrahydrofuran, methanol, ethanol, acetonitrile, acetone, water or a mixed solvent consisting of any two solvents, preferably a mixed solvent of 1,4-dioxane and water; a base used is selected from sodium ethoxide, sodium acetate, potassium acetate, potassium phosphate, potassium bicarbonate, sodium carbonate, potassium carbonate or triethylamine, preferably potassium carbonate; and a catalyst used is selected from Pd(PPh₃)₄, Pd(dppf)Cl₂, Pd(PPh₃)₂Cl₂, Pd(OAc)₂ or Pd₂(dba)₃, preferably Pd(PPh₃)₄. The reaction temperature is selected from 50-120°C, preferably 60-100°C.

The compound IX reacts with the compound XIV to prepare a compound I-C, wherein a catalyst used is selected from Pd(PPh₃)₄, Pd(dppf)Cl₂, Pd(PPh₃)₂Cl₂, Pd(OAc)₂ or Pd₂(dba)₃, preferably Pd(OAc)₂; a ligand used is selected from triphenylphosphine, 2-di-tert-butylphosphino-2',4',6'-triisopropylbiphenyl (t-BuXPhos), 2-dicyclohexylphosphino-2',4',6'-triisopropylbiphenyl (X-Phos), 4,5-bis(diphenylphosphino)-9,9-dimethylxanthene (Xantphos) or 2-(dicyclohexylphosphino)-3,6-dimethoxy-2'-4'-6'-tri-ipropyl-1,1'-biphenyl (Brett-Phos), preferably t-BuXPhos; a base used is selected from sodium hydroxide, potassium hydroxide, cesium carbonate, potassium carbonate or sodium carbonate, preferably cesium carbonate; and a solvent used is selected from tetrahydrofuran, 1,4-dioxane, toluene or a mixed solvent consisting of any two solvents, preferably toluene. The reaction temperature is selected from 50-120°C, preferably 60-100°C.

When Ar represents 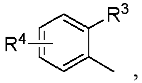 X¹ represents N, X² represents CH, L represents - CH₂O-, T is CH₂, U represents N, V represents CH₂, and n=1, a synthetic route for formula I-D is as follows: 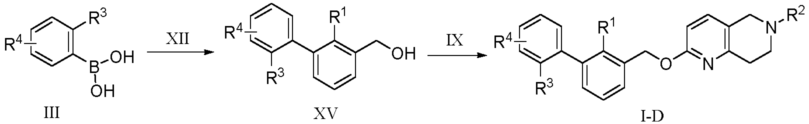 wherein R¹, R², R³ and R⁴ are as previously defined.

A compound XV is prepared from the compound XII and the compound III via a Suzuki reaction, wherein a solvent used is selected from toluene, DMF, DMAc, ethylene glycol dimethyl ether, 1,4-dioxane, tetrahydrofuran, methanol, ethanol, acetonitrile, acetone, water or a mixed solvent consisting of any two solvents, preferably a mixed solvent of 1,4-dioxane and water; a base used is selected from sodium ethoxide, sodium acetate, potassium acetate, potassium phosphate, potassium bicarbonate, sodium carbonate, potassium carbonate or triethylamine, preferably potassium carbonate; and a catalyst used is selected from Pd(PPh₃)₄, Pd(dppf)Cl₂, Pd(PPh₃)₂Cl₂, Pd(OAc)₂ or NiCl₂(dppf), preferably Pd(PPh₃)₄. The reaction temperature is selected from 50-120°C, preferably 60-100°C.

The compound IX reacts with the compound XV to prepare a compound I-D, wherein a catalyst used is selected from Pd(PPh₃)₄, Pd(dppf)Cl₂, Pd(PPh₃)₂Cl₂, Pd(OAc)₂ or Pd₂(dba)₃, preferably Pd(OAc)₂; a ligand used is selected from t-BuXPhos, X-Phos, XantPhos or Brett-Phos, preferably t-BuXPhos; a base used is selected from sodium hydroxide, potassium hydroxide, cesium carbonate, potassium carbonate or sodium carbonate, preferably cesium carbonate; and a solvent used is selected from tetrahydrofuran, 1,4-dioxane, toluene or a mixed solvent consisting of any two solvents, preferably toluene.

When Ar represents 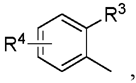 X¹ represents N, X² represents CH, L represents - CH₂O-, T represents CH₂, U represents N, V represents NCH₃, and n=1, a synthetic route for formula I-E is as follows: 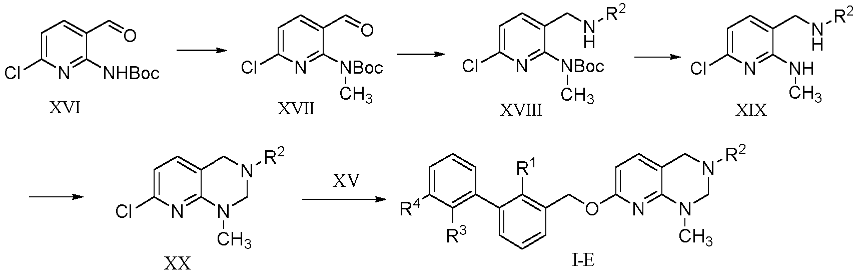 wherein R¹, R², R³ and R⁴ are as previously defined.

A compound XVII is prepared from a compound XVI and methyl iodide under basic conditions, wherein a solvent used is selected from acetone, DMF, acetonitrile or tetrahydrofuran, or a mixed solvent consisting of the above solvents, preferably DMF; and a base used is selected from sodium hydride, sodium methoxide, sodium ethoxide, potassium tert-butoxide, potassium carbonate, sodium carbonate, cesium carbonate, sodium hydroxide, potassium hydroxide, or lithium hydroxide, preferably sodium hydride.

A target compound XVIII is prepared from the compound XVII and an amine compound by reductive amination, wherein a solvent used is selected from toluene, DMF, dichloromethane, dichloroethane, trichloromethane, methanol, 1,4-dioxane, tetrahydrofuran, ethanol, acetonitrile, acetone, or a mixed solvent consisting of the above solvents, preferably a mixed solvent consisting of dichloromethane and methanol; and a reduction agent used is selected from sodium triacetoxyborohydride, sodium cyanoborohydride, sodium borohydride or sodium dithionite, preferably sodium triacetoxyborohydride.

A target compound XIX is prepared from the compound XVIII through a reaction under acidic conditions, wherein a solvent used is selected from ethyl acetate, acetone, dichloromethane, acetonitrile, tetrahydrofuran or a mixed solvent consisting of the above solvents, preferably ethyl acetate; and an acid used is selected from an ethyl acetate solution saturated with hydrogen chloride, a 1,4-dioxane solution saturated with hydrogen chloride, hydrochloric acid, trifluoroacetic acid or trifluoromethanesulfonic acid, preferably the ethyl acetate solution saturated with hydrogen chloride.

The compound XIX reacts with paraformaldehyde to prepare a target compound XX, wherein a solvent used is selected from methanol, ethanol, ethyl acetate, acetone, dichloromethane, DMF, acetonitrile, tetrahydrofuran or a mixed solvent consisting of the above solvents, preferably methanol; and a base used is selected from triethylamine, dimethylaminopyridine (DMAP) or *N,N-*diisopropylethylamine, preferably triethylamine.

The compound XX reacts with the compound XV to prepare a compound I-E, wherein a catalyst used is selected from Pd(PPh₃)₄, Pd(dppf)Cl₂, Pd(PPh₃)₂Cl₂, Pd(OAc)₂ or Pd₂(dba)₃, preferably Pd(OAc)₂; a ligand used is selected from t-BuXPhos, X-Phos, XantPhos or Brett-Phos, preferably *t*-BuXPhos; a base used is selected from sodium hydroxide, potassium hydroxide, cesium carbonate, potassium carbonate or sodium carbonate, preferably cesium carbonate; and a solvent used is selected from tetrahydrofuran, 1,4-dioxane, toluene or a mixed solvent consisting of any two solvents, preferably toluene. The reaction temperature is selected from 50-120°C, preferably 60-100°C.

When Ar represents 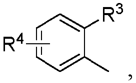 X¹ represents N, X² represents CH, L represents - NHCO-, T represents CH₂, U represents N, V represents CH₂, and n=1, a synthetic route for formula I-F is as follows: 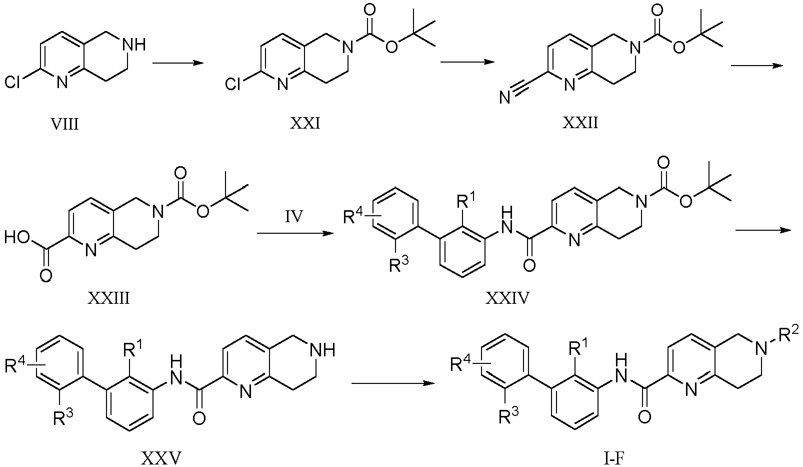 wherein R¹, R², R³ and R⁴ are as previously defined.

A compound XXI is prepared from the compound VIII and di-tert-butyl dicarbonate under basic conditions, wherein a solvent used is selected from acetone, dichloromethane, DMF, acetonitrile, tetrahydrofuran or a mixed solvent consisting of the above solvents, preferably tetrahydrofuran; a base used is selected from DMAP, triethylamine, *N,N*-diisopropylethylamine, potassium tert-butoxide, sodium hydride, sodium methoxide, sodium ethoxide, potassium carbonate, sodium carbonate, cesium carbonate, sodium hydroxide, potassium hydroxide, sodium bicarbonate, potassium bicarbonate or lithium hydroxide, preferably triethylamine.

A compound XXII is prepared from the compound XXI, wherein a solvent used is selected from acetone, dichloromethane, DMF, acetonitrile, tetrahydrofuran or a mixed solvent consisting of the above solvents, preferably DMF; a cyano donor is selected from zinc cyanide, cuprous cyanide or potassium ferrocyanide, preferably zinc cyanide; and a catalyst used is selected from Pd(dppf)Cl₂, Pd(PPh₃)₄, Pd(PPh₃)₂Cl₂, Pd₂(dba)₃ or Pd(OAc)₂, preferably Pd(PPh₃)₄. The reaction temperature is selected from 50-150°C, preferably 80-120°C.

A compound XXIII is prepared from the compound XXII by a hydrolysis reaction under basic conditions, wherein a solvent used is selected from methanol, ethanol, water, acetonitrile, tetrahydrofuran, acetone, dichloromethane, DMF, acetonitrile or a mixed solvent consisting of the above solvents, preferably a mixed solvent of ethanol and water; and a base used is selected from potassium carbonate, sodium carbonate, cesium carbonate, sodium hydroxide, potassium hydroxide or lithium hydroxide, preferably potassium hydroxide. The reaction temperature is selected from 0-120°C, preferably 60-100°C.

The compound IV reacts with the compound XXIII to prepare compound XXIV, wherein a condensation agent used is selected from carbonyldiimidazole (CDI), dicyclohexylcarbodiimide (DCC), diisopropylcarbodiimide (DIC), 1-(3-dimethylaminopropyl)-3-ethylcarbodiimide (EDCI), 2-(7-azabenzotriazole)-N,N,N',N'-tetramethyluronium hexafluorophosphate (HATU) or benzotriazol-1-yl-oxytripyrrolidinophosphonium hexafluorophosphate (PyBOP), preferably HATU; an acid binding agent is selected from N,N-diisopropylethylamine or triethylamine, preferably N,N-diisopropylethylamine; a solvent used is selected from acetone, dichloromethane, DMF, acetonitrile, tetrahydrofuran or a mixed solvent consisting of the above solvents, preferably DMF; and the reaction temperature is selected from 0-80°C, preferably 25-50°C.

A target compound XXV is prepared from the compound XXIV through a reaction under acidic conditions, wherein a solvent used is selected from ethyl acetate, acetone, dichloromethane, acetonitrile, tetrahydrofuran or a mixed solvent consisting of the above solvents, preferably ethyl acetate; and an acid used is selected from an ethyl acetate solution saturated with hydrogen chloride, a 1,4-dioxane solution saturated with hydrogen chloride, hydrochloric acid, trifluoroacetic acid or trifluoromethanesulfonic acid, preferably the ethyl acetate solution saturated with hydrogen chloride.

A compound I-F is prepared from the compound XXV, wherein an acid binding agent used is selected from triethylamine, N,N-diisopropylethylamine, sodium carbonate, potassium carbonate, cesium carbonate or sodium hydride, preferably sodium hydride; and a solvent used is selected from dichloromethane, tetrahydrofuran, 1,4-dioxane, acetone, ethyl acetate, DMF or a mixed solvent of any two solvents, preferably tetrahydrofuran or DMF.

The present invention also discloses a pharmaceutical composition, including the compound of general formula (I) as described above (including chiral isomers), or a pharmaceutically acceptable salt thereof, and a pharmaceutically acceptable carrier. The compounds can be prepared into conventional pharmaceutical preparations such as tablets, capsules, syrups, suspensions and injections by the addition of the pharmaceutically acceptable carrier, and commonly used pharmaceutical excipients such as flavors, sweeteners, liquid or solid fillers or diluents.

Also within the protection scope of the present invention is a use of the compound of general formula (I) described in the present invention and a hydrate, solvate or crystal thereof in the preparation of a PD-1/PD-L1 protein-protein interaction inhibitor medicament.

Further, the PD-1/PD-L1 protein-protein interaction inhibitor may be used in the treatment of cancers or tumors, for example, in the preparation of a medicament for the treatment of cancers such as non-small cell lung cancer, colon cancer, melanoma, breast cancer, and liver cancer.

Pharmacological experiments showed that the pyridoheterocycle derivative of the present invention can exert good inhibitory effects on the PD-1/PD-L1 interaction in a homogeneous time-resolved fluorescence (HTRF) assay. The pyridoheterocyclic derivative of the present invention is excellent in activity, and therefore the development of biphenyl inhibitors of PD-1/PD-L1 has significant practical significance and potential application prospects.

Beneficial effects: compared with the prior art, the present invention has the following significant advantages:
(1) The novel pyridoheterocycle derivative of the present invention can significantly inhibit the PD-1/PD-L1 interaction and has an activity better than that of a known PD-1/PD-L1 inhibitor BMS-202.
(2) The synthetic route of the pyridoheterocycle derivative of the present invention is skillful in design, and simple and easy to implement, the raw materials are inexpensive and readily available, and the synthesis process is safe, environmentally friendly, and easy for mass production.
(3) The pyridoheterocycle derivative of the present invention has wide applicability, and can be used in the treatment of various cancers or tumors related to the immune checkpoint PD-1/PD-L1 as an active ingredient of a medicament.

### BRIEF DESCRIPTION OF THE DRAWINGS

FIG. 1 shows a body weight growth curve of rats in a toxicity test during continuous administration of the compounds of the present invention.
FIG. 2 shows an animal tumor growth curve of a 4T1 subcutaneously grafted tumor model of BALB/c mice administrated with the compounds of the present invention.

### DETAILED DESCRIPTION

### Example 1

Synthesis of ethyl 2-(5-(2'-fluoro-2-methyl-[1,1'-biphenyl]-3-yl)-1-oxoisoindolin-2-yl)acetate **(I-A-1:** R¹=CH₃, R²= 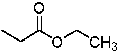 , R³=F, R⁴=H, and T represents C=O)

### Synthesis of 2'-fluoro-2-methyl-[1,1'-biphenyl]-3-amine (IV-1)

3-Bromo-2-methylaniline **II-1** (2.50 g, 13.44 mmol), 2-fluorobenzeneboronic acid **III-1** (2.26 g, 16.15 mmol) and 1,4-dioxane (25 mL) were sequentially added into a three-necked flask, potassium carbonate (5.21 g, 37.69 mmol) dissolved in water (2.5 mL) was added into the above reaction solution, Pd(PPh₃)₄ (0.39 g, 0.34 mmol) was added, and under nitrogen protection, the temperature was raised to 80°C, and a reaction was carried out for 10 h. After the raw materials were monitored by TLC to be completely reacted, heating was stopped, and the resulting reaction solution was cooled to room temperature. A palladium catalyst and an insoluble substance were removed by suction filtration, diluting was performed with water (25 mL), the reaction solution was extracted with ethyl acetate (25 mL×3), and organic phases were mixed, washed with a saturated aqueous NaCl solution (25 mL×3), and dried over anhydrous magnesium sulfate. Suction filtration was performed, a solvent was removed through distillation under reduced pressure, and a residue was purified by column chromatography (petroleum ether:ethyl acetate=20:1-15:1) to give 2.59 g of a yellow solid (yield: 95.6%). m.p.61.0-62.0°C.

¹H NMR (300 MHz, DMSO-*d*₆) δ 7.48-7.35 (m, 1H, ArH), 7.34-7.22 (m, 3H, ArH), 6.98 (t, J = 7.7 Hz, 1H, ArH), 6.70 (dd, J = 8.0 Hz, 1.4 Hz, 1H, ArH), 6.42 (dd, J = 7.5 Hz, 1.3 Hz, 1H, ArH), 5.00 (s, 2H, NH₂), 1.85 (s, 3H, CH₃).

### Synthesis of 2-(2'-fluoro-2-methyl-[1,1'-biphenyl]-3-yl)-4,4,5,5-tetramethyl-1,3,2-dioxaborolane (V-1)

The compound **IV-1** (2.00 g, 9.94 mmol) was dissolved in methanol (20 mL) and water (10 mL), and a solution of hydrochloric acid (9.92 mL, 3 mmol/mL) was slowly added dropwise. After stirring at 25°C until the reaction solution was clear, the temperature was reduced to 0°C, and an aqueous solution of NaNO₂ (4.96 mL, 2.2 mmol/mL) was added slowly dropwise. After dropwise addition was completed, stirring was continued to be performed at 0°C for 30 min, bis(pinacolato)diboron (7.56 g, 29.85 mmol) dissolved in methanol (20 mL) was slowly added dropwise into the reaction solution, a large amount of gas was generated, and stirring was continued to be performed at room temperature for 2 h after dropwise addition was completed. After the raw materials were monitored by TLC (petroleum ether:ethyl acetate=30:1) to be completely reacted, the reaction solution was extracted with dichloromethane (20 mL×2), and organic phases were mixed, washed with a saturated sodium chloride solution (20 mL×2), and dried over anhydrous sodium sulfate. Suction filtration was performed, a solvent was removed through distillation under reduced pressure, and a residue was subjected to column chromatography (petroleum ether:ethyl acetate=200:1) to give 1.88 g of a yellow solid (yield: 60.4%). m.p.94.0-95.0°C.

¹H NMR (300 MHz, DMSO-*d*₆) δ 7.70 (dd, J = 5.4 Hz, 3.7 Hz, 1H, ArH), 7.53-7.36 (m, 1H, ArH), 7.34-7.25 (m, 5H, ArH), 2.27 (s, 3H, ArCH₃), 1.31 (s, 12H, CH₃).

### Synthesis of 5-(2'-fluoro-2-methyl-[1,1'-biphenyl]-3-yl)isoindol-1-one (VII)

A compound **V** (0.50 g, 2.36 mmol), a compound **VI** (0.74 g, 2.48 mmol) and 1,4-dioxane (10 mL) were sequentially added into a three-necked flask, potassium carbonate (0.92 g, 6.60 mmol) was dissolved in water (1.0 mL) to be added into the reaction solution, and under nitrogen protection, Pd(PPh₃)₄ (0.27 g, 0.25 mmol) was added. The reaction solution was heated to 80°C, and a reaction was carried out for 12 h. After the raw materials were monitored by TLC (petroleum ether:ethyl acetate=4:1) to be completely reacted, heating was stopped, and the resulting reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated aqueous NaCl solution (10 mL×3), and dried over anhydrous magnesium sulfate. Suction filtration was performed, a solvent was removed through distillation under reduced pressure, and a residue was purified by column chromatography (petroleum ether:ethyl acetate=15:1-3:1) to give 0.40 g of a white solid powder (yield: 50.9%). m.p.190-192°C.

¹H NMR (300 MHz, DMSO-*d*₆) δ 8.63 (s, 1H, ArH), 7.76 (d, J = 7.8 Hz, 1H, ArH), 7.60 (s, 1H, ArH), 7.49 (d, J = 7.6 Hz, 2H, ArH), 7.44-7.26 (m, 6H, ArH), 4.46 (s, 2H, CH₂), 2.01 (s, 3H, CH₃).

### Synthesis of ethyl 2-(5-(2'-fluoro-2-methyl-[1,1'-biphenyl]-3-yl)-1-oxoisoindolin-2-yl)acetate (I-A-1)

A compound **VII** (0.50 g, 1.58 mmol) was added into a 25 mL eggplant shaped flask and dissolved in THF, and NaH (0.12 g, 3.16 mmol) was added at 0°C. Ethyl bromoacetate (0.32 g, 1.89 mmol) was added dropwise into the mixture. A reaction was carried out at room temperature, after the raw materials were monitored to be completely reacted, the reaction was quenched with a saturated ammonium chloride solution, the resulting reaction solution was extracted with EA (10 mL×3), washing was performed with a saturated salt solution (10 mL×3), drying was performed by using anhydrous sodium sulfate, and separation and purification were performed by column chromatography to give 0.63 g of a white solid (yield: 98.7%). m.p.100-101°C.

¹H NMR (300 MHz, DMSO-*d*₆) δ 7.81 (d, J = 7.8 Hz, 1H, ArH), 7.67-7.62 (m, 1H, ArH), 7.57-7.47 (m, 2H, ArH), 7.43-7.30 (m, 6H, ArH), 4.61 (s, 2H, NCH₂), 4.44 (s, 2H, NCH₂), 4.19 (q, J = 7.1 Hz, 2H, CH₂CH₃), 2.03 (s, 3H, CH₃), 1.25 (t, J = 7.1 Hz, 3H, ArCH₃).

### Example 2

Synthesis of ethyl 3-(5-(2'-fluoro-2-methyl-[1,1'-biphenyl]-3-yl)-1-oxoisoindolin-2-yl)propanoate **(I-A-2:** R¹=CH₃, R²= 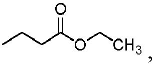 R³=F, R⁴=H, and T represents C=O)
0.40 g of a colorless viscous liquid was obtained by using a compound **VII** (0.50 g, 1.58 mmol) and ethyl 3-bromopropionate (0.34 g, 1.89 mmol) as raw materials and by using the same procedures as those for the compound **I-A-1** (yield: 60.6%).

¹H NMR (300 MHz, DMSO-*d*₆) δ 7.77 (d, J = 7.7 Hz, 1H, ArH), 7.67-7.62 (m, 1H, ArH), 7.54-7.46 (m, 2H, ArH), 7.45-7.26 (m, 6H, ArH), 4.58 (s, 2H, NCH₂), 4.10 (q, J = 7.1 Hz, 2H, CH₂CH₃), 3.82 (t, J = 7.0 Hz, 2H, NCH₂CH₂), 2.73 (t, J = 7.0 Hz, 2H, COCH₂), 2.01 (s, 3H, ArCH₃), 1.19 (t, J = 7.1 Hz, 3H, CH₂CH₃).

### Example 3

Synthesis of ethyl 2-(5-(2'-fluoro-2-methyl-[1,1'-biphenyl]-3-yl)-1-oxoisoindolin-2-yl)propanoate **(I-A-3:** R¹=CH₃, R²= 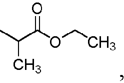 R³=F, R⁴=H, and T represents C=O)
0.58 g of a white solid was obtained by using a compound **VII** (0.50 g, 1.58 mmol) and ethyl 2-bromopropionate (0.34 g, 1.89 mmol) as raw materials and by using the same procedures as those for the compound **I-A-1** (yield: 88.3%). m.p.50-52°C.

¹H NMR (300 MHz, DMSO-*d*₆) δ 7.80 (d, J = 7.8 Hz, 1H, ArH), 7.68-7.63 (m, 1H, ArH), 7.56-7.45 (m, 2H, ArH), 7.34-7.21 (m, 6H, ArH), 4.97 (q, J = 7.3 Hz, 1H, CH₃CH), 4.62 (d, J = 7.0 Hz, 2H, NCH₂), 4.16 (q, J = 7.1 Hz, 2H, CH₂CH₃), 2.02 (s, 3H, ArCH₃), 1.56 (d, J = 7.4 Hz, 3H, CHCH₃), 1.22 (t, J = 7.0 Hz, 3H, CH₂CH₃).

### Example 4

Synthesis of 2-(5-(2'-fluoro-2-methyl-[1,1'-biphenyl]-3-yl)-1-oxoisoindolin-2-yl)acetic acid (I-A-4: R¹=CH₃, R²= 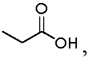 R³=F, R⁴=H, and T represents C=O)

The compound **I-A-1** (30 mg, 0.16 mmol) was added into a 10 mL eggplant shaped flask, methanol (2 mL) was added to dissolve the compound **I-A-1,** a solution of LiOH (8 mg, 0.20 mmol) in water (0.5 mL) was added, and stirring was performed at room temperature for 4 h. After the reaction was monitored by TLC to be complete, the methanol was removed through distillation under reduced pressure, a pH was adjusted to 5-6 with a 2M HCl solution, so that a white solid was precipitated, and suction filtration was performed to give 20 mg of an off-white solid product (yield: 72.1%). m.p.164-166°C.

¹H NMR (400 MHz, DMSO-*d*₆) δ 12.95 (s, 1H, OH), 7.82-7.74 (m, 1H, ArH), 7.66-7.58 (m, 1H, ArH), 7.53-7.43 (m, 2H, ArH), 7.41-7.23 (m, 6H, ArH), 4.58 (s, 2H, NCH₂), 4.32 (s, 2H, NCH₂), 2.00 (s, 3H, CH₃).

HRMS (ESI): m/z [M+H]⁺ Calcd for C₂₃H₁₉FNO₃: 376.1349; found: 376.1350.

### Example 5

Synthesis of 3-(5-(2'-fluoro-2-methyl-[1,1'-biphenyl]-3-yl)-1-oxoisoindolin-2-yl)propanoic acid **(I-A-5:** R¹=CH₃, R²= 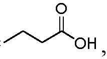 R³=F, R⁴=H, and T represents C=O)
40 mg of a yellow solid product was obtained by using the compound I-A-2 (50 mg, 0.12 mmol) and a solution of LiOH (10 mg, 0.24 mmol) in water (0.5 mL) as raw materials and by using the same procedures as those for the compound **I-A-4** (yield: 92.2%). m.p. 56-58°C.

¹H NMR (300 MHz, DMSO-*d*₆) δ 7.77 (d, J = 7.8 Hz, 1H, ArH), 7.63 (s, 1H, ArH), 7.50 (d, J = 7.7 Hz, 2H, ArH), 7.44-7.28 (m, 6H, ArH), 4.58 (s, 2H, NCH₂), 3.78 (t, J = 6.9 Hz, 2H, NCH₂CH₂), 2.65 (t, J = 7.0 Hz, 2H, COCH₂), 2.01 (s, 3H, CH₃).

HRMS (ESI): m/z [M+H]⁺ Calcd for C₂₄H₂₁FNO₃: 390.1505; found: 390.1503.

### Example 6

Synthesis of 2-(5-(2'-fluoro-2-methyl-[1,1'-biphenyl]-3-yl)-1-oxoisoindolin-2-yl)propanoic acid **(I-A-6:** R¹=CH₃, R²= 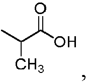 R³=F, R⁴=H, and T represents C=O)

30 mg of a white solid product was obtained by using the compound I-A-3 (50 mg, 0.12 mmol) and a solution of LiOH (10 mg, 0.24 mmol) in water (0.5 mL) as raw materials and by using the same procedures as those for the compound **I-A-4** (yield: 64.2%). m.p.140-142°C.

¹H NMR (300 MHz, DMSO-*d*₆) δ 7.78 (d, J = 7.8 Hz, 1H, ArH), 7.65 (s, 1H, ArH), 7.54-7.46 (m, 2H, ArH), 7.42-7.29 (m, 6H, ArH), 4.92-4.78 (m, 1H, CHCH₃), 4.72-4.52 (m, 2H, CH₂), 2.02 (s, 3H, ArCH₃), 1.51 (d, J = 7.3 Hz, 3H, CH₃).

HRMS (ESI): m/z [M+H]⁺ Calcd for C₂₄H₂₁FNO₃: 390.1505; found: 390.1502.

### Example 7

Synthesis of 5-(2'-fluoro-2-methyl-[1,1'-biphenyl]-3-yl)-2-(2-hydroxyethyl)isoindol-1-one **(I-A-7:** R¹=CH₃, R²= 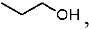 R³=F, R⁴=H, and T represents C=O)
The compound **I-A-1** (60 mg, 0.12 mmol) was added into a 25 mL eggplant shaped flask and was dissolved in THF, and lithium aluminum hydride (8 mg, 0.24 mmol) was added slowly at 0°C. After the raw materials were monitored to be completed reacted, the reaction was quenched with a saturated ammonium chloride solution, so that a white solid was precipitated. The white solid was removed by suction filtration, and washing was performed with EA. A filtrate was concentrated, and separated and purified to give 24 mg of a yellow solid (yield: 56.8%). m.p. 86-88°C.

¹H NMR (300 MHz, DMSO-*d*₆) δ 7.77 (d, J = 7.8 Hz, 1H, ArH), 7.63 (s, 1H, ArH), 7.52-7.45 (m, 2H, ArH), 7.40-7.25 (m, 6H, ArH), 4.90 (brs, 1H, OH), 4.63 (s, 2H, NCH₂), 3.68-3.60 (m, 4H, CH₂CH₂), 2.01 (s, 3H, CH₃).

HRMS (ESI): m/z [M+H]⁺ Calcd for C₂₃H₂₁FNO₂: 362.1556; found: 362.1556.

### Example 8

Synthesis of 2-(5-(2'-fluoro-2-methyl-[1,1'-biphenyl]-3-yl)-1-oxoisoindolin-2-yl)acetamide **(I-A-8:** R¹=CH₃, R²= 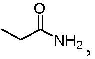 R³=F, R⁴=H, and T represents C=O)

The compound **I-A-2** (100 mg, 0.27 mmol) was added into a 25 mL three-necked flask and dissolved in THF, a small amount of DMF was added dropwise, sulfoxide chloride (95 mg, 0.78 mmol) was added slowly dropwise, the temperature was raised to 55°C, a reaction was carried out, after the raw materials were monitored to be completely reacted, the temperature was cooled to room temperature, the three-necked flask was transferred to -20°C, ammonia water was added slowly dropwise to adjust a pH to be 8, after the raw materials were monitored to be completely reacted, and a new spot was formed, water (10 mL) was added, the resulting reaction solution was extracted with EA (10 mL×3), washing was performed with a saturated salt solution (10 mL×3), drying was performed by using anhydrous sodium sulfate, and separation and purification were performed by column chromatography to give 42 mg of a light brown solid (yield: 42.0%). m.p. higher than 250°C.

¹H NMR (300 MHz, DMSO-*d*₆) δ 7.77 (d, J = 7.8 Hz, 1H, ArH), 7.61 (d, J = 7.6 Hz, 1H, ArH), 7.52-7.45 (m, 2H, ArH), 7.40-7.25 (m, 6H, ArH), 4.57 (s, 2H, NCH₂), 4.16 (s, 2H, NCH₂CO), 2.00 (s, 3H, CH₃).

HRMS (ESI): m/z [M+H]⁺ Calcd for C₂₃H₂₀FN₂O₂: 375.1509; found: 375.1511.

Example 9

Synthesis of 2-(5-(2'-fluoro-2-methyl-[1,1'-biphenyl]-3-yl)isoindolin-2-yl)ethan-1-ol **(I-A-9:** R¹=CH₃, R²= 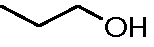 , R³=F, R⁴=H, and T represents CH₂)

The compound **I-A-7** (30 mg, 0.08 mmol) was added into a 25 mL eggplant shaped flask and dissolved in THF, and a 1M borane-tetrahydrofuran complex (0.46 mL, 0.48 mmol) was added slowly at 0°C. After the addition, a reaction was carried out under reflux at 68°C for 3h. After the raw materials were monitored to be completely reacted, the reaction was quenched with a saturated ammonium chloride solution, so that a white solid was precipitated. The white solid was removed by suction filtration, and washing was performed with EA. A filtrate was concentrated, and separated and purified to give 14 mg of a brown solid (yield: 48.5%). m.p. 80-82°C.

¹H NMR (300 MHz, Chloroform-*d*) δ 7.36-7.29 (m, 4H, ArH), 7.25-7.13 (m, 6H, ArH), 4.25 (s, 4H, 2NCH₂), 3.86-3.80 (m, 2H, CH₂OH), 3.14-3.08 (m, 2H, CH₂CH₂OH), 2.05 (s, 3H, CH₃).

HRMS (ESI): m/z [M+H]⁺ Calcd for C₂₃H₂₃FNO: 348.1764; found: 348.1762.

### Example 10

Synthesis of ethyl 2-(2-(2'-fluoro-2-methyl-[1,1'-biphenyl]-3-yl)-7,8-dihydro-1,6-naphthyridin-6(5*H*)-yl)acetate **(I-B-1:** R¹=CH₃, R²= 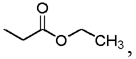 R³=F, and R⁴=H)

### Ethyl 2-(2-chloro-7,8-dihydro-1,6-naphthyridin-6(5H)-yl)acetate (IX-1)

2-Chloro-5,6,7,8-tetrahydro-1,6-naphthyridine hydrochloride VIII (0.50 g, 2.43 mmol) was added into a 25 mL eggplant shaped flask and was dissolved in THF. NaH (0.20 g, 4.86 mmol) was added at 0°C. Ethyl bromoacetate (0.49 g, 2.93 mmol) was added dropwise into the reaction solution. A reaction was carried out at room temperature, after the raw materials were monitored by TLC to be completely reacted, the reaction was quenched with a saturated ammonium chloride solution, the resulting reaction solution was extracted with ethyl acetate (10 mL×3), washing was performed with a saturated salt solution (10 mL×3), drying was performed by using anhydrous sodium sulfate, suction filtration was performed, and a solvent was removed through distillation under reduced pressure. A residue was separated and purified by column chromatography to give 0.60 g of a light brown solid (yield: 97.2%). m.p.58-60°C.

¹H NMR (300 MHz, Chloroform-*d*) δ 7.45-7.37 (m, 5H, ArH), 7.28-7.22 (m, 4H, ArH), 4.24 (q, J = 7.1 Hz, 2H, CH₂CH₃), 3.82 (s, 2H, NCH₂), 3.47 (s, 2H, COCH₂), 3.09-3.05 (m, 2H, NCH₂CH₂), 3.02-2.97 (m, 2H, NCH₂CH₂), 1.31 (t, J = 7.2 Hz, 3H, CH₃).

### Synthesis of ethyl 2-(2-(2'-fluoro-2-methyl-[1,1'-biphenyl]-3-yl)-7,8-dihydro-1,6-naphthyridin-6(5H)-yl)acetate (I-B-1)

The compound **IX-1** (0.25 g, 0.98 mmol), the compound **V-1 (10,** 0.44 g, 1.47 mmol) and 1,4-dioxane (5 mL) were sequentially added to a three-necked flask. Potassium carbonate (0.37 g, 2.75 mmol) was dissolved in water (0.5 mL) to be added into the reaction solution. Under nitrogen protection, Pd(PPh₃)₄ (0.14 g, 0.10 mmol) was added, the temperature was raised to 80°C, and a reaction was carried out for 12 h. After the raw materials were monitored by TLC (petroleum ether:ethyl acetate=8:1) to be completely reacted, heating was stopped, and the reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated salt solution (10 mL×3), and dried over anhydrous magnesium sulfate. Suction filtration was performed, a solvent was removed through distillation under reduced pressure, and a residue was purified by column chromatography (petroleum ether:ethyl acetate=20:1-10:1) to give 180 mg of a yellow solid (yield: 44.6%). m.p.46-48°C.

¹H NMR (300 MHz, Chloroform-*d*) δ 7.46-7.37 (m, 5H, ArH), 7.28-7.22 (m, 4H, ArH), 4.29 (q, J = 7.2 Hz, 2H, CH₂CH₃), 3.95 (s, 2H, NCH₂), 3.55 (s, 2H, COCH₂), 3.23 (t, J = 5.9 Hz, 2H, NCH₂CH₂), 3.11 (t, J = 5.9 Hz, 2H, NCH₂CH₂), 2.10 (s, 3H, ArCH₃), 1.36 (t, J = 7.2 Hz, 3H, CH₃).

### Example 11

Synthesis of 2-(2-(2'-fluoro-2-methyl-[1,1'-biphenyl]-3-yl)-7,8-dihydro-1,6-naphthyridin-6(5*H*)-yl)acetic acid **(I-B-2:** R¹=CH₃, R²= 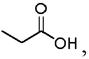 R³=F, and R⁴=H)

The compound **I-B-1** (80 mg, 0.20 mmol) was added into a 10 mL eggplant shaped flask and was dissolved in ethanol (2 mL), a solution of LiOH (17 mg, 0.40 mmol) in water (0.3 mL) was added, and the mixture was stirred at room temperature for 4 h. After the reaction was monitored by TLC to be complete, ethanol was removed through distillation under reduced pressure, a pH was adjusted to 5-6 with a 2M HCl solution, so that a white solid was precipitated, suction filtration was performed, and drying was performed to give 7 mg of a yellow solid product (yield: 9.4%). m.p.188-190°C.

¹H NMR (300 MHz, DMSO-*d*₆) δ 8.29 (d, J = 8.0 Hz, 1H, ArH), 7.58 (d, J = 8.0 Hz, 1H, ArH), 7.48-7.29 (m, 7H, ArH), 4.23 (s, 2H, NCH₂), 3.74 (t, J = 6.7 Hz, 2H, NCH₂), 3.26 (s, 2H, COCH₂), 3.19 (t, J = 6.4 Hz, 2H, NCH₂), 2.08 (s, 3H, CH₃).

HRMS (ESI): m/z [M+H]⁺ Calcd for C₂₃H₂₂FN₂O₂: 377.1665; found: 377.1667.

### Example 12

Synthesis of 2-(2-(2'-fluoro-2-methyl-[1,1'-biphenyl]-3-yl)-7,8-dihydro-1,6-naphthyridin-6(5*H*)-yl)ethan-1-ol **(I-B-3:** R¹=CH₃, R²= 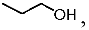 R³=F, and R⁴=H)

The compound **I-B-1** (100 mg, 0.25 mmol) was added into a 25 mL eggplant shaped flask and was dissolved in 3 mL of THF, and lithium aluminum hydride (19 mg, 0.50 mmol) was added slowly at 0°C. After the raw materials were monitored to be completely reacted, the reaction was quenched with a saturated ammonium chloride solution, and a white solid was precipitated. The white solid was removed by suction filtration, and washing was performed with EA. A filtrate was concentrated, and separated and purified to give 50 mg of a yellow solid (yield: 27.9%). m.p.66-68°C.

¹H NMR (300 MHz, Chloroform-*d*) δ 7.43-7.38 (m, 1H, ArH), 7.36-7.29 (m, 3H, ArH), 7.24-7.11 (m, 5H, ArH), 3.79 (s, 2H, NCH₂), 3.56 (t, J = 5.4 Hz, 2H, CH₂OH), 3.13 (t, J = 5.6 Hz, 2H, NCH₂CH₂), 2.98 (t, J = 5.8 Hz, 2H, NCH₂CH₂), 2.80 (t, J = 5.3 Hz, 2H, NCH₂), 2.10 (s, 3H, CH₃).

HRMS (ESI): m/z [M+H]⁺ Calcd for C₂₃H₂₃FN₂O: 363.1873; found: 363.1872.

### Example 13

Synthesis of ethyl 2-(2-((3-(2,3-dihydrobenzo[*b*][1,4]dioxin-6-yl)-2-methylbenzyl)oxy)-7,8-dihydro-1,6-naphthyridin-6(5H)-yl)acetate **(I-C-1:** R¹=CH₃, and R²= 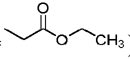 )

### Synthesis of methyl 3-bromo-2-methylbenzoate (XI-1)

3-Bromo-2-methylbenzoic acid **X-1** (0.30 g, 1.40 mmol) and methanol (5 mL) were added into a 50 mL eggplant shaped flask. Thionyl chloride (0.20 mL, 2.79 mmol) was added slowly dropwise under an ice bath. After the addition, a reflux reaction was carried out under heating at 70°C for 1 h. After the raw materials were monitored by TLC (petroleum ether:ethyl acetate=8:1) to be completely reacted, concentration was performed under reduced pressure to give 0.32 g of a white solid powder (yield: 99.0%). m.p.31.0-33.0°C.

¹H NMR (300 MHz, Chloroform-*d*) δ 7.72 (d, J = 8.5 Hz, 1H, ArH), 7.68 (d, J = 8.6 Hz, 1H, ArH), 7.09 (t, J = 7.8 Hz, 1H, ArH), 3.90 (s, 3H, OCH₃), 2.63 (s, 3H, CH₃).

### Synthesis of (3-bromo-2-methylphenyl)methanol (XII-1)

The compound **XI-1** (10.50 g, 46.10 mmol) and anhydrous tetrahydrofuran (50.00 mL) was added into a three-necked flask, under N₂ protection, the temperature was reduced to 0°C, LiAIH₄ (3.10 g, 55.30 mmol) was added slowly in batches, after the addition, an ice bath was removed, and the mixture was stirred at room temperature for 30 min. After the raw materials were monitored by TLC (petroleum ether:ethyl acetate=8:1) to be completely reacted, a saturated NH₄Cl solution was slowly added dropwise until no bubbles were formed, diluting was performed with ethyl acetate (100 mL), an insoluble substance was removed by suction filtration, an organic phase was washed with water (50 mL×2) and a saturated salt solution (50 mL×2), respectively, and dried over anhydrous sodium sulfate, suction filtration was performed, and a solvent was removed under reduced pressure to give 9.22 g of a white solid powder (yield: 98.9%). m.p.103.0-104.0°C.

¹H NMR (300 MHz, DMSO-*d*₆) δ 7.49 (d, J = 8.0 Hz, 1H, ArH), 7.39 (d, J = 7.7 Hz, 1H, ArH), 7.12 (t, J = 7.7 Hz, 1H, ArH), 5.26 (br, 1H, CH₂OH), 4.52 (s, 2H, CH₂), 2.30 (s, 3H, CH₃).

### Synthesis of (3-(2,3-dihydrobenzo[b] [1,4]dioxin-6-yl)-2-methylphenyl)methanol (XIV-1)

The compound **XII-1** (500 mg, 2.49 mmol), benzo-1,4-dioxane-6-boronic acid **XIII-1** (537 mg, 2.98 mmol) and 1,4-dioxane (10 mL) were sequentially added to a three-necked flask, potassium carbonate (962 mg, 6.96 mmol) was dissolved in water (1 mL) to be added to the reaction solution, under nitrogen protection, Pd(PPh₃)₄ (144 mg, 0.15 mmol) was added, the temperature was raised to 80°C, and a reaction was carried out for 12 h. After the raw materials were monitored by TLC (petroleum ether:ethyl acetate=8:1) to be completely reacted, heating was stopped, and the resulting reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated salt solution (10 mL×3), and dried over anhydrous magnesium sulfate. Suction filtration was performed, a solvent was removed through distillation under reduced pressure, and a residue was purified by column chromatography (petroleum ether:ethyl acetate=20:1-15:1) to give 440 mg of a light brown oil (yield: 69.07%).

¹H NMR (300 MHz, DMSO-*d*₆) δ 7.38 (dd, J = 7.6 Hz, 1.4 Hz, 1H, ArH), 7.20 (t, J = 7.6 Hz, 1H, ArH), 7.06 (dd, J = 7.6 Hz, 1.5 Hz, 1H, ArH), 6.92 (d, J = 8.1 Hz, 1H, ArH), 6.78-6.70 (m, 2H, ArH), 4.55 (s, 2H, CH₂OH), 4.29 (s, 4H, OCH₂, OCH₂), 2.14 (s, 3H, CH₃).

### Synthesis of ethyl 2-(2-((3-(2,3-dihydrobenzo[b][1,4]dioxin-6-yl)-2-methylbenzyl)oxy)-7,8-dihydro-1,6-naphthyridin-6(5H)-yl)acetate (I-C-1)

The compound **IX-1** (120 mg, 0.47 mmol), the compound **XIV-1** (145 mg, 0.57 mmol), t-BuXphos (40 mg, 0.09 mmol) and toluene (5 mL) were sequentially added to a three-necked flask, cesium carbonate (307 mg, 0.94 mmol) was added to the reaction solution, and under nitrogen protection, Pd(OAc)₂ (11 mg, 0.05 mmol) was added, the temperature was raised to 80°C, and a reaction was carried out for 12 h. After the raw materials were monitored by TLC (petroleum ether:ethyl acetate=4:1) to be completely reacted, heating was stopped, and the resulting reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated aqueous NaCl solution (10 mL×3), and dried over anhydrous magnesium sulfate. A drying agent was removed by suction filtration, a solvent was removed through distillation under reduced pressure, and purification was performed by column chromatography (petroleum ether:ethyl acetate=20:1-10:1) to give 110 mg of a yellow viscous liquid (yield: 49.21%).

¹H NMR (300 MHz, Chloroform-*d*) δ 7.42 (dd, J = 6.4 Hz, 2.7 Hz, 1H, ArH), 7.23-7.19 (m, 3H, ArH), 6.90 (d, J = 8.2 Hz, 1H, ArH), 6.83 (d, J = 1.8 Hz, 1H, ArH), 6.79 (dd, J = 8.2 Hz, 1.9 Hz, 1H, ArH), 6.59 (d, J = 8.3 Hz, 1H, ArH), 5.37 (s, 2H, OCH₂), 4.30 (s, 4H, OCH₂CH₂O), 4.26 (q, J = 7.1 Hz, 2H, CH₂CH₃), 3.76 (s, 2H, NCH₂), 3.46 (s, 2H, CH₂CO), 3.00 (s, 4H, NCH₂CH₂), 2.28 (s, 3H, ArCH₃), 1.30 (t, J = 7.1 Hz, 3H, CH₃).

### Example 14

Synthesis of ethyl 2-(2-((2'-fluoro-2-methyl-[1,1'-biphenyl]-3-yl)methoxy)-7,8-dihydro-1,6-naphthyridin-6(5*H*)-yl)acetate **(I-D-1:** R¹=CH₃, R²= 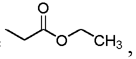 R³=F, and R⁴=H)

### Synthesis of (2'-fluoro-2-methyl-[1,1'-biphenyl]-3-yl)methanol (XV-1)

The compound **XII-1** (2.00 g, 9.95 mmol), 2-fluorobenzeneboronic acid **III-1** (2.09 g, 14.92 mmol) and 1,4-dioxane (45 mL) were sequentially added to a three-necked flask. Potassium carbonate (3.85 g, 27.85 mmol) was dissolved in water (4.5 mL) to be added to the reaction solution, under nitrogen protection, Pd(PPh₃)₄ (0.57 g, 0.50 mmol) was added, the temperature was raised to 80°C, and a reaction was carried out for 12 h. After the raw materials were monitored by TLC (petroleum ether:ethyl acetate=8:1) to be completely reacted, heating was stopped, and the resulting reaction solution was cooled to room temperature. A palladium catalyst and an insoluble substance were removed by suction filtration, diluting was performed with water (50 mL), the reaction solution was extracted with ethyl acetate (50 mL×3), and organic phases were mixed, washed with a saturated salt solution (50 mL×3), and dried over anhydrous magnesium sulfate. Suction filtration was performed, a solvent was removed through distillation under reduced pressure, and a residue was purified by column chromatography (petroleum ether:ethyl acetate=20:1-15:1) to give 2.05 g of a colorless oily liquid (yield: 95.3%). m.p.40-42°C.

¹H NMR (300 MHz, DMSO-*d*₆) δ 7.52-7.42 (m, 2H, ArH), 7.35-7.25 (m, 4H, ArH), 7.11 (d, J = 7.6 Hz, 1H, ArH), 5.20 (s, 1H, OH), 4.58 (s, 2H, CH₂), 2.06 (s, 3H, CH₃).

### Synthesis of ethyl 2-(2-((2'-fluoro-2-methyl-[1,1'-biphenyl]-3-yl)methoxy)-7,8-dihydro-1,6-naphthyridin-6(5H)-yl)acetate (I-D-1)

The compound **IX-1** (0.17 g, 0.67 mmol), the compound **XV-1** (0.17 g, 0.80 mmol), t-BuXphos (0.06 g, 0.13 mmol) and toluene (5 mL) were sequentially added to a three-necked flask, cesium carbonate (0.43 g, 1.33 mmol) was added to the reaction solution, under nitrogen protection, Pd(OAc)₂ (0.01 g, 0.07 mmol) was added, the temperature was raised to 80°C, and a reaction was carried out for 12 h. After the raw materials were monitored by TLC (petroleum ether:ethyl acetate=4:1) to be completely reacted, heating was stopped, and the resulting reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated salt solution (10 mL×3), and dried over anhydrous magnesium sulfate. Suction filtration was performed, a solvent was removed through distillation under reduced pressure, and a residue was purified by column chromatography (petroleum ether:ethyl acetate=20:1-10:1) to give 0.15 g of a colorless clear liquid (yield: 51.8%).

¹H NMR (300 MHz, Chloroform-*d*) δ 7.54 (dd, J = 7.3 Hz, 1.7 Hz, 1H, ArH), 7.43-7.35 (m, 1H, ArH), 7.30-7.13 (m, 6H, ArH), 6.64 (d, J = 8.3 Hz, 1H, ArH), 5.43 (s, 2H, OCH₂), 4.27 (q, J = 7.1 Hz, 2H, CH₂CH₃), 3.78 (s, 2H, NCH₂), 3.49 (s, 2H, NCH₂), 3.03 (s, 4H, NCH₂CH₂), 2.25 (s, 3H, ArCH₃), 1.34 (t, J = 7.1 Hz, 3H, CH₂CH₃).

### Example 15

Synthesis of ethyl 2-(2-((2,2'-dimethyl-[1,1'-biphenyl]-3-yl)methoxy)-7,8-dihydro-1,6-naphthyridin-6(5H)-yl)acetate **(I-D-2:** R¹=CH₃, R²= 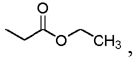 R³=CH₃, and R⁴=H)

### Synthesis of (2,2'-dimethyl-[1,1'-biphenyl]-3-yl)methanol (XV-2)

2.00 g of A white solid was obtained by using the compound **XII-1** (2.00 g, 9.95 mmol) and a compound **III-2** (2.03 g, 14.92 mmol) as raw materials and by using the same procedures as those for the compound **XV-1** (yield: 94.7%). m.p.48-50°C.

¹H NMR (300 MHz, DMSO-*d*₆) δ 7.43 (d, J = 7.5 Hz, 1H, ArH), 7.32-7.23 (m, 4H, ArH), 7.08-7.03 (m, 1H, ArH), 6.98 (d, J = 7.5 Hz, 1H, ArH), 5.17 (t, J = 5.3 Hz, 1H, OH), 4.58 (s, 2H, CH₂), 2.00 (s, 3H, CH₃), 1.94 (s, 3H, CH₃).

### Synthesis of ethyl 2-(2-((2,2'-dimethyl-[1,1'-biphenyl]-3-yl)methoxy)-7,8-dihydro-1,6-naphthyridin-6(5H)-yl)acetate (I-D-2)

67 mg of A yellow viscous liquid was obtained by using the compound **IX-1** (120 mg, 0.47 mmol) and the compound **XV-2** (120 mg, 0.57 mmol) as raw materials and by using the same procedures as those for the compound **I-D-1** (yield: 32.9%).

¹H NMR (300 MHz, Chloroform-d) δ 7.45 (dd, J = 7.7 Hz, 1.5 Hz, 1H, ArH), 7.27 (s, 1H, ArH), 7.25-7.18 (m, 4H, ArH), 7.13-7.08 (m, 2H, ArH), 6.61 (d, J = 8.4 Hz, 1H, ArH), 5.38 (s, 2H, OCH₂), 4.24 (q, J = 7.1 Hz, 2H, CH₂CH₃), 3.87 (s, 2H, NCH₂), 3.54 (s, 2H, COCH₂), 3.11-2.86 (m, 4H, NCH₂CH₂), 2.07 (s, 3H, ArCH₃), 2.06 (s, 3H, ArCH₃), 1.31 (t, J = 7.1 Hz, 3H, CH₂CH₃).

### Example 16

Synthesis of ethyl 2-(2-((2'-chloro-2-methyl-[1,1'-biphenyl]-3-yl)methoxy)-7,8-dihydro-1,6-naphthyridin-6(5H)-yl)acetate **(I-D-3:** R¹=CH₃, R²= 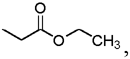 R³=Cl, and R⁴=H)

### Synthesis of (2'-chloro-2-methyl-[1,1'-biphenyl]-3-yl)methanol (XV-3)

500 mg of A white solid was obtained by using the compound **XII-1** (500 mg, 2.49 mmol) and a compound **III-3** (580 mg, 3.73 mmol) as raw materials and by using the same procedures as those for the compound **XV-1** (yield: 86.4%). m.p. 74-76°C.

¹H NMR (400 MHz, DMSO-*d*₆) δ 7.56-7.54 (m, 1H, ArH), 7.46-7.39 (m, 3H, ArH), 7.29-7.22 (m, 2H, ArH), 7.01 (dd, J = 7.6 Hz, 1.4 Hz, 1H, ArH), 4.56 (s, 2H, CH₂), 1.96 (s, 3H, CH₃).

### Synthesis of ethyl 2-(2-((2'-chloro-2-methyl-[1,1'-biphenyl]-3-yl)methoxy)-7,8-dihydro-1,6-naphthyridin-6(5H)-yl)acetate (I-D-3)

150 mg of a yellow viscous liquid was obtained by using the compound **IX-1** (170 mg, 0.73 mmol) and the compound **XV-3** (136 mg, 0.88 mmol) as raw materials and by using the same procedures as those for the compound **I-D-1** (yield: 51.8%).

¹H NMR (300 MHz, Chloroform-d) δ 7.57-7.55 (m, 2H, ArH), 7.28-7.20 (m, 3H, ArH), 7.18-7.13 (m, 2H, ArH), 7.06 (dd, J = 7.6 Hz, 1.5 Hz, 1H, ArH), 6.53 (d, J = 8.4 Hz, 1H, ArH), 5.38 (s, 2H, OCH₂), 4.17 (t, J = 7.1 Hz, 2H, OCH₂CH₃), 3.69 (s, 2H, NCH₂), 3.39 (s, 2H, NCH₂CO), 2.93 (s, 4H, NCH₂CH₂), 2.06 (s, 3H, ArCH₃), 1.22 (d, J = 7.1 Hz, 3H, CH₃).

### Example 17

Synthesis of ethyl 2-(2-((2-methyl-[1,1'-biphenyl]-3-yl)methoxy)-7,8-dihydro-1,6-naphthyridin-6(5*H*)-yl)acetate **(I-D-4:** R¹=CH₃, R²= 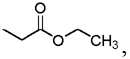 R³=H, and R⁴=H)

### Synthesis of (2-methyl-[1,1'-biphenyl]-3-yl)methanol (XV-4)

320 mg of A white solid was obtained by using the compound **XII-1** (500 mg, 2.49 mmol) and a compound **III-4** (470 mg, 2.98 mmol) as raw materials and by using the same procedures as those for the compound **XV-1** (yield: 64.9%). m.p. 63-65°C.

¹H NMR (300 MHz, DMSO-*d*₆) δ 7.48-7.35 (m, 4H, ArH), 7.30-7.21 (m, 3H, ArH), 7.09 (dd, J = 7.6 Hz, 1.5 Hz, 1H, ArH), 5.16 (s, 1H, OH), 4.56 (s, 2H, CH₂), 2.12 (s, 3H, CH₃).

### Synthesis of ethyl 2-(2-((2-methyl-[1,1'-biphenyl]-3-yl)methoxy)-7,8-dihydro-1,6-naphthyridin-6(5H)-yl)acetate (I-D-4)

115 mg of A colorless viscous liquid was obtained by using the compound **IX-1** (150 mg, 0.59 mmol) and the compound **XV-4** (140 mg, 0.71 mmol) as raw materials and by using the same procedures as those for the compound **I-D-1** (yield: 46.9%).

¹H NMR (300 MHz, Chloroform-*d*) δ 7.47-7.36 (m, 4H, ArH), 7.35-7.29 (m, 3H, ArH), 7.24-7.20 (m, 2H, ArH), 6.60 (d, J = 8.3 Hz, 1H, ArH), 5.39 (s, 2H, OCH₂), 4.24 (q, J = 7.1 Hz, 2H, CH₂CH₃), 3.80 (s, 2H, NCH₂), 3.49 (s, 2H, CH₂CO), 3.03 (s, 4H, NCH₂CH₂), 2.27 (s, 3H, ArCH₃), 1.32 (t, J = 7.1 Hz, 3H, CH₃).

### Example 18

Synthesis of ethyl 2-(2-((2-chloro-2'-fluoro-[1,1'-biphenyl]-3-yl)methoxy)-7,8-dihydro-1,6-naphthyridin-6(5*H*)-yl)acetate **(I-D-5:** R¹=Cl, R²= 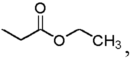 R³=F, and R⁴=H)

### Synthesis of methyl 3-bromo-2-chlorobenzoate (XI-2)

5.29 g of a light brown oil was obtained by using 3-bromo-2-chlorobenzeneboronic acid **X-2** (5.00 g, 21.23 mmol) and thionyl chloride (3.10 mL, 42.47 mmol) as raw materials and by using the same procedures as those for the compound **XI-1** (yield: 99.88%).

¹H NMR (300 MHz, DMSO-*d*₆) δ 8.18 (dd, J = 8.0 Hz, 1.6 Hz, 1H, ArH), 7.96 (dd, J = 7.7 Hz, 1.6 Hz, 1H, ArH), 7.61 (t, J = 7.9 Hz, 1H, ArH), 4.08 (s, 3H, CH₃).

### Synthesis of (3-bromo-2-chlorophenyl)methanol (XII-2)

4.60 g of a white solid powder was obtained by using the compound **XI-2** (5.29 g, 21.20 mmol) as a raw material which was reduced by LiAIH₄ (0.80 g, 21.20 mmol) and by using the same procedures as those for the compound **XII-1** (yield: 97.9%). m.p.56-58°C.

¹H NMR (300 MHz, DMSO-*d*₆) δ 7.66 (d, J = 7.9 Hz, 1H, ArH), 7.56 (d, J = 7.5 Hz, 1H, ArH), 7.31 (t, J = 7.7 Hz, 1H, ArH), 5.55 (t, J = 5.7 Hz, 1H, OH), 4.58 (d, J = 5.5 Hz, 2H, CH₂).

### Synthesis of (2-chloro-2'-fluoro-[1,1'-biphenyl]-3-yl)methanol (XV-5)

0.32 g of a light brown solid was obtained by using the compound **XV-2** (0.50 g, 2.26 mmol) and the compound **III-1** (0.38 g, 2.71 mmol) as raw materials and by using the same procedures as those for the compound **XV-1** (yield: 59.9%). m.p.44-46°C.

¹H NMR (300 MHz, DMSO-*d*₆) δ 7.84 (d, J = 7.7 Hz, 1H, ArH), 7.73-7.61 (m, 2H, ArH), 7.57-7.46 (m, 4H, ArH), 5.68 (s, 1H, OH), 4.83 (s, 2H, CH₂).

### Synthesis of ethyl 2-(2-((2-chloro-2'-fluoro-[1,1'-biphenyl]-3-yl)methoxy)-7,8-dihydro-1,6-naphthyridin-6(5H)-yl)acetate (I-D-5)

116 mg of a colorless viscous liquid was obtained by using the compound **IX-1** (150 g, 0.59 mmol) and the compound **XV-5** (170 mg, 0.71 mmol) as raw materials and by using the same procedures as those for the compound **I-D-1** (yield: 43.3%).

¹H NMR (300 MHz, Chloroform-d) δ 7.59 (dd, J = 7.4 Hz, 1.9 Hz, 1H, ArH), 7.44-7.27 (m, 4H, ArH), 7.25-7.12 (m, 3H, ArH), 6.64 (d, J = 8.3 Hz, 1H, ArH), 5.52 (s, 2H, OCH₂), 4.23 (q, J = 7.1 Hz, 2H, CH₂CH₃), 3.75 (s, 2H, COCH₂), 3.45 (s, 2H, NCH₂), 2.98 (s, 4H, NCH₂CH₂), 1.29 (t, J = 7.1 Hz, 3H, CH₃).

### Example 19

Synthesis of ethyl 2-(2-((2-chloro-2'-methyl-[1,1'-biphenyl]-3-yl)methoxy)-7,8-dihydro-1,6-naphthyridin-6(5*H*)-yl)acetate **(I-D-6:** R¹=Cl, R²= 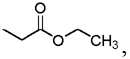 R³=CH₃, and R⁴=H)

### Synthesis of (2-chloro-2'-methyl-[1,1'-biphenyl]-3-yl)methanol (XV-6)

0.32 g of a light brown solid was obtained by using the compound **XII-2** (0.50 g, 2.26 mmol) and the compound **III-2** (0.37 g, 2.71 mmol) as raw materials and by using the same procedures as those for the compound **XV-1** (yield: 59.9%). m.p. 44-46°C.

¹H NMR (400 MHz, DMSO-*d*₆) δ 7.63-7.51 (m, 1H, ArH), 7.44-7.00 (m, 6H, ArH), 5.40 (s, 1H, OH), 4.65 (s, 2H, CH₂), 2.03 (s, 3H, CH₃).

### Synthesis of ethyl 2-(2-((2-chloro-2'-methyl-[1,1'-biphenyl]-3-yl)methoxy)-7,8-dihydro-1,6-naphthyridin-6(5H)-yl)acetate (I-D-6)

117 mg of a colorless viscous liquid was obtained by using the compound **IX-1** (150 mg, 0.59 mmol) and the compound **XV-6** (165 mg, 0.71 mmol) as raw materials and by using the same procedures as those for the compound **I-D-1** (yield: 44.0%).

¹H NMR (300 MHz, Chloroform-d) δ 7.56 (dd, J = 7.7 Hz, 1.7 Hz, 1H, ArH), 7.34-7.27 (m, 3H, ArH), 7.25-7.21 (m, 2H, ArH), 7.20-7.12 (m, 2H, ArH), 6.67-6.65 (d, J = 8.3 Hz, 1H, ArH), 5.51 (s, 2H, OCH₂), 4.22 (q, J = 7.1 Hz, 2H, CH₂CH₃), 3.78 (s, 2H, NCH₂), 3.47 (s, 2H, CH₂CO), 2.99 (s, 4H, NCH₂CH₂), 2.12 (s, 3H, ArCH₃), 1.32-1.30 (t, J = 7.1 Hz, 3H, CH₂CH₃).

### Example 20

Synthesis of ethyl 2-(2-((2,2'-dichloro-[1,1'-biphenyl]-3-yl)methoxy)-7,8-dihydro-1,6-naphthyridin-6(5*H*)-yl)acetate **(I-D-7:** R¹=Cl, R2= 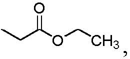 R³=Cl, and R⁴=H)

### Synthesis of (2,2'-dichloro-[1,1'-biphenyl]-3-yl)methanol (XV-7)

0.31 g of a light brown oil was obtained by using the compound **XII-2** (0.50 g, 2.26 mmol) and the compound **III-3** (0.42 g, 2.71 mmol) as raw materials and by using the same procedures as those for the compound **XV-1** (yield: 54.2%).

¹H NMR (300 MHz, DMSO-*d*₆) δ 7.88-7.82 (m, 1H, ArH), 7.82-7.76 (m, 1H, ArH), 7.70-7.62 (m, 3H, ArH), 7.57-7.51 (m, 1H, ArH), 7.47-7.41 (m, 1H, ArH), 5.71 (s, 1H, OH), 4.85 (s, 2H, CH₂).

### Synthesis of ethyl 2-(2-((2,2'-dichloro-[1,1'-biphenyl]-3-yl)methoxy)-7,8-dihydro-1,6-naphthyridin-6(5H)-yl)acetate (I-D-7)

130 mg of A colorless viscous liquid was obtained by using a compound **IX-1** (150 mg, 0.59 mmol) and the compound **XV-7** (165 mg, 0.71 mmol) as raw materials and by using the same procedures as those for the compound **I-D-1** (yield: 46.8%).

¹H NMR (300 MHz, Chloroform-d) δ 7.60 (dd, J = 7.6 Hz, 1.8 Hz, 1H, ArH), 7.51-7.46 (m, 1H, ArH), 7.35-7.32 (m, 5H, ArH), 7.24-7.19 (m, 2H, ArH), 6.64 (d, J = 8.4 Hz, 1H, ArH), 5.52 (s, 2H, OCH₂), 4.22 (q, J = 7.1 Hz, 2H, CH₂CH₃), 3.76 (s, 2H, NCH₂), 3.45 (s, 2H, COCH₂), 2.98 (s, 4H, NCH₂CH₂), 1.30 (t, J = 7.1 Hz, 3H, CH₃).

### Example 21

Synthesis of ethyl 2-(2-((2-chloro-[1,1'-biphenyl]-3-yl)methoxy)-7,8-dihydro-1,6-naphthyridin-6(5*H*)-yl)acetate **(I-D-8:** R¹=Cl, R²= 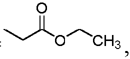 R³=H, and R⁴=H)

### Synthesis of (2-chloro-[1,1'-biphenyl]-3-yl)methanol (XV-8)

0.41 g of a light yellow solid was obtained by using the compound **XII-2** (0.50 g, 2.26 mmol) and the compound **III-4** (0.41 g, 2.71 mmol) as raw materials and by using the same procedures as those for the compound **XV-1** (yield: 83.1%). m.p.76-78°C.

¹H NMR (400 MHz, DMSO-*d*₆) δ 7.59 (dd, J = 7.6 Hz, 1.7 Hz, 1H, ArH), 7.49-7.38 (m, 6H, ArH), 7.28 (dd, J = 7.6 Hz, 1.8 Hz, 1H, ArH), 5.48 (s, 1H, OH), 4.63 (s, 2H, CH₂).

### Synthesis of ethyl 2-(2-((2-chloro-[1,1'-biphenyl]-3-yl)methoxy)-7,8-dihydro-1,6-naphthyridin-6(5H)-yl)acetate (I-D-8)

187 mg of a colorless viscous liquid was obtained by using the compound **IX-1** (150 mg, 0.59 mmol) and the compound **XV-8** (155 mg, 0.71 mmol) as raw materials and by using the same procedures as those for the compound **I-D-1** (yield: 72.7%).

¹H NMR (300 MHz, Chloroform-d) δ 7.55 (dd, J = 6.9 Hz, 2.5 Hz, 1H, ArH), 7.46-7.37 (m, 6H, ArH), 7.34-7.28 (m, 2H, ArH), 6.65 (d, J = 8.4 Hz, 1H, ArH), 5.52 (s, 2H, OCH₂), 4.23 (q, J = 7.2 Hz, 2H, CH₂CH₃), 3.81 (s, 2H, NCH₂), 3.49 (s 2H, COCH₂), 3.02 (s, 4H, NCH₂CH₂), 1.30 (t, J = 7.1 Hz, 3H, CH₃).

### Example 22

Synthesis of ethyl 2-(2-((2,2'-difluoro-[1,1'-biphenyl]-3-yl)methoxy)-7,8-dihydro-1,6-naphthyridin-6(5*H*)-yl)acetate **(I-D-9:** R¹=F, R²= 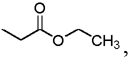 R³=F, and R⁴=H)

### Synthesis of methyl 3-bromo-2-fluorobenzoate (XI-3)

5.30 g of a light yellow solid was obtained by using 3-bromo-2-chlorobenzeneboronic acid **X-3** (5.00 g, 21.23 mmol) and thionyl chloride (3.10 mL, 42.47 mmol) as raw materials and by using the same procedures as those for the compound **XI-1** (yield: 99.6%). m.p. 35-36°C.

¹H NMR (300 MHz, DMSO-*d*₆) δ 7.94 (ddd, J = 8.1 Hz, 6.4 Hz, 1.8 Hz, 1H, ArH), 7.83 (ddd, J = 8.1 Hz, 6.6 Hz, 1.7 Hz, 1H, ArH), 7.25 (td, J = 7.9 Hz, 1.0 Hz, 1H, ArH), 3.83 (s, 3H, CH₃).

### Synthesis of (3-bromo-2-fluorophenyl)methanol (XII-3)

4.30 g of a light yellow solid was obtained by using the compound **XI-3** (5.30 g, 22.74 mmol) as a raw material which was reduced by LiAlH₄ (0.86 g, 22.74 mmol) and by using the same procedures as those for the compound **XII-1** (yield: 92.2%). m.p. 34-35°C.

¹H NMR (300 MHz, DMSO-*d*₆) δ 7.65-7.55 (m, 1H, ArH), 7.53-7.43 (m, 1H, ArH), 7.17 (t, J = 7.8 Hz, 1H, ArH), 5.42 (t, J = 6.0 Hz, 1H, OH), 4.59 (s, 2H, CH₂).

### Synthesis of (2,2'-difluoro-[1,1'-biphenyl]-3-yl)methanol (XV-9)

0.37 g of a white solid was obtained by using the compound **XII-3** (0.50 g, 2.44 mmol) and the compound **III-1** (0.41 g, 2.93 mmol) as raw materials and by using the same procedures as those for the compound **XV-1** (yield: 68.9%). m.p.50-52°C.

¹H NMR (300 MHz, DMSO-*d*₆) δ 7.49-7.32 (m, 3H, ArH), 7.28-7.18 (m, 4H, ArH), 4.53 (s, 2H, CH₂).

### Synthesis of ethyl 2-(2-((2,2'-difluoro-[1,1'-biphenyl]-3-yl)methoxy)-7,8-dihydro-1,6-naphthyridin-6(5H)-yl)acetate (I-D-9)

100 mg of a colorless viscous liquid was obtained by using the compound **IX-1** (150 mg, 0.59 mmol) and the compound **XV-9** (156 mg, 0.71 mmol) as raw materials and by using the same procedures as those for the compound **I-D-1** (yield: 38.7%).

¹H NMR (300 MHz, Chloroform-*d*) δ 7.56-7.51 (m, 1H, ArH), 7.42-7.33 (m, 4H, ArH), 7.22-7.13 (m, 3H, ArH), 6.61 (d, J = 8.4 Hz, 1H, ArH), 5.48 (s, 2H, OCH₂), 4.23 (q, J = 7.2 Hz, 2H, CH₂CH₃), 3.79 (s, 2H, NCH₂), 3.48 (s, 2H, CH₂CO), 3.02-2.99 (m, 4H, NCH₂CH₂), 1.31 (t, J = 7.1 Hz, 3H, CH₃).

### Example 23

Synthesis of ethyl 2-(2-((2-fluoro-2'-methyl-[1,1'-biphenyl]-3-yl)methoxy)-7,8-dihydro-1,6-naphthyridin-6(5*H*)-yl)acetate **(I-D-10:** R¹=F, R²= 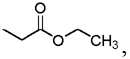 R³=CH₃, and R⁴=H)

### Synthesis of (2-fluoro-2'-methyl-[1,1'-biphenyl]-3-yl)methanol (XV-10)

0.34 g of A colorless oil was obtained by using the compound **XII-3** (0.50 g, 2.44 mmol) and the compound **III-2** (0.40 g, 2.93 mmol) as raw materials and by using the same procedures as those for the compound **XV-1** (yield: 63.7%).

¹H NMR (300 MHz, DMSO-*d*₆) δ 7.51 (td, J = 7.3 Hz, 1.9 Hz, 1H, ArH), 7.34-7.32 (m, 2H, ArH), 7.31-7.24 (m, 2H, ArH), 7.23-7.16 (m, 2H, ArH), 4.61 (s, 2H, CH₂), 2.14 (s, 3H, CH₃).

### Synthesis of ethyl 2-(2-((2-fluoro-2'-methyl-[1,1'-biphenyl]-3-yl)methoxy)-7,8-dihydro-1,6-naphthyridin-6(5H)-yl)acetate (I-D-10)

130 mg of A colorless viscous liquid was obtained by using the compound **IX-1** (150 mg, 0.59 mmol) and the compound **XV-10** (153 mg, 0.71 mmol) as raw materials and by using the same procedures as those for the compound **I-D-1** (yield: 50.8%).

¹H NMR (300 MHz, Chloroform-d) δ 7.54-7.48 (m, 1H, ArH), 7.31-7.28 (m, 2H, ArH), 7.24-7.15 (m, 5H, ArH), 6.61 (d, J = 8.3 Hz, 1H, ArH), 5.46 (s, 2H, OCH₂), 4.23 (q, J = 7.1 Hz, 2H, CH₂CH₃), 3.79 (s, 2H, NCH₂), 3.48 (s, 2H, CH₂CO), 3.00 (s, 4H, NCH₂CH₂), 2.21 (s, 3H, ArCH₃), 1.31 (t, J = 7.1 Hz, 3H, CH₂CH₃).

### Example 24

Synthesis of ethyl 2-(2-((2'-chloro-2-fluoro-[1,1'-biphenyl]-3-yl)methoxy)-7,8-dihydro-1,6-naphthyridin-6(5*H*)-yl)acetate **(I-D-11:** R¹=F, R²= 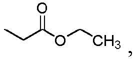 R³=Cl, and R⁴=H)

### Synthesis of (2'-chloro-2-fluoro-[1,1'-biphenyl]-3-yl)methanol (XV-11)

0.34 g of a colorless oil was obtained by using the compound **XII-3** (0.50 g, 2.44 mmol) and the compound **III-3** (0.46 g, 2.93 mmol) as raw materials and by using the same procedures as those for the compound **XV-1** (yield: 58.9%). m.p.34-35°C.

¹H NMR (300 MHz, DMSO-*d*₆) δ 7.62-7.51 (m, 2H, ArH), 7.50-7.38 (m, 3H, ArH), 7.32-7.21 (m, 2H, ArH), 5.36 (s, 1H, OH), 4.61 (s, 2H, CH₂).

### Synthesis of ethyl 2-(2-((2'-chloro-2-fluoro-[1,1'-biphenyl]-3-yl)methoxy)-7,8-dihydro-1,6-naphthyridin-6(5H)-yl) acetate (I-D-11)

104 mg of a colorless viscous liquid was obtained by using the compound **IX-1** (150 mg, 0.59 mmol) and the compound **XV-11** (167 mg, 0.71 mmol) as raw materials and by using the same procedures as those for the compound **I-D-1** (yield: 38.8%).

¹H NMR (300 MHz, Chloroform-d) δ 7.58-7.52 (m, 2.0 Hz, 1H, ArH), 7.50-7.47 (m, 1H, ArH), 7.35-7.32 (m, 3H, ArH), 7.25-7.16 (m, 3H, ArH), 6.61 (d, J = 8.4 Hz, 1H, ArH), 5.47 (s, 2H, OCH₂), 4.23 (q, J = 7.1 Hz, 2H, CH₂CH₃), 3.77 (s, 2H, NCH₂), 3.46 (s, 2H, CH₂CO), 2.99 (s, 4H, NCH₂CH₂), 1.30 (t, J = 7.1 Hz, 3H, CH₃).

### Example 25

Synthesis of ethyl 2-(2-((2-fluoro-[1,1'-biphenyl]-3-yl)methoxy)-7,8-dihydro-1,6-naphthyridin-6(5*H*)-yl)acetate **(I-D-12:** R¹=F, R²= 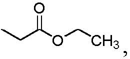 R³=H, and R⁴=H)

### Synthesis of (2-fluoro-[1,1'-biphenyl]-3-yl)methanol (XV-12)

0.37 g of a white solid was obtained by using the compound **XII-3** (0.50 g, 2.44 mmol) and the compound **III-4** (0.45 g, 2.93 mmol) as raw materials and by using the same procedures as those for the compound **XV-1** (yield: 75.1%). m.p.84-86°C.

¹H NMR (300 MHz, DMSO-*d*₆) δ 7.54-7.44 (m, 5H, ArH), 7.43-7.36 (m, 2H, ArH), 7.27 (t, J = 7.6 Hz, 1H, ArH), 5.32 (t, J = 5.7 Hz, 1H, OH), 4.60 (d, J = 5.2 Hz, 2H, CH₂).

### Synthesis of ethyl 2-(2-((2-fluoro-[1,1'-biphenyl]-3-yl)methoxy)-7,8-dihydro-1,6-naphthyridin-6(5H)-yl)acetate (I-D-12)

149 mg of a colorless viscous liquid was obtained by using the compound **IX-1** (150 mg, 0.59 mmol) and the compound **XV-12** (143 mg, 0.71 mmol) as raw materials and by using the same procedures as those for the compound **I-D-1** (yield: 60.2%).

¹H NMR (300 MHz, Chloroform-d) δ 7.57-7.55 (m, 1H, ArH), 7.49-7.38 (m, 6H, ArH), 7.23-7.16 (m, 2H, ArH), 6.61 (d, J = 8.4 Hz, 1H, ArH), 5.47 (s, 2H, OCH₂),

4.27 (q, J = 7.1 Hz, 2H, CH₂CH₃), 3.83 (s, 2H, NCH₂), 3.51 (s, 2H, COCH₂), 3.06-3.01 (m, 4H, NCH₂CH₂), 1.32 (t, J = 7.1 Hz, 3H, CH₃).

### Example 26

Synthesis of 2-(2-((2'-fluoro-2-methyl-[1,1'-biphenyl]-3-yl)methoxy)-7,8-dihydro-1,6-naphthyridin-6(5*H*)-yl)acetic acid **(I-D-13:** R¹=CH₃, R²= 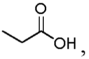 R³=F, and R⁴=H)

The compound **I-D-1** (60 mg, 0.14 mmol) was added into a 10 mL eggplant shaped flask, ethanol (2 mL) was added to dissolve the compound **I-D-1,** a solution of NaOH (11 mg, 0.28 mmol) in water (0.3 mL) was added, and stirring was performed at room temperature for 4 h. After the reaction was monitored by TLC to be complete, ethanol was removed through distillation under reduced pressure, a pH was adjusted to 5-6 with a 2M HCl solution, so that a white solid was precipitated, and suction filtration was performed to give 30 mg of a white solid product (yield: 53.5%). m.p.130-132°C.

¹H NMR (300 MHz, DMSO-*d*₆) δ 7.50-7.40 (m, 3H, ArH), 7.32-7.25 (m, 4H, ArH), 7.18-7.16 (m, 1H, ArH), 6.66 (d, J = 8.3 Hz, 1H, ArH), 5.34 (s, 2H, OCH₂), 3.67 (s, 4H, NCH₂, CH₂COOH), 2.92-2.79 (m, 4H, NCH₂CH₂), 2.11 (s, 3H, CH₃).

HRMS (ESI): m/z [M+H]⁺ Calcd for C₂₄H₂₄FN₂O₃: 407.1771; found: 407.1768.

### Example 27

Synthesis of 2-(2-((2'-fluoro-2-methyl-[1,1'-biphenyl]-3-yl)methoxy)-7,8-dihydro-1,6-naphthyridin-6(5*H*)-yl)ethan-1-ol **(I-D-14:** R¹=CH₃, R²= 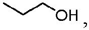 R³=F, and R⁴=H)

The compound **I-D-1** (80 mg, 018 mmol) was added into a 25 mL eggplant shaped flask and dissolved in THF, and lithium aluminum hydride (14 mg, 0.37 mmol) was added slowly at 0°C. After the raw materials were monitored to be completed reacted, the reaction was quenched with a saturated ammonium chloride solution, so that a white solid was precipitated. The white solid was removed by suction filtration, and washing was performed with EA. A filtrate was concentrated, and separated and purified to give 34 mg of a yellow solid (yield: 41.5%). m.p. 72-74°C.

¹H NMR (300 MHz, Chloroform-*d*) δ 7.54 (d, J = 7.3 Hz, 1H, ArH), 7.45-7.32 (m, 2H, ArH), 7.30-7.14 (m, 5H, ArH), 6.67 (d, J = 8.3 Hz, 1H, ArH), 5.44 (s, 2H, OCH₂), 3.81 (t, J = 5.3 Hz, 2H, CH₂OH), 3.76 (s, 2H, NCH₂), 3.06-2.95 (m, 4H, NCH₂CH₂), 2.85 (t, J = 5.3 Hz, 2H, NCH₂), 2.26 (s, 3H, CH₃).

HRMS (ESI): m/z [M+H]⁺ Calcd for C₂₄H₂₆FN₂O₂: 393.1978; found: 393.1975.

### Example 28

Synthesis of 2-(2-((2,2'-dimethyl-[1,1'-biphenyl]-3-yl)methoxy)-7,8-dihydro-1, 6-naphthyridin-6(5*H*)-yl)ethan-1-ol **(I-D-15:** R¹=CH₃, R²= 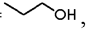 R³=CH₃, and R⁴=H) 34 mg of a yellow solid was obtained by using the compound **I-D-2** (100 mg, 0.23 mmol) and LiAIH₄ (18 mg, 0.47 mmol) as raw materials and by using the same procedures as those for the compound **I-D-13** (yield: 41.5%). m.p.96-98°C.

¹H NMR (300 MHz, Chloroform-*d*) δ 7.47 (d, J = 7.6 Hz, 1H, ArH), 7.27-7.21 (m, 5H, ArH), 7.16-7.08 (m, 2H, ArH), 6.61 (d, J = 8.4 Hz, 1H, ArH), 5.39 (s, 2H, OCH₂), 3.73 (t, J = 5.3 Hz, 2H, CH₂OH), 3.64 (s, 2H, NCH₂), 2.96-2.90 (m, 4H, NCH₂CH₂), 2.76 (t, J = 5.3 Hz, 2H, NCH₂), 2.09 (s, 3H), 2.06 (s, 3H).

HRMS (ESI): m/z [M+H]⁺ Calcd for C₂₅H₂₉N₂O₂: 389.2229; found: 389.2230.

### Example 29

Synthesis of 2-(2-((2'-chloro-2-methyl-[1,1'-biphenyl]-3-yl)methoxy)-7,8-dihydro-1,6-naphthyridin-6(5*H*)-yl)ethan-1-ol **(I-D-16:** R¹=CH₃, R²= 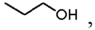 R³=Cl, and R⁴=H)

100 mg of a yellow solid was obtained by using the compound **I-D-3** (150 mg, 0.37 mmol) and LiAIH₄ (24 mg, 0.73 mmol) as raw materials and by using the same procedures as those for the compound **I-D-13** (yield: 66.6%). m.p.108-110°C.

¹H NMR (300 MHz, Chloroform-*d*) δ 7.58-7.50 (m, 2H, ArH), 7.40-7.32 (m, 3H, ArH), 7.31-7.26 (m, 2H, ArH), 7.19-7.17 (m, 1H, ArH), 6.67 (d, J = 8.4 Hz, 1H, ArH), 5.50-5.39 (m, 2H, OCH₂), 3.79 (t, J = 5.3 Hz, 2H, CH₂OH), 3.72 (s, 2H, NCH₂), 3.02-2.97 (m, 4H, NCH₂CH₂), 2.83 (t, J = 5.3 Hz, 2H, NCH₂), 2.19 (s, 3H, CH₃).

HRMS (ESI): m/z [M+H]⁺ Calcd for C₂₄H₂₆ClN₂O₂: 409.1683; found: 409.1687.

### Example 30

Synthesis of 2-(2-((2-methyl-[1,1'-biphenyl]-3-yl)methoxy)-7,8-dihydro-1,6-naphthyridin-6(5*H*)-yl)ethan-1-ol **(I-D-17:** R¹=CH₃, R²= 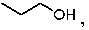 R³=H, and R⁴=H)

55 mg of A yellow solid was obtained by using the compound **I-D-4** (100 mg, 0.26 mmol) and LiAIH₄ (20 mg, 0.53 mmol) as raw materials and by using the same procedures as those for the compound **I-D-12** (yield: 55.6%). m.p.152-154°C.

¹H NMR (300 MHz, Chloroform-*d*) δ 7.48-7.36 (m, 4H, ArH), 7.35-7.28 (m, 3H, ArH), 7.23 (s, 2H, ArH), 6.62 (d, J = 8.3 Hz, 1H, ArH), 5.39 (s, 2H, OCH₂), 3.74 (t, J = 5.3 Hz, 2H, CH₂OH), 3.66 (s, 2H, NCH₂), 3.00-2.92 (m, 4H, NCH₂CH₂), 2.77 (t, J = 5.3 Hz, 2H, CH₂CH₂OH), 2.28 (s, 3H, CH₃).

HRMS (ESI): m/z [M+H]⁺ Calcd for C₂₄H₂₇N₂O₂: 375.2073; found: 375.2074.

### Example 31

Synthesis of 2-(2-((2-chloro-2'-fluoro-[1,1'-biphenyl]-3-yl)methoxy)-7,8-dihydro-1,6-naphthyridin-6(5*H*)-yl)ethan-1-ol **(I-D-18:** R¹=Cl, R²= 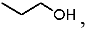 R³=F, and R⁴=H)

76 mg of a yellow solid was obtained by using the compound **I-D-5** (106 mg, 0.23 mmol) and LiAIH₄ (18 mg, 0.47 mmol) as raw materials and by using the same procedures as for those for the compound **I-D-13** (yield: 79.0%). m.p.116-118°C.

¹H NMR (300 MHz, Chloroform-*d*) δ 7.60 (dd, J = 7.5 Hz, 2.0 Hz, 1H, ArH), 7.44-7.27 (m, 5H, ArH), 7.21-7.18 (m, 1H, ArH), 7.17-7.12 (m, 1H, ArH), 6.66 (d, J = 8.4 Hz, 1H, ArH), 5.52 (s, 2H, OCH₂), 3.75 (t, J = 5.3 Hz, 2H, CH₂OH), 3.69 (s, 2H, NCH₂), 2.99-2.93 (m, 4H, NCH₂CH₂), 2.79 (t, J = 5.3 Hz, 2H, CH₂CH₂OH).

HRMS (ESI): m/z [M+H]⁺ Calcd for C₂₃H₂₃ClFN₂O₂: 413.1432; found: 413.1432.

### Example 32

Synthesis of 2-(2-((2-chloro-2'-methyl-[1,1'-biphenyl]-3-yl)methoxy)-7,8-dihydro-1,6-naphthyridin-6(5*H*)-yl)ethan-1-ol **(I-D-19:** R¹=Cl, R²= 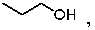 R³=CH₃, and R⁴=H)

30 mg of a white solid was obtained by using the compound **I-D-6** (60 mg, 0.13 mmol) and LiAlH₄ (10 mg, 0.27 mmol) as raw materials and by using the same procedures as those for the compound **I-D-13** (yield: 55.2%). m.p.88-90°C.

¹H NMR (300 MHz, Chloroform-*d*) δ 7.56 (dd, J = 7.6 Hz, 1.7 Hz, 1H, ArH), 7.34-7.26 (m, 4H, ArH), 7.24-7.21 (m, 1H, ArH), 7.20-7.12 (m, 2H, ArH), 6.66 (d, J = 8.5 Hz, 1H, ArH), 5.51 (s, 2H, OCH₂), 3.74 (t, J = 5.3 Hz, 2H, CH₂OH), 3.68 (s, 2H, NCH₂), 2.96-2.93 (m, 4H, NCH₂CH₂), 2.78 (t, J = 5.3 Hz, 2H, CH₂CH₂OH), 2.11 (s, 3H, CH₃).

HRMS (ESI): m/z [M+H]⁺ Calcd for C₂₄H₂₆ClN₂O₂: 409.1683; found: 409.1682.

### Example 33

Synthesis of 2-(2-((2,2'-dichloro-[1,1'-biphenyl]-3-yl)methoxy)-7,8-dihydro-1,6-naphthyridin-6(5*H*)-yl)ethan-1-ol **(I-D-20:** R¹=Cl, R²= 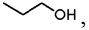 R³=Cl, and R⁴=H)

64 mg of a yellow solid was obtained by using the compound **I-D-7** (120 mg, 0.26 mmol) and LiAlH₄ (19 mg, 0.51 mmol) as raw materials and by using the same procedures as those for the compound **I-D-13** (yield: 58.5%). m.p.110-112°C.

¹H NMR (300 MHz, Chloroform-d) δ 7.61 (dd, J = 7.7 Hz, 1.8 Hz, 1H, ArH), 7.51-7.46 (m, 1H, ArH), 7.38-7.27 (m, 5H, ArH), 7.25-7.20 (m, 1H, ArH), 6.66 (d, J = 8.4 Hz, 1H, ArH), 5.52 (s, 2H, OCH₂), 3.75 (t, J = 5.3 Hz, 2H, CH₂OH), 3.69 (s, 2H, NCH₂), 2.99-2.93 (m, 4H, NCH₂CH₂), 2.79 (t, J = 5.3 Hz, 2H, CH₂CH₂OH).

HRMS (ESI): m/z [M+H]⁺ Calcd for C₂₃H₂₃CI₂N₂O₂: 429.1137; found: 429.1141.

### Example 34

Synthesis of 2-(2-((2-chloro-[1,1'-biphenyl]-3-yl)methoxy)-7,8-dihydro-1,6-naphthyridin-6(5*H*)-yl)ethan-1-ol **(I-D-21:** R¹=Cl, R²= 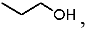 R³=H, and R⁴=H)

80 mg of a white solid was obtained by using the compound **I-D-8** (187 mg, 0.43 mmol) and LiAlH₄ (27 mg, 0.46 mmol) as raw materials and by using the same procedures as those for the compound **I-D-13** (yield: 47.3%). m.p.148-150°C.

¹H NMR (300 MHz, Chloroform-d) δ 7.39 (dd, J = 6.9 Hz, 2.5 Hz, 1H, ArH), 7.26-7.21 (m, 2H, ArH), 7.17-7.06 (m, 5H, ArH), 6.48 (d, J = 8.5 Hz, 1H, ArH), 5.35 (s, 2H, OCH₂), 3.55 (t, J = 5.3 Hz, 2H, CH₂OH), 3.47 (s, 2H, NCH₂), 2.80 - 2.70 (m, 4H, NCH₂CH₂), 2.58 (t, J = 5.3 Hz, 2H, CH₂CH₂OH).

HRMS (ESI): m/z [M+H]⁺ Calcd for C₂₃H₂₄ClN₂O₂: 395.1526; found: 395.1522.

### Example 35

Synthesis of 2-(2-((2,2'-difluoro-[1,1'-biphenyl]-3-yl)methoxy)-7,8-dihydro-1,6-naphthyridin-6(5*H*)-yl)ethan-1-ol **(I-D-22:** R¹=F, R²= 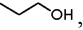 R³=F, and R⁴=H)

60 mg of a brown solid was obtained by using the compound **I-D-9** (100 mg, 0.23 mmol) and LiAIH₄ (17 mg, 0.46 mmol) as raw materials and by using the same procedures as those for the compound **I-D-13** (yield: 66.4%). m.p.60-62°C.

¹H NMR (300 MHz, Chloroform-*d*) δ 7.57-7.52 (m, 1H, ArH), 7.43-7.32 (m, 3H, ArH), 7.25-7.13 (m, 4H, ArH), 6.62 (d, J = 8.3 Hz, 1H, ArH), 5.48 (s, 2H, OCH₂), 3.74 (t, J = 5.3 Hz, 2H, CH₂OH), 3.66 (s, 2H, NCH₂), 2.95-2.92 (m, 4H, NCH₂CH₂), 2.77 (t, J = 5.3 Hz, 2H, CH₂CH₂OH).

HRMS (ESI): m/z [M+H]⁺ Calcd for C₂₃H₂₃F₂N₂O₂: 397.1728; found: 397.1730.

### Example 36

Synthesis of 2-(2-((2-fluoro-2'-methyl-[1,1'-biphenyl]-3-yl)methoxy)-7,8-dihydro-1,6-naphthyridin-6(5*H*)-yl)ethan-1-ol **(I-D-23:** R¹=F, R²= 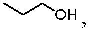 R³=CH₃, and R⁴=H)
90 mg of an off-white solid was obtained by using the compound **I-D-10** (130 mg, 0.30 mmol) and LiAlH₄ (23 mg, 0.59 mmol) as raw materials and by using the same procedures as those for the compound **I-D-13** (yield: 76.7%). m.p.62-64°C.

¹H NMR (300 MHz, Chloroform-*d*) δ 7.56-7.48 (m, 1H, ArH), 7.31-7.29 (m, 3H, ArH), 7.25-7.18 (m, 4H, ArH), 6.62 (d, J = 8.3 Hz, 1H, ArH), 5.47 (s, 2H, OCH₂), 3.73 (t, J = 5.3 Hz, 2H, CH₂OH), 3.66 (s, 2H, NCH₂), 2.98-2.89 (m, 4H, NCH₂CH₂), 2.77 (t, J = 5.3 Hz, 2H, CH₂CH₂OH), 2.21 (d, J = 1.4 Hz, 3H, CH₃).

HRMS (ESI): m/z [M+H]⁺ Calcd for C₂₄H₂₆FN₂O₂: 393.1978; found: 393.1981.

### Example 37

Synthesis of 2-(2-((2'-chloro-2-fluoro-[1,1'-biphenyl]-3-yl)methoxy)-7,8-dihydro-1,6-naphthyridin-6(5*H*)-yl)ethan-1-ol **(I-D-24:** R¹=F, R²= 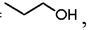 R³=Cl, and R⁴=H)

60 mg of a light yellow solid was obtained by using the compound **I-D-11** (104 mg, 0.23 mmol) and LiAIH₄ (17 mg, 0.46 mmol) as raw materials and by using the same procedures as those for the compound **I-D-13** (yield: 74.0%). m.p.68-70°C.

¹H NMR (300 MHz, Chloroform-*d*) δ 7.59-7.54 (m, 1H, ArH), 7.51-7.47 (m, 1H, ArH), 7.38-7.30 (m, 3H, ArH), 7.26-7.18 (m, 3H, ArH), 6.62 (d, J = 8.3 Hz, 1H, ArH), 5.48 (s, 2H, OCH₂), 3.74 (t, J = 5.3 Hz, 2H, CH₂OH), 3.68 (s, 2H, NCH₂), 2.97-2.92 (m, 4H, NCH₂CH₂), 2.78 (t, J = 5.3 Hz, 2H, CH₂CH₂OH).

HRMS (ESI): m/z [M+H]⁺ Calcd for C₂₃H₂₃ClFN₂O₂: 413.1432; found: 413.1430.

### Example 38

Synthesis of 2-(2-((2-fluoro-[1,1'-biphenyl]-3-yl)methoxy)-7,8-dihydro-1,6-naphthyridin-6(5*H*)-yl)ethan-1-ol **(I-D-25:** R¹=F, R²= 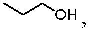 R³=H, and R⁴=H)
70 mg of an off-white solid was obtained by using the compound **I-D-11** (149 mg, 0.35 mmol) and LiAlH₄ (17 mg, 0.46 mmol) as raw materials and by using the same procedures as those for the compound **I-D-13** (yield: 52.3%). m.p.72-74°C.

¹H NMR (300 MHz, Chloroform-*d*) δ 7.57-7.36 (m, 7H, ArH), 7.25-7.17 (m, 2H, ArH), 6.62 (d, J = 8.4 Hz, 1H, ArH), 5.48 (s, 2H, OCH₂), 3.74 (t, J = 5.3 Hz, 2H, CH₂OH), 3.68 (s, J = 2.9 Hz, 2H, NCH₂), 2.99-2.90 (m, 4H, NCH₂CH₂), 2.77 (t, J = 5.3 Hz, 2H, CH₂CH₂OH).

HRMS (ESI): m/z [M+H]⁺ Calcd for C₂₃H₂₄FN₂O₂: 379.1822; found: 379.1822.

### Example 39

Synthesis of 2-(2-((2-chloro-2'-fluoro-[1,1'-biphenyl]-3-yl)methoxy)-7,8-dihydro-1,6-naphthyridin-6(5*H*)-yl)acetic acid **(I-D-26:** R¹=Cl, R²= 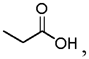 R³=F, and R⁴=H)

The compound **I-D-5** (400 mg, 0.88 mmol) was added into a 10 mL eggplant shaped flask, ethanol (3 mL) was added to dissolve the compound **I-D-5,** a solution of NaOH (69 mg, 1.76 mmol) in water (0.2 mL) was added, and stirring was performed at room temperature for 4 h. After the reaction was monitored by TLC to be complete, ethanol was removed through distillation under reduced pressure, a pH was adjusted to 5-6 with a 2M HCl solution, so that a white solid was precipitated, and suction filtration was performed to give 262 mg of a white solid product (yield: 69.8%). m.p.101-108°C.

¹H NMR (300 MHz, Chloroform-*d*) δ 7.59-7.52 (m, 1H, ArH), 7.41-7.27 (m, 4H, ArH), 7.22-7.01 (m, 3H, ArH), 6.64 (d, J = 8.4 Hz, 1H, ArH), 5.46 (s, 2H, OCH₂), 4.18 (s, 2H, NCH₂), 3.59 (s, 2H, COCH₂), 3.38-3.05 (m, 4H, NCH₂CH₂).

HRMS (ESI): m/z [M+H]⁺ Calcd for C₂₃H₂₁CIFN₂O₃: 427.1225 Found: 427.1223.

In preparation of I-D-26, a side product was obtained, structurally identified as I-D-27, m/z: 369.10 (M+H). ¹H-NMR (400 MHz, DMSO-*d*₆) δ: 7.66 (dd, J =7.6 &1.6Hz, 1H), 7.53-7.47 (m, 1H), 7.48-7.44 (m, 1H), 7.41-7.30 (m, 5H), 6.71 (d, 1H, J =8.4Hz), 5.44 (s, 2H), 3.78 (s, 2H), 3.01-2.98 (m, 1H), 2.70-2.67 (m, 1H).

### Example 40

Synthesis of methyl 2-(7-((2,2'-dimethyl-[1,1'-biphenyl]-3-yl)methoxy)-1-methyl-1,4-dihydropyrido[2,3-*d*] pyrimidin-3(2*H*)-yl)acetate **(I-E-1:** R¹=CH₃, R²= 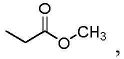 R³=CH₃, and R⁴=H)

### Synthesis of tert-butyl (6-chloro-3-formylpyridin-2-yl)(methyl)carbamate (XVII)

A compound **XVI** (100 mg, 0.39 mmol) was added into a 25 mL eggplant shaped flask and was dissolved in DMF (10 mL), CH₃I (49 µL, 0.78 mmol) was slowly added dropwise into the reaction solution, the temperature was reduced to 0°C, 60% NaH (18 mg, 0.469 mmol) was added in batches, after the addition was completed, stirring was continued to be performed in an ice bath for 30 min, and a reaction was carried out overnight at room temperature. After the raw materials were monitored by TLC (petroleum ether:ethyl acetate=15:1) to be almost complete, the reaction was quenched with saturated ammonium chloride, the resulting reaction solution was extracted with ethyl acetate (10 mL×2), organic phases were mixed, washed with a saturated salt solution (10 mL×2), dried over anhydrous sodium sulfate, concentrated under reduced pressure to give a crude product, and the crude product was purified by column chromatography to give 0.09 g of a colorless liquid (yield: 85.2%).

¹H NMR (300 MHz, Chloroform-d) δ 9.90 (s, 1H, CHO), 8.13 (d, J = 8.1 Hz, 1H, ArH), 7.29 (d, J = 8.1 Hz, 1H, ArH), 3.44 (s, 3H, NCH₃), 1.43 (s, 9H, CH₃).

### Synthesis of methyl ((2-((tert-butoxycarbonyl)(methyl)amino)-6-chloropyridin-3-yl)methyl)glycinate (XVIII-1)

A compound **XVII** (0.70 g, 2.73 mmol), glycine methyl ester hydrochloride (0.69 g, 5.47 mmol) and dichloromethane (10 mL) were added into a 25 mL eggplant shaped flask, stirring was performed at room temperature for 1 h, and sodium triacetoxyborohydride (1.16 g, 5.47 mmol) was added in batches at 0°C. After the addition was complete, a reaction was carried out at room temperature for 4 h. After the raw materials were monitored by TLC to be completely reacted, saturated sodium carbonate was added dropwise to adjust a pH to 7, water (5 mL) was added, the resulting reaction solution was extracted with dichloromethane (10 mL×3), organic phases were mixed, washed with a saturated salt solution (10 mL×3), dried over anhydrous sodium sulfate, and concentrated under reduced pressure to give an oily crude product, and the oily crude product was separated by column chromatography to give 0.35 g of a colorless liquid (yield: 37.4%).

¹H NMR (300 MHz, Chloroform-*d*) δ 7.95 (d, J = 8.0 Hz, 1H, ArH), 7.29 (d, J = 8.2 Hz, 1H, ArH),4.86 (br, 1H, NH) 3.78 (s, 3H, OCH₃), 3.75 (s, 2H, ArCH₂), 3.42 (s, 2H, CH₂CO), 3.28 (s, 3H, NCH₃), 1.46 (s, 9H, CH₃).

### Synthesis of methyl ((6-chloro-2-(methylamino)pyridin-3-yl)methyl)glycinate (XIX-1)

The compound **XVIII-1** (0.67 g, 1.95 mmol) was added into a 50 mL eggplant shaped flask and was dissolved in ethyl acetate (5 mL), a solution of ethyl acetate in hydrogen chloride was added, after a reaction was carried out at room temperature for 12 h, a white solid was precipitated, and suction filtration was performed to give 0.34 g of a white solid (yield: 71.6%). m.p.110-112°C.

¹H NMR (300 MHz, DMSO-*d*₆) δ 9.67 (s, 2H, NH-HCl), 7.58 (d, J = 7.7 Hz, 1H, ArH), 6.60 (d, J = 7.6 Hz, 1H, ArH), 4.11 (s, 2H, ArCH₂), 4.03 (s, 2H, CH₂CO), 3.72 (s, 3H, OCH₃), 2.79 (s, 3H, NHCH₃).

### Synthesis of methyl 2-(7-chloro-1-methyl-1,4-dihydropyrido[2,3-d]pyrimidin-3(2H)-yl)acetate (XX-1)

The compound **XIX-1** (0.25 g, 0.90 mmol) was added into a 25 mL eggplant shaped flask and was dissolved in methanol (5 mL), under nitrogen protection, triethylamine (249 µL, 1.80 mmol) was added, stirring was performed for 10 min, paraformaldehyde (0.03 g, 1.08 mmol) was added, and a reaction was carried out at room temperature for 8 h. After the raw materials were monitored by TLC to be complete, an organic solvent was removed through concentration under reduced pressure, water was added, extraction was performed with dichloromethane (10 mL×3), organic phases were mixed, washed with a saturated salt solution (10 mL×3), dried over anhydrous sodium sulfate and concentrated under reduced pressure to give an oily crude product, and the oily crude product was separated by column chromatography to give 0.20 g of a colorless liquid (yield: 87.4%).

¹H NMR (300 MHz, Chloroform-*d*) δ 7.01 (d, J = 7.5 Hz, 1H, ArH), 6.50 (d, J = 7.5 Hz, 1H, ArH), 4.28 (s, 2H, NCH₂N), 3.97 (s, 2H, ArCH₂), 3.75 (s, 3H, OCH₃), 3.43 (s, 2H, CH₂CO), 3.05 (s, 3H, NCH₃).

### Synthesis of methyl 2-(7-((2,2'-dimethyl-[1,1'-biphenyl]-3-yl)methoxy)-1-methyl-1,4-dihydropyrido[2,3-d]pyrimidin-3(2H)-yl)acetate (I-E-1)

The compound **XX-1** (170 mg, 0.67 mmol), the compound **XV-2** (173 mg, 0.80 mmol), t-BuXphos (57 mg, 0.13 mmol) and toluene (5 mL) were sequentially added to a three-necked flask, followed by addition of cesium carbonate (430 mg, 1.33 mmol), under nitrogen protection, Pd(OAc)₂ (15 mg, 0.07 mmol) was added, the temperature was raised to 80°C, and a reaction was carried out for 12 h. After the raw materials were monitored by TLC (petroleum ether:ethyl acetate=4:1) to be completely reacted, heating was stopped, and the resulting reaction solution was cooled to room temperature. A palladium catalyst and an insoluble substance were removed by suction filtration, diluting was performed with water (10 mL), the reaction solution was extracted with ethyl acetate (10 mL×3), and organic phases were mixed, washed with a saturated salt solution (10 mL×3), and dried over anhydrous magnesium sulfate. Suction filtration was performed, a solvent was removed through distillation under reduced pressure, and a residue was purified by column chromatography (petroleum ether:ethyl acetate=20:1-10:1) to give 150 mg of a colorless clear liquid (yield: 51.79%).

¹H NMR (400 MHz, Chloroform-*d*) δ (ppm): 7.56-7.35 (m, 2H, ArH), 7.29 (d, J = 1.8 Hz, 1H, ArH), 7.27-7.20 (m, 2H, ArH), 7.12-7.08 (m, 3H, ArH), 5.98-5.96 (m, 1H, ArH), 5.50-5.32 (s, 2H, OCH₂), 3.79 (s, 3H, CH₃), 4.13(s, 2H, NCH₂N), 3.69 (q, J = 4.1 Hz, 2H, NCH₂), 3.42 (dd, J = 3.1 Hz, 2.6 Hz, 2H, COCH₂), 3.03 (q, J = 3.8 Hz, 2.7 Hz, 3H, ArCH₃), 2.60 (s, 3H, NCH₃), 2.11 (s, 3H, ArCH₃).

### Example 41

Synthesis of methyl (R)-2-(7-((3'-(3-(3-hydroxypyrrolidin-1-yl)propoxy)-2,2'-dimethyl-[1,1'-biphenyl]-3-yl)methoxy)-1-methyl-1,4-dihydropyrido[2,3-*d*]pyrimidin-3(2*H*)-yl)acetate **(I-E-2:** R¹=CH₃, R²= 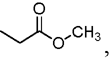 R³=CH₃, and R⁴=

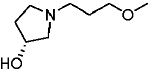

)

The compound **XV-2** (0.43 g, 0.64 mmol), the compound **XX-1** (0.25 g, 0.93 mmol), and Cs₂CO₃ (0.61 g, 1.56 mmol) were added into 50 mL sealed tube, Pd(OAc)₂ (0.02 g, 0.09 mmol), t-BuXPhos (0.08 g, 0.19 mmol) and toluene (10 mL) were added, N₂ was introduced for protection, and reflux was performed at 80°C for 36 h. After most of the raw materials were monitored by TLC to be completely reacted, suction filtration was performed with celite, a liquid was concentrated under reduced pressure to give a crude product, and the crude product was separated by column chromatography to give 110 mg of a light yellow oil which was purified to obtain 90 mg of a light yellow oil (yield: 16.8%).

¹H NMR (300 MHz, Chloroform-d) δ 7.50 (d, J = 7.5 Hz, 1H, ArH), 7.30-7.21 (m, 2H, ArH), 7.15-7.09 (m, 2H, ArH), 6.90 (d, J = 7.9 Hz, 1H, ArH), 6.81 (d, J = 7.5 Hz, 1H, ArH), 6.12 (d, J = 7.9 Hz, 1H, ArH), 5.44 (s, 2H, OCH₂), 4.45 (s, 1H, OH), 4.31 (s, 2H, NCH₂N), 4.19-4.08 (m, 2H, OCH₂), 4.02 (s, 2H, NCH₂), 3.82 (s, 3H, OCH₃), 3.54 (s, 2H, COCH₂), 3.11 (s, 3H, NCH₃), 2.93-2.90 (m, 1H, 1/2CH₂), 2.86-2.81 (m, 2H, CH₂), 2.71-2.64 (m, 1H, 1/2CH₂), 2.50-2.43 (m, 1H, 1/2CH₂), 2.34-2.24 (m, 3H, 1/2CH₂, CH₂), 2.15 (s, 3H, ArCH₃), 1.99 (s, 3H, ArCH₃), 2.00-1.83 (m, 2H, CH₂).

### Example 42

Synthesis of 2-(7-((2,2'-dimethyl-[1,1'-biphenyl]-3-yl)methoxy)-1-methyl-1,4-dihydropyrido[2,3-*d*]pyrimidin-3(2*H*)-yl)acetic acid **(I-E-3:** R¹=CH₃, R²= 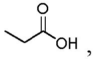 R³=CH₃, and R⁴=H)

The compound **I-E-1** (0.20 g, 0.46 mmol) was added into a 10 mL eggplant shaped flask, methanol (2 mL) was added to dissolve the compound I-E-1, 2M LiOH (1 mL) was added, and stirring was performed at room temperature for 4 h. After the reaction was monitored by TLC to be complete, the methanol was removed through distillation under reduced pressure, a pH was adjusted to 5-6 with a 1M HCl solution, so that a white solid was precipitated, suction filtration was performed, and a crude product was slurried with dichloromethane for three times to give 70 mg of a white solid product (yield: 36.5%). m.p.132-134°C.

¹H NMR (400 MHz, Chloroform-d) δ 7.59-7.38 (m, 2H, ArH), 7.32 (d, J = 1.8 Hz, 1H, ArH), 7.30-7.23 (m, 2H, ArH), 7.15-7.12 (m, 3H, ArH), 6.01-5.97 (m, 1H, ArH), 5.53 (s, 2H, OCH₂), 4.16 (s, 2H, NCH₂N), 3.72 (q, J = 4.1 Hz, 2H, NCH₂), 3.45 (dd, J = 3.1 Hz, 2.6 Hz, 2H, COCH₂), 3.06 (s, 3H, ArCH₃), 2.63 (s, 3H, NCH₃), 2.14 (s, 3H, ArCH₃).

HRMS (ESI): m/z [M+H]⁺ Calcd for C₂₅H₂₈N₃O₃: 418.2030; Found: 418.2132.

### Example 43

Synthesis of (R)-2-(7-((3'-(3-(3-hydroxypyrrolidin-1-yl)propoxy)-2,2'-dimethyl-[1,1'-biphenyl]-3-yl)methoxy)-1-methyl-1,4-dihydropyrido[2,3-d]pyrimidin-3(2H)-yl)acetic acid (I-E-4: R¹=CH₃, R²= 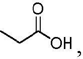 R³=CH₃, and R⁴= 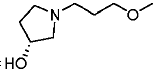 )
80 mg of an off-white solid product was obtained by using the compound **I-E-2** (90 mg, 0.16 mmol) and 2M LiOH (1 mL) as raw materials and by using the same procedures as those for **I-E-3** (yield: 54.7%). m.p.162-164°C.

¹H NMR (300 MHz, DMSO-*d*₆) δ 7.43 (d, J = 7.5 Hz, 1H, ArH), 7.26 (d, J = 8.1 Hz, 1H, ArH), 7.2 (d, J = 8.5 Hz, 1H, ArH), 7.15 (d, J = 7.8 Hz, 1H, ArH), 7.04 (d, J = 7.5 Hz, 1H, ArH), 6.97 (d, J = 8.2 Hz, 1H, ArH), 6.69 (d, J = 7.6 Hz, 1H, ArH), 6.01 (d, J = 7.8 Hz, 1H, ArH), 5.36 (s, 2H, OCH₂), 4.25 (s, 1H, OH), 4.16 (s, 2H, NCH₂N), 4.13-4.00 (m, 3H, OCH₂CH₂, CH), 3.82 (s, 2H, ArCH₂N), 3.32 (s, 4H, CHCH₂N, NCH₂), 2.97 (s, 3H, NCH₃), 2.90-2.79 (m, 1H, 1/2NCH₂CH₂), 2.79-2.68 (m, 3H, 1/2NCH₂CH₂, NCH₂CH₂), 2.67-2.58 (m, 1H, 1/2CH₂CH₂CH), 2.02 (s, 3H, ArCH₃), 1.85 (s, 3H, ArCH₃), 1.99-1.91 (m, 2H, CH₂), 1.70-1.65 (m, 1H, 1/2CH₂CH₂CH).

HRMS (ESI): m/z [M+H]⁺ Calcd for C₃₂H₄₁N₄O₅: 561.3077; found: 561.3079.

### Example 44

Synthesis of ethyl 2-(2-((2-chloro-2'-fluoro-[1,1'-biphenyl]-3-yl)carbamoyl)-7,8-dihydro-1,6-naphthyridin-6(5*H*)-yl)acetate **(I-F-1:** R¹=Cl, R²= 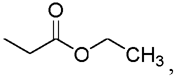 R³=F, and R⁴=H)

Synthesis of tert-butyl 2-chloro-7,8-dihydro-1,6-naphthyridine-6(5*H*)-acetate **(XXI)** 2-Chloro-5,6,7,8-tetrahydro-1,6-naphthyridine hydrochloride **VIII** (250 mg, 1.22 mmol) was added into a 25 mL eggplant shaped flask and was dissolved in 5 mL of DCM, triethylamine (508 µL, 3.66 mmol) was added dropwise, (Bco)₂O (399 mg, 1.83 mmol) was added, after the raw materials were monitored to be completely reacted, the reaction solution was diluted with 5 mL of water, washing was performed with a saturated salt solution, and drying was performed by using anhydrous sodium sulfate. Suction filtration was performed, a solvent was removed through distillation under reduced pressure, and a crude product was purified to give 320 mg of a white solid (yield: 97.7%). m.p.66-68°C.

¹H NMR (300 MHz, Chloroform-d) δ 7.39 (d, J = 8.1 Hz, 1H, ArH), 7.18 (d, J = 8.1 Hz, 1H, ArH), 4.57 (s, 2H, NCH₂), 3.74 (t, J = 5.9 Hz, 2H, NCH₂CH₂), 2.99 (t, J = 6.0 Hz, 2H, NCH₂CH₂), 1.50 (s, 9H, CH₃).

### Synthesis of tert-butyl 2-cyano-7,8-dihydro-1,6-naphthyridine-6(5H)-acetate (XXII)

A compound **XXI** (500 mg, 1.86 mmol) and zinc cyanide (240 mg, 2.05 mmol) were added into a three-necked flask and were dissolved in 5 mL of DMF, under nitrogen protection, Pd(PPh₃)₄ (215 mg, 0.19 mmol) was added, the temperature was raised to 120°C, a reaction was carried out for 10 h, after the raw materials were monitored by TLC (petroleum ether:ethyl acetate=2:1) to be completely reacted, heating was stopped, and the reaction solution was cooled to room temperature. A palladium catalyst and an insoluble substance were removed by suction filtration, diluting was performed with water (5 mL), the reaction solution was extracted with ethyl acetate (5 mL×3), and organic phases were mixed, washed with a saturated salt solution, and dried over anhydrous magnesium sulfate. Suction filtration was performed, a solvent was removed through distillation under reduced pressure, and a residue was purified by column chromatography (petroleum ether:ethyl acetate=20:1-5:1) to give 300 mg of a white solid (yield: 62.2%). m.p.112-114°C.

¹H NMR (300 MHz, Chloroform-*d*) δ 7.54 (s, 2H, ArH), 4.66 (s, 2H, NCH₂), 3.77 (t, J = 6.0 Hz, 2H, NCH₂CH₂), 3.04 (t, J = 6.0 Hz, 2H, NCH₂CH₂), 1.49 (s, 9H, CH₃).

### Synthesis of 6-(2-(tert-butoxy)-2-oxoethyl)-5,6,7,8-tetrahydro-1,6-naphthyridine-2-carboxylic acid (XXIII)

The compound **XXII** (1.00 g, 3.86 mol) was dissolved in methanol, an aqueous KOH (1.08 g, 19.28 mol) solution was added, the temperature was raised to 80°C, a reaction was carried out for 12 h, after the raw materials was monitored by TLC (dichloromethane:methane=10:1) to be completely reacted, the reaction was stopped, and the reaction solution was cooled to room temperature. A pH was adjusted to be neutral with dilute hydrochloric acid, diluting was performed with 5 mL of water, extraction was performed with ethyl acetate (5 mL×3), and organic phases were mixed, washed with a saturated salt solution, and dried over anhydrous magnesium sulfate. Suction filtration was performed, a solvent was removed through distillation under reduced pressure, and a residue was separated by column chromatography (dichloromethane:methanol=25:1-5:1) to give 630 mg of a white solid (yield: 58.7%). m.p.162-164°C.

¹H NMR (300 MHz, Chloroform-*d*) δ 8.05 (s, 1H, ArH), 7.66 (s, 1H, ArH), 4.69 (s, 2H, NCH₂), 3.78 (s, 2H, NCH₂CH₂), 3.06 (s, 2H, NCH₂CH₂), 1.50 (s, 9H, CH₃).

### Synthesis of 2-chloro-2'-fluoro-[1,1'-biphenyl]-3-amine (IV-2)

3-Bromo-2-chloroaniline **II-2** (1.0 g, 4.80 mmol), 2-fluorobenzeneboronic acid **III-1** (1.02 g, 7.30 mmol) and 20 mL of 1,4-dioxane were sequentially added to a three-necked flask, potassium carbonate (1.87 g, 13.6 mmol) was dissolved in water to be added to the reaction solution, under nitrogen protection, Pd(PPh₃)₄ (0.28 g, 0.20 mmol) was added, the temperature was raised to 80°C, and a reaction was carried out for 10 h. After the raw materials were monitored by TLC (petroleum ether:ethyl acetate=15:1) to be completely reacted, heating was stopped, and the resulting reaction solution was cooled to room temperature. A palladium catalyst and an insoluble substance were removed by suction filtration, diluting was performed with water (5 mL), the reaction solution was extracted with ethyl acetate (5 mL×3), and organic phases were mixed, washed with a saturated salt solution, and dried over anhydrous magnesium sulfate. Suction filtration was performed, a solvent was removed through distillation under reduced pressure, and a residue was purified by column chromatography to give 0.97 g of a white solid powder (yield: 91.4%). m.p.78-80°C.

¹H NMR (300 MHz, DMSO-*d*₆) δ 7.43-7.40 (m, 1H, ArH), 7.32-7.22 (m, 3H, ArH), 7.08 (dd, J = 8.1 Hz, 7.4 Hz, 1H, ArH), 6.85 (dd, J = 8.1 Hz, 1.6 Hz, 1H, ArH), 6.50 (dd, J = 7.4 Hz, 1.6 Hz, 1H, ArH), 5.48 (s, 2H, NH).

### Synthesis of tert-butyl 2-((2-chloro-2'-fluoro-[1,1'-biphenyl]-3-yl)carbamoyl)-7,8-dihydro-1,6-naphthyridine-6(5H)-yl)carboxylate (XXIV)

The compound **XXIII** (500 mg, 1.80 mmol) and the compound **IV-2** (397 mg, 1.80 mmol) were dissolved in dichloromethane, HATU (820 mg, 2.16 mmol) and DIPEA (697 mg, 5.39 mmol) were added, and a reaction was carried out at room temperature for 12 h. After the raw materials were monitored by TLC (dichloromethane:methanol=25:1) to be completely reacted, the reaction was stopped. The reaction was diluted with 5 mL of water and was extracted with ethyl acetate (5 mL×3), and organic phases were mixed, washed with a saturated salt solution, and dried over anhydrous magnesium sulfate. Suction filtration was performed, a solvent was removed through distillation under reduced pressure, and a residue was separated and purified by column chromatography to give 580 mg of a white solid (yield: 67.1%). m.p.134-136°C.

¹H NMR (300 MHz, Chloroform-d) δ 10.86 (s, 1H, NH), 8.70 (dd, J = 8.3 Hz, 1.6 Hz, 1H, ArH), 8.13 (d, J = 7.9 Hz, 1H, ArH), 7.63 (d, J = 8.0 Hz, 1H, ArH), 7.45-7.37 (m, 2H, ArH), 7.30-7.34 (m, 1H, ArH), 7.24-7.20 (m, 1H, ArH), 7.19-7.13 (m, 1H, ArH), 7.11 (dd, J = 7.6 Hz, 1.6 Hz, 1H, ArH), 4.68 (s, 2H, NCH₂), 3.79 (t, J = 5.9 Hz, 2H, NCH₂CH₂), 3.07 (t, J = 6.0 Hz, 2H, NCH₂CH₂), 1.51 (s, 9H, CH₃).

### Synthesis of N-(2-chloro-2'-fluoro-[1,1'-biphenyl]-3-yl)-5,6,7,8-tetrahydro-1,6-naphthyridine-2-carboxamide (XXV)

The compound **XXIV** (580 mg, 1.21 mmol) was dissolved in methanol, and a solution of hydrogen chloride in 1,4-dioxane was slowly added dropwise. After the raw materials were monitored by TLC (dichloromethane:methanol=15:1) to be completely reacted, a solvent was removed through distillation under reduced pressure to give 616 mg of a light yellow solid (yield: 99.9%). m.p. higher than 250°C.

¹H NMR (400 MHz, DMSO-*d*₆) δ 10.72 (s, 1H, NH), 8.45 (m, 1H, ArH), 8.11 (d, J = 8.0 Hz, 1H, ArH), 8.00 (d, J = 8.0 Hz, 1H, ArH), 7.50-7.55 (m, 2H, ArH), 7.43-7.33 (m, 3H, ArH), 7.25 (dd, J = 7.6 Hz, 1.6 Hz, 1H, ArH), 4.44 (s, 2H, NCH₂), 3.54 (s, 2H, NCH₂CH₂), 3.24 (t, J = 6.2 Hz, 2H, NCH₂CH₂).

Synthesis of ethyl 2-(2-((2-chloro-2'-fluoro-[1,1'-biphenyl]-3-yl)carbamoyl)-7,8-dihydro-1,6-naphthyridin-6(5*H*)-yl)acetate **(I-F-1)** The compound **XXV** (250 mg, 0.66 mmol) was dissolved in THF, 60% NaH (31.5 mg, 1.31 mmol) was added slowly under an ice bath, after dropwise addition, the mixture was transferred to room temperature, and a reaction was carried out for 30 min, and ethyl bromoacetate (87 µL, 0.79 mmol) was added slowly dropwise to the reaction solution. After the raw materials were monitored by TLC (dichloromethane:methanol=25:1) to be completely reacted, the reaction was stopped. The reaction was quenched with a saturated NH₄Cl solution, diluting was performed with 5 mL of water, extraction was performed with ethyl acetate (5 mL×3), drying was performed by using anhydrous sodium sulfate, suction filtration was performed, and a solvent was removed through distillation under reduced pressure. A residue was separated by column chromatography to give 126 mg of a light yellow solid (yield: 41.11%). m.p.158-160°C.

¹H NMR (400 MHz, Chloroform-*d*) δ 10.86 (s, 1H, NH), 8.70 (d, J = 8.2 Hz, 1H, ArH), 8.08 (d, J = 7.9 Hz, 1H, ArH), 7.55 (d, J = 7.9 Hz, 1H, ArH), 7.44-7.36 (m, 2H, ArH), 7.34-7.29 (m, 1.7 Hz, 1H, ArH), 7.23-7.15 (m, 2H, ArH), 7.14-7.09 (m, 1H, ArH), 4.28 (q, J = 7.1 Hz, 2H, CH₃CH₂), 3.99 (s, 2H, NCH₂), 3.53 (s, 2H, COCH₂), 3.22-3.07 (m, 4H, NCH₂CH₂), 1.31 (t, J = 7.1 Hz, 3H, CH₃).

Example 45

Synthesis of N-(2-chloro-2'-fluoro-[1,1'-biphenyl]-3-yl)-6-(2-hydroxyethyl)-5,6,7,8-tetrahydro-1,6-naphthyridine-2-carboxamide **(I-F-2:** R¹=Cl, R²= 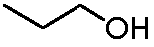 , R³=F, and R⁴=H)

The compound **I-F-1** (60 mg, 0.13 mmol) was dissolved in THF, LiAIH₄ (10 mg, 0.26 mmol) was added under an ice bath, the mixture was transferred to room temperature, and a reaction was carried out. After the raw materials were monitored by TLC (dichloromethane:methanol=25:1) to be completely reacted, the reaction was stopped. The reaction was quenched with a saturated NH₄Cl solution, diluting was performed with 5 mL of water, extraction was performed with ethyl acetate (5 mL×3), drying was performed by using anhydrous sodium sulfate, suction filtration was performed, and a solvent was removed through distillation under reduced pressure. A residue was separated by column chromatography to give 36 mg of a white solid (yield: 54.6%). m.p.88-90°C.

¹H NMR (300 MHz, Chloroform-d) δ 10.85 (s, 1H, NH), 8.70 (d, J = 8.4 Hz, 1H, ArH), 8.09 (d, J = 7.9 Hz, 1H, ArH), 7.57 (d, J = 7.9 Hz, 1H, ArH), 7.40 (t, J = 7.7 Hz, 2H, ArH), 7.35-7.28 (m, 1H, ArH), 7.23-7.09 (m, 2H, ArH), 3.88 (s, 2H, NCH₂), 3.79 (t, J = 5.3 Hz, 2H, CH₂OH), 3.16 (t, J = 5.4 Hz, 2H, NCH₂CH₂), 3.04 (t, J = 5.7 Hz, 2H, NCH₂CH₂), 2.84 (t, J = 5.3 Hz, 2H, CH₂CH₂OH).

HRMS (ESI): m/z [M+H]⁺ Calcd for C₂₃H₂₂CIFN₃O₂: 426.1385; found: 426.1382.

### Example 46

Synthesis of 2-(2-((2-chloro-2'-fluoro-[1,1'-biphenyl]-3-yl)carbamoyl)-7,8-dihydro-1,6-naphthyridin-6(5*H*)-yl)acetic acid **(I-F-3:** R¹=Cl, R²= 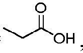 , R³=F, and R⁴=H)
The compound **I-F-1** (60 mg, 0.13 mmol) was dissolved in ethanol, an aqueous NaOH (10 mg, 0.26 mmol) solution was added, and stirring was performed at room temperature. After the raw materials were monitored by TLC (dichloromethane:methanol=5:1) to be completely reacted, the reaction was stopped. The reaction was diluted with 5 mL of water and was extracted with ethyl acetate (5 mL×3), and organic phases were mixed, washed with a saturated salt solution, and dried over anhydrous magnesium sulfate. Suction filtration was performed, a solvent was removed through distillation under reduced pressure, and a residue was purified by column chromatography to give 42 mg of a white solid (yield: 74.5%). m.p.224-226°C.

¹H NMR (300 MHz, DMSO-*d*₆) δ 10.75 (s, 1H, NH), 8.49 (d, J = 8.2 Hz, 1H, ArH), 8.01-7.90 (m, 1H, ArH), 7.81-7.69 (m, 1H, ArH), 7.54-7.45 (m, 2H, ArH), 7.40-7.28 (m, 3H, ArH), 7.24-7.13 (m, 1H, ArH), 3.84 (s, 2H, NCH₂), 3.14 (s, 2H, COCH₂), 3.03-2.88 (m, 4H, NCH₂CH₂).

HRMS (ESI): m/z [M+H]⁺ Calcd for C₂₃H₂₀CIFN₃O₃: 440.1177; found: 440.1172.

### Example 47

### Evaluation of inhibitory activity of the compounds of the present invention on PD-1/PD-L1 protein-protein interaction

Experimental objective: the activity of the compounds of formula (I) to inhibit the PD-1/PD-L1 interaction was tested by using a PD-1/PD-L1 binding assay kit (CISBIO).

Experimental principle: HTRF (Homogeneous Time-Resolved Fluorescence) is a technique used to detect substances to be tested in pure liquid phase systems. It primarily utilizes energy transfer of two fluorescent groups, which are divided into an energy donor europium (Eu⁺) and an energy acceptor. When the donor is externally excited (e.g., a flash lamp or laser), the energy can be resonantly transferred to the acceptor if the donor is close enough to the acceptor, and the acceptor is excited at a specific wavelength. By using the HTRF technology, the assay enables simple and rapid characterization of compounds and antibody blockers in a high-throughput format. By using anti-Tag1 labeled with Eu⁺ (Europium) (the HTRF energy donor) and anti-Tag2 labeled with XL665 (the HTRF energy acceptor), the interaction between PD-L1 and PD-1 can be detected. PD-L1 protein and PD-1 protein are labeled with Tag1 and Tag2, respectively, and Eu⁺ and XL665 bind to PD-L1 and PD-1, respectively, through antibodies to form complexes. When PD-L1 and PD-1 bind close to each other, Eu⁺ will trigger fluorescence resonance energy transfer towards XL665 after being excited by a foreign laser, and XL665 then emits specifically at 665 nm. This specific signal is directly proportional to the degree of PD1/PD-L1 interaction. Thus, a compound or antibody that prevents the PD-1/PD-L1 interaction will result in a decrease in HTRF signal.

Experimental materials: kit: PD-1/PD-L1 binding assay kits purchased from CISBIO; and 96-well plate: purchased from CISBIO.

Test instrument: Perkin Elmer, model: EnVision.

Test compounds: the compounds in formula (I). Dissolving was performed by using DMSO, and diluting was performed with a diluent buffer; and DMSO concentration does not exceed 0.5%.

Experimental procedure: PD-1/PD-L1 binding assay kits were used. A negative group, a positive group and an administration group were set, with 2 replicates in each group. In the positive control group, 2 µL of a diluent, and 4 µL of PD-L1 and 4 µL of PD-1 which were diluted as per instructions were added into a 96-well plate; in the negative control group, 6 µL of a diluent and 4 µL of PD-L1 were added into a 96-well plate; and in the administration group, 2 µL of test compounds of formula (I) (or a positive compound BMS-202), 4 µL of PD-L1 and 4 µL of PD-1 were sequentially added into a 96-well plate. The plates were sealed by using microplate sealers, centrifuging was performed at 1000 rpm for 1 min, and incubation was performed at room temperature for 15 min. The Anti-Tag-Eu³⁺ and Anti-tag-XL665 which were diluted with Buffer were then mixed in equal volumes, then 10 µL of the mixed solution was added to each well, the sealed plates were centrifuged at 1000 rpm for 1 min, and incubation was performed at room temperature for 2 h. The microplate sealers were rmoved, fluorescence intensities at 665 nm and 615 nm were read by using EnVision, and it was calculated that ratio=Signal 665 nm/Signal 620 nm*10⁴. IC₅₀ of the compounds was calculated by using Graphpad. BMS-202 in a patent WO2015034820 of BMS was used as a positive drug in this experiment. Activity data are shown in Table 1.

Values represent IC₅₀ of less than 0.07 µM; B represents 0.07-1 µM; and C means more than 1 µM.

**Table 1 Blocking effect of compounds on hPD-1/hPD-L1 at a protein level**

| Compound No. | PD-1/PD-L1 IC₅₀ (nM) | Compound No. | PD-1/PD-L1 IC₅₀ (nM) |
|---|---|---|---|
| I-A-4 | C | I-D-17 | B |
| I-A-5 | C | I-D-18 | 16.34 |
| I-A-6 | C | I-D-19 | 20.06 |
| I-A-7 | B | I-D-20 | B |
| I-A-8 | B | I-D-21 | 31.14 |
| I-A-9 | 11.04 | I-D-22 | 10.49 |
| I-B-2 | 18.97 | I-D-23 | B |
| I-B-3 | B | I-D-24 | B |
| I-D-13 | 23.0 | I-D-25 | B |
| I-D-14 | 15.8 | I-D-26 | 9.06 |
| I-D-27 | 10.06 | I-D-28 | 24.16 |
| I-D-30 | 13.43 | I-D-15 | B |
| I-D-16 | B | I-E-4 | 58.51 |
| BMS-202 | 65.03 | | |

The experimental results show that the compounds of the present invention have significant inhibitory activity of PD-1/PD-L1 protein-protein interaction, wherein the inhibitory activities of compounds I-A-9, I-B-2, I-D-13, I-D-14, I-D-18, I-D-19, I-D-21, I-D-22, I-D-26, I-D-27, I-D-28, I-D-30 and I-E-4 are superior to that of the compound BMS-202 in the document WO2015034820. This shows that the biphenyl compounds of the present invention can be used as immune checkpoint PD-1/PD-L1 inhibitors.

### Example 48 Acute toxicity test of compounds

### Test samples: Compound I-D-18, Compound I-D-26, and Compound I-D-27.

Animal species and number: SD rats; 6 rats in each group (half male and half female); Mode of administration: oral gavage;
Animal grouping and administration dosage: a vehicle blank group, compound I-D-18 (500 mg/kg, 1000 mg/kg, and 2000 mg/kg) groups, compound I-D-26 (500 mg/kg, 1000 mg/kg, and 2000 mg/kg) groups, and compound I-D-27 (500 mg/kg, 1000 mg/kg, and 2000 mg/kg) groups;
Administration frequency: administration once.

### Experimental procedures:

Side-cage observation on the day of administration (D1): frequency and time of observation: side-cage observation of acute toxicity reactions was performed for 4 h after administration of animals in each group, and detailed clinical observations were performed on animals with apparent abnormal manifestations. Mortality, morbidity, respiration, secretion, feces, and diet, water drinking, etc. were observed, and changes in body weight of rats during administration were recorded. Detailed clinical observations include, but are not limited to, behavioral activities, skin, hair, eyes, ears, noses, abdomens, external genitalia, anuses, limbs, feet, and breathing. The animals in each group were euthanized at the end of the observation period, and all animals were dissected and gross observed.

Experimental results: the compound I-D-18, the compound I-D-26, and the compound I-D-27 were administered to the SD rats at doses of 500 mg/kg, 1000 mg/kg, and 2000 mg/kg by single gavage. No mortality or moribundity was observed in each group of animals. No test sample-related changes were observed grossly in each dose group of animals. Under the conditions of this experiment, the compound I-D-24, the compound I-D-26, and the compound I-D-27 have a maximum tolerated dose (MTD) of greater than or equal to 2000 mg/kg.

### Example 49 Safety test of compounds after repeated administration for 14 days

### Test samples: Compound I-D-18, Compound I-D-26, and Compound I-D-27.

Animal species and number: SD rats; 6 rats in each group (half male and half female); Mode of administration: oral gavage;
Animal grouping and administration dosage: a vehicle blank group, a compound I-D-18 (300 mg/kg) group, a compound I-D-26 (300 mg/kg) group, and a compound I-D-27 (300 mg/kg) group;
Administration frequency: administration once a day for 14 days.

### Experimental procedures:

Side-cage observation of acute toxicity reactions was performed for 4 h after administration, and detailed clinical observations were performed on animals with apparent abnormal manifestations. General clinical observations included observation twice a day during the test (observation once in the morning and observation once in the afternoon). Mortality, morbidity, respiration, secretion, feces, and diet, water drinking, etc. were observed, and changes in body weight of rats during administration were recorded. Detailed clinical observations include, but are not limited to, behavioral activities, skin, hair, eyes, ears, noses, abdomens, external genitalia, anuses, limbs, feet, and breathing. The animals in each group were euthanized at the end of administration, and all animals were dissected and gross observed.

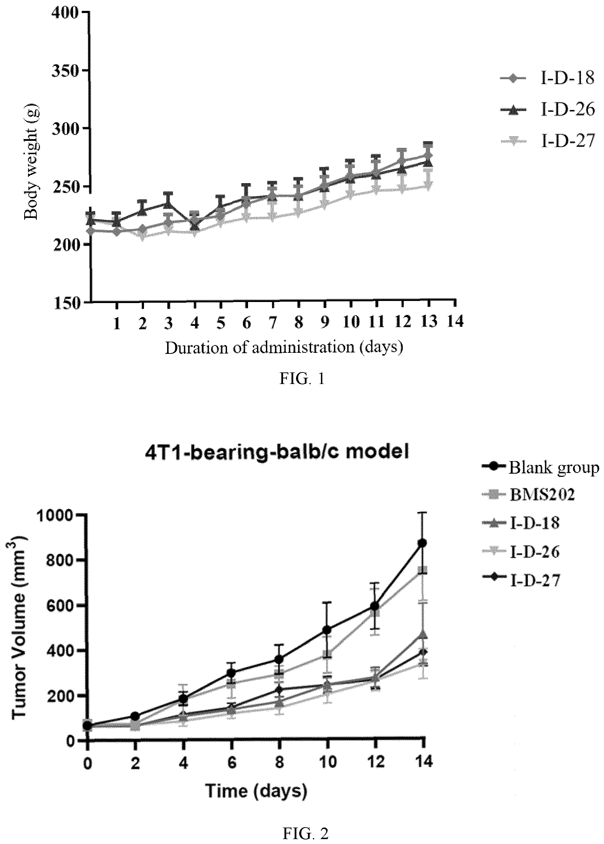

A body weight growth curve of the animals during administration is shown in FIG. 1.

Experimental results: for the compound I-D-18, the compound I-D-26, and the compound I-D-27, within the administration period (14 days), animals in all dose groups had normal water intake and food intake, normal activity, and normal body weight, with no significant abnormalities.

### Example 50 Study on the pharmacodynamic effect of breast cancer tumor cell 4T1 xenograft tumor model

### Modeling and dosing regimen:

Animal species and number: Balb/c Nude; 6 nude mice in each group;
Test samples: Compound I-D-24, Compound I-D-26, and Compound I-D-27
Test group: a blank solvent control group;
Compound I-D-18 (10 mg/kg, i.g., QD×21 days);
Compound I-D-26 (10 mg/kg, i.g., QD×21 days);
Compound I-D-27 (10 mg/kg, i.g., QD×21 days);
Animal model establishment: breast cancer tumor cells 4T1 in a logarithmic growth phase were cultured in vitro, collected and inoculated subcutaneously in the right back of nude mice at a dose of 2×10⁶ cells/mouse in 0.1 mL, and tumor-bearing nude mice were randomized into groups when the tumor volume grew to 50-70 mm³. Subsequently, each group of animals was dosed, and the day of first administration was defined as test day 1;
Administration frequency: once a day;
General state observations: observation time and frequency: once a day; observation indicators or content: including, but not limited to, local administration of the animals, appearance signs, general behavioral activity, mental status, death, and other abnormal manifestations. The animals were euthanized at the end of the test.
Calculation of tumor volume: V=1/2×long diameter×short diameter² (mm³).

### Experimental results:

In the table below, "+" indicates a tumor inhibition rate of less than 20%; "++" indicates a tumor inhibition rate of 20-60%; and "+++" indicates a tumor inhibition rate of more than 60%.

### Tumor inhibition rate of compounds:

| Compound | Tumor inhibition rate |
|---|---|
| I-D-18 | +++ |
| I-D-26 | +++ |
| I-D-27 | +++ |

An animal tumor growth curve of a 4T1 subcutaneous graft tumor model of BALB/c mice administrated with the compounds of the present invention is shown in FIG. 2.

The above data indicate that the compound I-D-18, the compound I-D-26, and the compound I-D-27 have significant tumor-inhibiting efficacy, and the experimental animals had normal water intake and food intake, normal activity, normal body weight, and no toxicity manifestations during the administration test period.

The efficacy and safety of the compounds of the present invention are significantly superior to those of BMS-202, suggesting that the compounds of the present invention have better therapeutic advantages and potential application values.

Example 51 Preliminary study on a capsule formulation of the compound I-D-18 Formula composition:

| **Ingredient** | **Action** | **Dosage per capsule (mg)** |
|---|---|---|
| Compound I-D-18 | Main drug | 100 |
| Starch | Diluent | 100 |
| Starch slurry | Adhesive | Appropriate amount |
| Sodium carboxymethyl starch | Disintegrant | 30 |
| Magnesium stearate | Lubricant | 2 |
| Gelatin hollow capsule | Capsule shell | 1 capsule |

### Capsule preparation method:

**Mixing:** weighed compound I-D-18, starch, and sodium carboxymethyl starch were added to a wet mixing granulator to be mixed.

**Adhesive solution preparation:** purified water was weighed, an appropriate amount of starch was slowly added under stirring, and uniform dispersing was performed with stirring to obtain an adhesive-starch slurry.

**Preparation of soft material:** a soft material was prepared by using a wet mixing granulator, controlling a stirring rotation speed and a shearing rotation speed, slowly adding the starch slurry, stirring, and shearing.

**Granulation:** the prepared soft material was granulated by using an oscillating granulator using a 24-mesh screen to obtain wet granules.

**Drying:** the wet granules were added to a fluid bed granulator to obtain dried granules. **Sizing:** the dried granules were sized by using a sieve of an oscillating granulator to obtain the sized granules, and the sized granules were weighed.

**Mixing:** the sized granules were added to a universal mixer, after mixing was finished, magnesium stearate was added, and total mixing was performed to obtain totally mixed granules.

**Filling:** the totally mixed granules were filled into gelatin hollow capsules by using a filling machine, and qualified capsules were screened out to obtain capsules to be packaged.

Capsule samples with a neat appearance were obtained.

## Claims

1. A compound represented by general formula I, or a hydrate, solvate or pharmaceutically acceptable salt thereof: 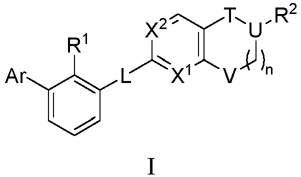 wherein:
Ar represents 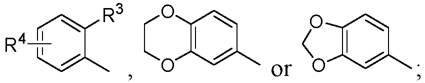
L represents -(CH₂)ₘ-, -O-, -NH-, -CH₂O-, -CF₂O-, -CH₂NH-, -CONH-, - HNCO-, -NHCH₂-, -OCF₂-, -OCH₂- or -CH=CH-, wherein m represents 0, 1 or 2;
X¹ and X² each independently represent N or CH;
T and V each independently represent 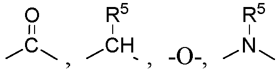 or -S-; wherein R⁵ represents H, C₁-C₆ alkyl or C₃-C₇ cycloalkyl;
U represents CH or N;
n represents 0, 1, 2 or 3;
R¹ and R³ each independently represent H, D, halogen, CN, C₁-C₃ haloalkyl, C₁-C₃ alkyl or cyclopropyl;
R² represents H, substituted C₁-C₆ alkyl, substituted C₃-C₇ cycloalkyl or substituted C₃-C₇ heterocycloalkyl, the substituent being H, OH, NH₂, COOH, an amide group, an ester group, alkoxy or an aldehyde group, and being monosubstituted or polysubstituted, the heterocycloalkyl containing 1 to 3 heteroatoms selected from N, O and S;
R⁴ represents H, halogen, CN, CF₃, OH, NH₂, -O(CH₂)ₚR⁶, substituted C₁-C₆ alkyl, substituted C₃-C₇ cycloalkyl or substituted C₃-C₇ heterocycloalkyl, the substituent being H, OH, NH₂, COOH, an amide group, an ester group or alkoxy, and being monosubstituted or polysubstituted, wherein p represents 1, 2, 3 or 4, the heterocycloalkyl containing 1 to 3 heteroatoms selected from N, O and S; and
R⁶ represents NR⁷R⁸, OR⁷ or substituted C₄-C₆ azacycloalkyl, wherein R⁷ represents H or C₁-C₃ alkyl, R⁸ represents substituted C₁-C₆ alkyl, the C₄-C₆ azacycloalkyl being tetrahydropyrrol-1-yl, piperidin-1-ylmorpholin-1-ylpiperazin-1-yl or azetidin-1-yl, the substituent being OH, NH₂, COOH, an amide group, an ester group or alkoxy, and being monosubstituted or polysubstituted.

2. The compound, or the hydrate, solvate or pharmaceutically acceptable salt thereof according to claim 1, **characterized in that**
Ar represents 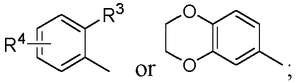
L represents -(CH₂)ₘ-, -CH₂O-, -CF₂O-, -CONH-, -NHCO- or -OCH₂-, wherein m represents 0;
X¹ and X² each independently represent N or CH;
T and V each represent 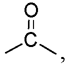 -CH₂-, -O-, -NH- or 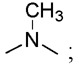
U represents N;
n represents 0 or 1;
R¹ and R³ each independently represent H, D, F, Cl, Br, CN, CH₃ or CF₃;
R² represents H, 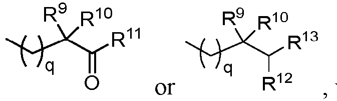
wherein q represents 0 or 1, and R⁹ and R¹⁰ each independently represent H, OH, COOH, CH₂COOH, CH₂NH₂, CH₂OH, CH₂CH₂OH, F, Cl, Br, CH₃ or CH₂CH₃;
R¹¹ represents OH, NH₂, NHCH₃, CH₃, OCH₃, OCH₂CH₃ or OCH(CH₃)₂;
R¹² represents CONH₂, NHCOCH₃, OH, CH₂OH, CH₂CH₂OH, COOH, COOCH₃, COOCH₂CH₃ or COOCH(CH₃)₂;
R¹³ represents H, CH₃, CH₂CH₃, CH₂OH or CH₂CH₂OH; and
R⁴ represents H, F, Cl, Br, CN, CF₃, OH, NH₂ or -O(CH₂)ₚR⁶, wherein p represents 2, 3 or 4, and R⁶ represents OH, 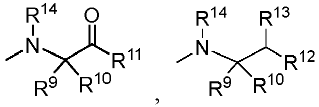 or 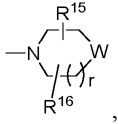 wherein R⁹, R¹⁰, R¹¹, R¹² and R¹³ are as previously defined, R¹⁴ represents CH₃, CH₂CH₃, CH₂CH₂OH, formyl or acetyl, R¹⁵ and R¹⁶ each independently represent H, OH, COOH, NH₂, CH₃, CH₂CH₃, CH₂OH, CH₂CH₂OH, CONH₂, cyclopropyl, COOCH₃, COOCH₂CH₃ or COOCH(CH₃)₂, W represents -CH₂-, -O-, - NH-, 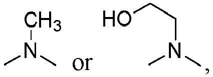 and r represents 0 or 1.

3. The compound, or the hydrate, solvate or pharmaceutically acceptable salt thereof according to claim 1 or 2, **characterized in that** Ar represents 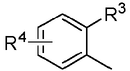 or 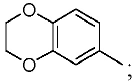
L represents -CH₂O- or- NHCO-;
X¹ represents CH or N;
X² represents CH;
T represents 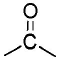 or -CH₂-;
U represents N;
V represents -CH₂- or 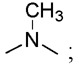
n=0 or 1;
R¹ and R³ each independently represent H, F, Cl, Br, CN, CH₃ or CF₃;
R² represents H, 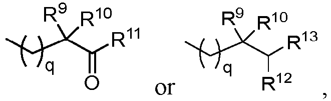 wherein q represents 0 or 1;
R⁹ and R¹⁰ each independently represent H, OH, COOH, CH₂COOH, CH₂OH, CH₃ or CH₂CH₃;
R¹¹ represents OH, NH₂, NHCH₃, OCH₃ or OCH₂CH₃;
R¹² represents CONH₂, NHCOCH₃, OH, CH₂OH, COOH, COOCH₃ or COOCH₂CH₃;
R¹³ represents H, CH₃, CH₂CH₃, CH₂OH or CH₂CH₂OH;
R⁴ represents H, F, Cl, Br, CN, CF₃, OH, NH₂ or -O(CH₂)ₚR⁶, wherein p represents 2, 3 or 4, and R⁶ represents OH, COOH, CH₂OH, NH₂, NHCH₃, CONH₂, NHCOCH₃, COOCH₃, COOCH₂CH₃ or 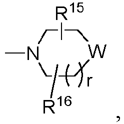 wherein R¹⁵ and R¹⁶ each independently represent H, OH, CH₃, CH₂OH, CONH₂, COOCH₃ or COOCH₂CH₃;
W represents CH₂, O, NH or N-CH₃; and
r represents 0 or 1.

4. The compound, or the hydrate, solvate or pharmaceutically acceptable salt thereof according to any one of claims 1 to 3, **characterized in that** Ar represents 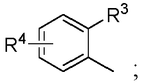
L represents -CH₂O- or -NHCO-;
X¹ represents CH or N, and X² represents CH;
T represents 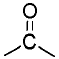 or -CH₂-;
U represents N;
V represents -CH₂-; and
n=0 or 1.

5. The compound, or the hydrate, solvate or pharmaceutically acceptable salt thereof according to any one of claims 1 to 4, **characterized in that** R¹ and R³ each independently represent F, Cl, Br, CN, CH₃ or CF₃.

6. The compound, or the hydrate, solvate or pharmaceutically acceptable salt thereof according to any one of claims 1 to 5, **characterized in that** R² represents H, 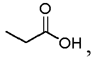 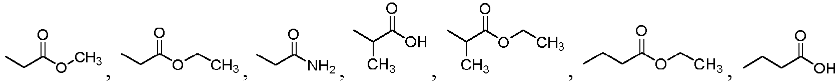 or

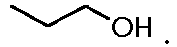

7. The compound, or the hydrate, solvate or pharmaceutically acceptable salt thereof according to any one of claims 1 to 6, **characterized in that** R⁴ represents H, F, Cl, CN, CF₃, OH, NH₂ or -O(CH₂)₃R⁶, wherein R⁶ represents OH, CONH₂, CH₂OH, COOH, COOCH₃, COOCH₂CH₃ or

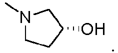

8. A compound selected from any one of the following formulae, or a hydrate, solvate or pharmaceutically acceptable salt thereof:

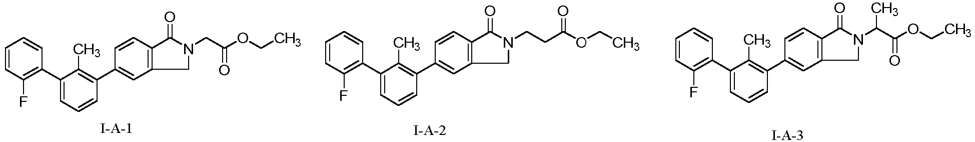

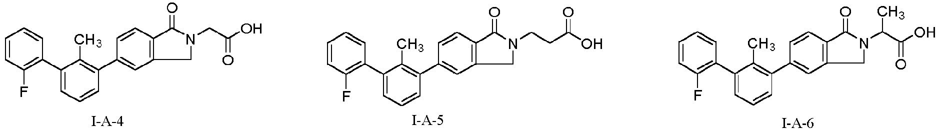

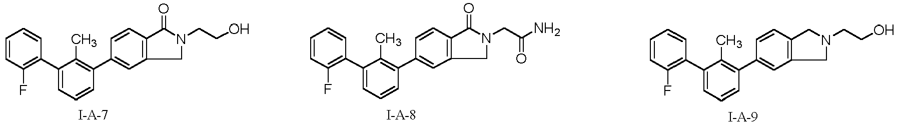

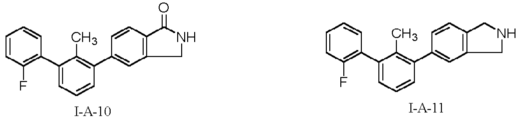

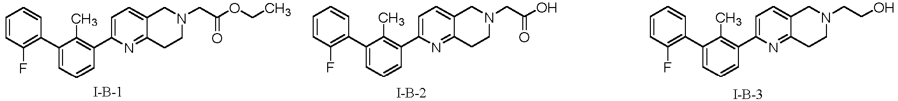

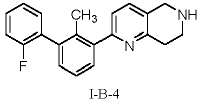

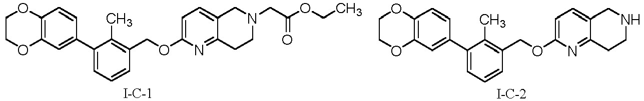

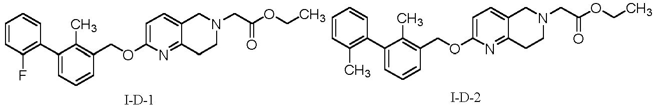

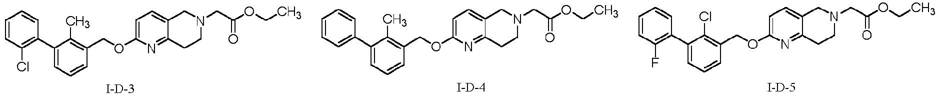

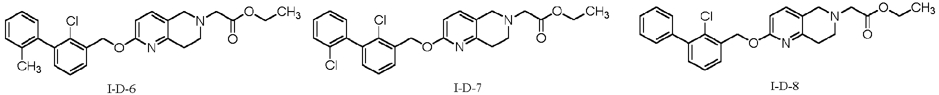

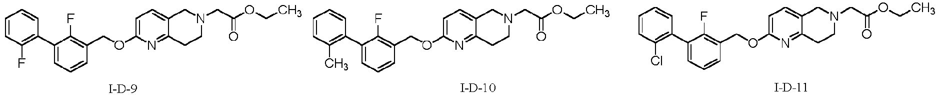

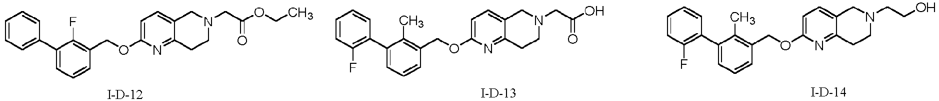

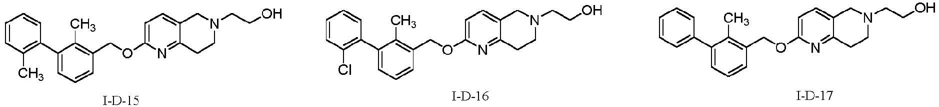

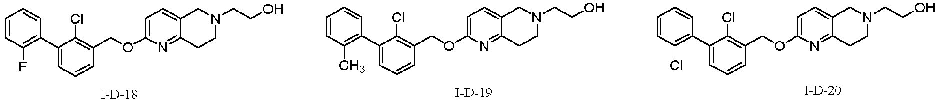

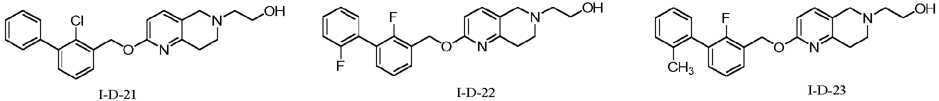

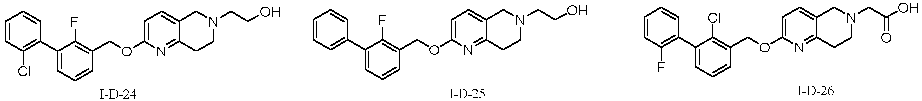

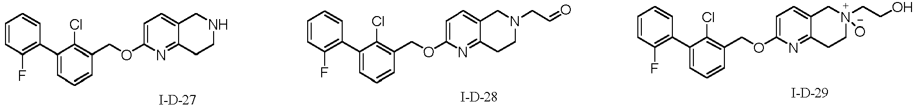

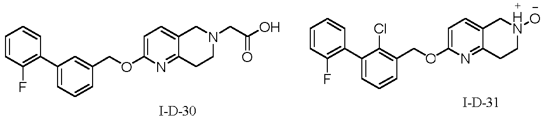

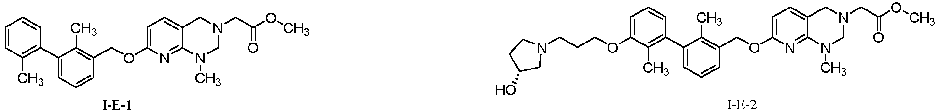

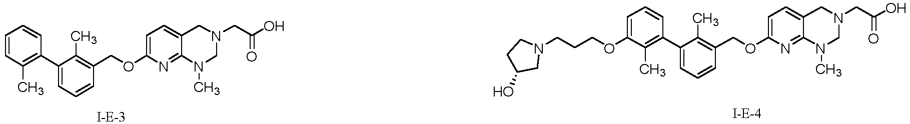

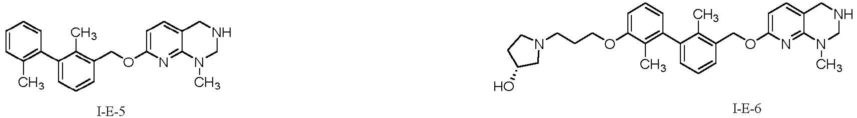

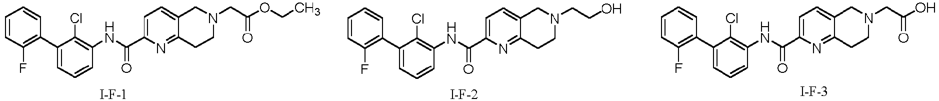

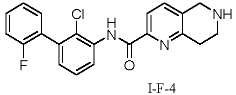

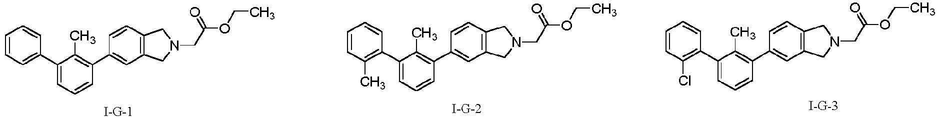

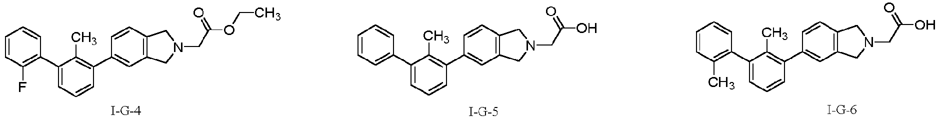

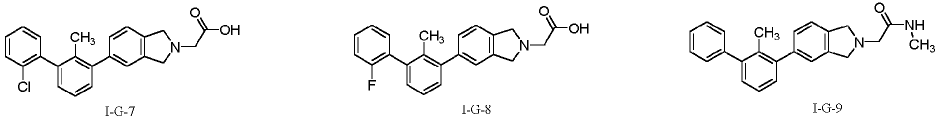

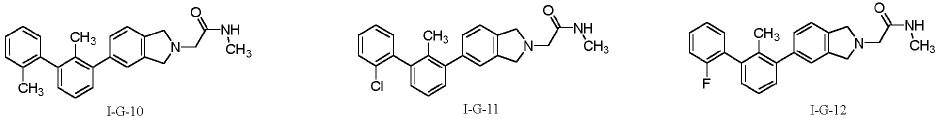

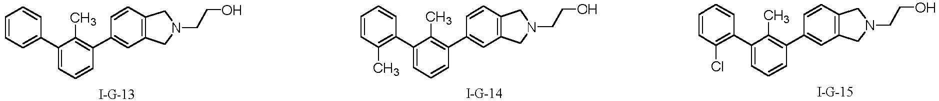

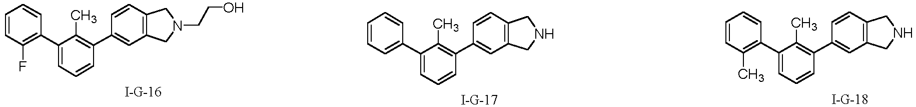

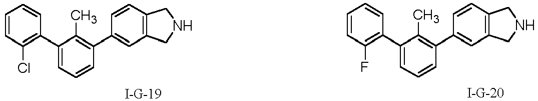

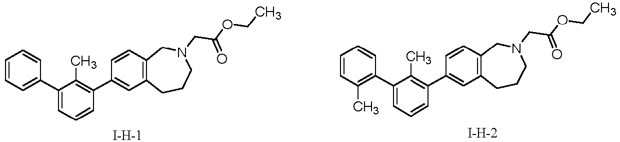

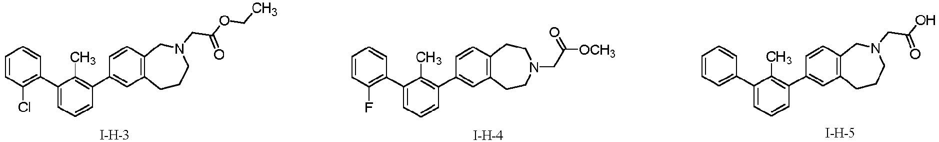

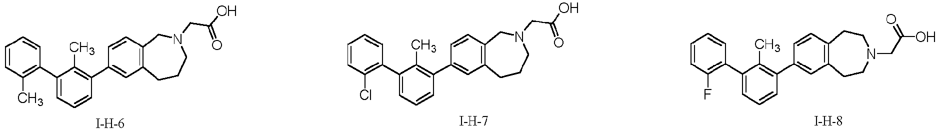

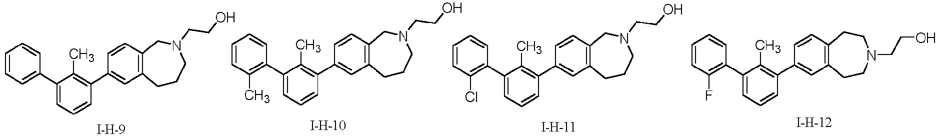

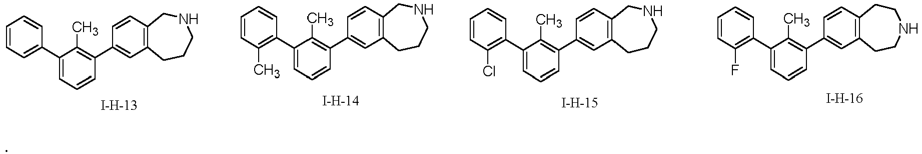

9. The compound, or the hydrate, solvate or pharmaceutically acceptable salt thereof according to claim 8, **characterized in that** the compound is I-A-9, I-B-2, I-D-13, I-D-14, I-D-18, I-D-19, I-D-21, I-D-22, I-D-26, I-D-27, I-D-28, I-D-30 or I-E-4.

10. The compound, or the hydrate, solvate or pharmaceutically acceptable salt thereof according to any one of claims 1 to 9, **characterized in that** the pharmaceutically acceptable salt is an acid addition salt formed by the compound of general formula I according to claim 1 and hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid or ferulic acid.

11. A method for the preparation of the compound according to claim 1, comprising the steps:
when Ar represents 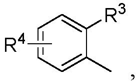 X¹ and X² each independently represent CH, L represents -(CH₂)ₘ-, m=0, T represents C=O, U represents N, V represents CH₂, and n=0, a synthetic route for formula I-A is as follows: 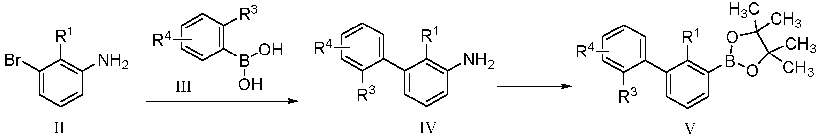 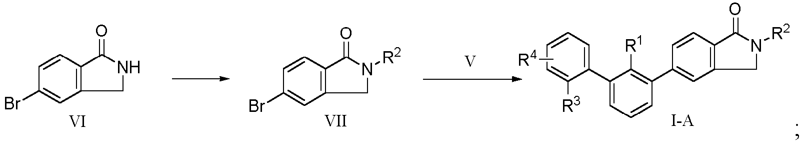
when Ar represents 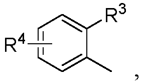 X¹ represents N, X² represents CH, L represents -(CH₂)ₘ-, m=0, T represents CH₂, U represents N, V represents CH₂, and n=1, a synthetic route for formula I-B is as follows: 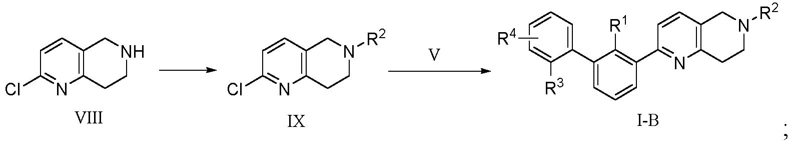
when Ar represents 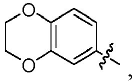 X¹ represents N, X² represents CH, L represents -(CH₂)ₘ-, m=0, T is CH₂, U represents N, V represents CH₂, and n=1, a synthetic route for formula I-C is as follows: 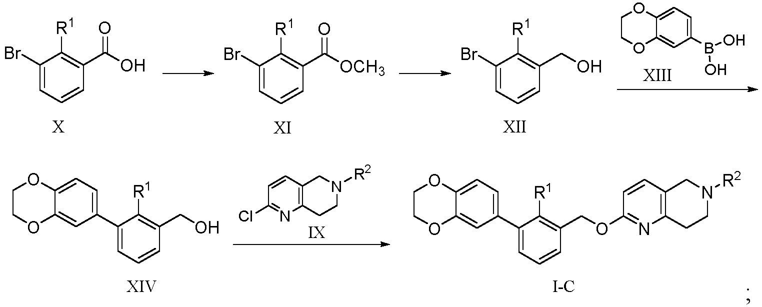
when Ar represents 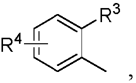 X¹ represents N, X² represents CH, L represents -CH₂O-, T is CH₂, U represents N, V represents CH₂, and n=1, a synthetic route for formula I-D is as follows: 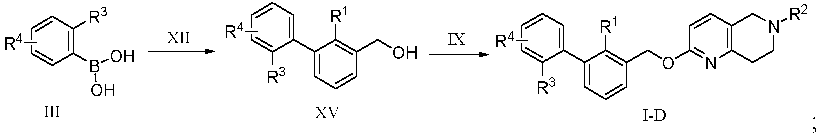
when Ar represents 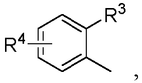 X¹ represents N, X² represents CH, L represents -CH₂O-, T represents CH₂, U represents N, V represents NCH₃, and n=1, a synthetic route for formula I-E is as follows: 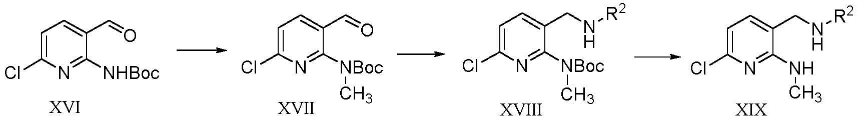 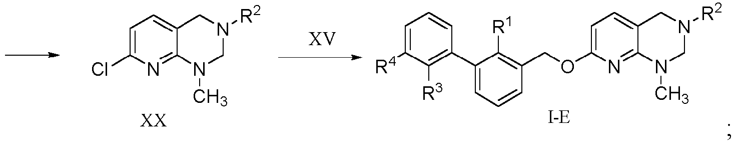 or,
when Ar represents 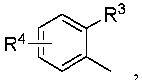 X¹ represents N, X² represents CH, L represents -HNCO-, T represents CH₂, U represents N, V represents CH₂, and n=1, a synthetic route for formula I-F is as follows:

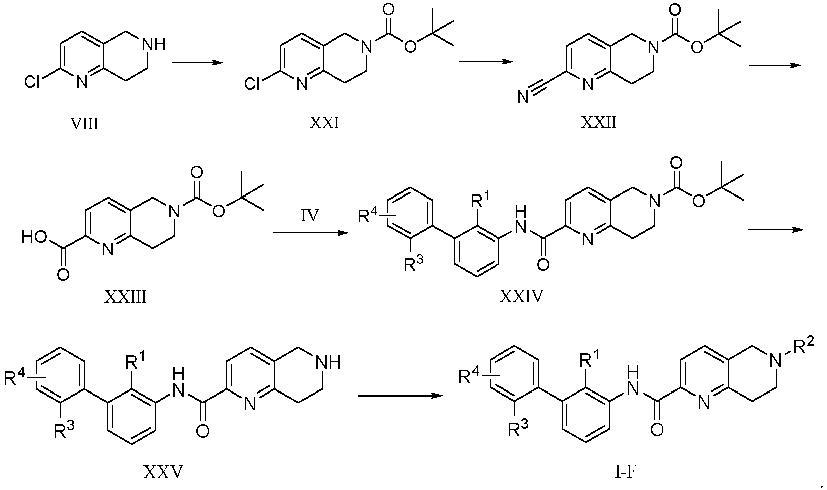

12. A pharmaceutical composition, **characterized by** comprising the compound, or the hydrate, solvate or pharmaceutically acceptable salt thereof according to any one of claims 1 to 10 and a pharmaceutically acceptable carrier.

13. A use of the compound, or the hydrate, solvate or pharmaceutically acceptable salt thereof according to any one of claims 1 to 10 in the preparation of a PD-1/PD-L1 inhibitor medicament.

14. A use of the compound, or the hydrate, solvate or pharmaceutically acceptable salt thereof according to any one of claims 1 to 10 in the preparation of an anti-tumor medicament.

15. A method for treating a tumor, comprising administering to a patient in need a therapeutically effective amount of the compound, or the hydrate, solvate or pharmaceutically acceptable salt thereof according to any one of claims 1 to 10.
